(19) Europäisches Patentamt
European Patent Office
Office européen des brevets

(11) EP 4 253 356 A1

(12) **EUROPEAN PATENT APPLICATION**
published in accordance with Art. 153(4) EPC

(43) Date of publication:
**04.10.2023 Bulletin 2023/40**

(21) Application number: **21897821.1**

(22) Date of filing: **17.11.2021**

(51) International Patent Classification (IPC):
***C07C 37/52*** *(2006.01)* ***C07C 37/68*** *(2006.01)*
***C07C 39/16*** *(2006.01)* ***C07C 68/08*** *(2006.01)*
***C07C 69/96*** *(2006.01)* ***C08G 64/04*** *(2006.01)*
***C08G 64/06*** *(2006.01)* ***C08G 64/20*** *(2006.01)*
***C08J 11/16*** *(2006.01)* ***C08J 11/24*** *(2006.01)*
***C08J 11/28*** *(2006.01)*

(52) Cooperative Patent Classification (CPC):
(C-Sets available)
**C07C 37/0555; C07C 68/06; C08G 64/04; C08G 64/06; C08G 64/20; C08J 11/16; C08J 11/24; C08J 11/28;** Y02W 30/62 (Cont.)

(86) International application number:
**PCT/JP2021/042234**

(87) International publication number:
**WO 2022/113847 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 JP 2020196659**
**16.07.2021 JP 2021118115**

(71) Applicant: **Mitsubishi Chemical Corporation**
**Chiyoda-ku**
**Tokyo 100-8251 (JP)**

(72) Inventor: **UCHIYAMA, Kei**
**Tokyo 100-8251 (JP)**

(74) Representative: **Vossius & Partner**
**Patentanwälte Rechtsanwälte mbB**
**Siebertstraße 3**
**81675 München (DE)**

(54) **POLYCARBONATE RESIN DECOMPOSITION METHOD, BISPHENOL PRODUCTION METHOD, DIALKYL CARBONATE PRODUCTION METHOD, ALKYL ARYL CARBONATE PRODUCTION METHOD, DIARYL CARBONATE PRODUCTION METHOD, RECYCLED POLYCARBONATE RESIN PRODUCTION METHOD, EPOXY RESIN PRODUCTION METHOD, AND EPOXY RESIN CURED PRODUCT PRODUCTION METHOD**

(57) A method for degrading a polycarbonate resin that can degrade a polycarbonate resin with high reactivity even under a condition that is mild and has a small environmental load is provided. In addition, a method for producing an alkyl aryl carbonate that can degrade a polycarbonate resin even under a condition that is mild and has a small environmental load and can efficiently obtain a dialkyl aryl carbonate is provided. A method for degrading a polycarbonate resin, comprising, in a slurry-like reaction liquid comprising a polycarbonate resin, an aromatic monoalcohol, an aliphatic monoalcohol, and a catalyst, degrading the polycarbonate resin. In addition, a method for producing an alkyl aryl carbonate, comprising: a polycarbonate resin degradation step of degrading a polycarbonate resin in the presence of an aromatic monoalcohol, an aliphatic monoalcohol, and a catalyst; and an alkyl aryl carbonate recovery step of recovering an alkyl aryl carbonate generated by the degradation of the polycarbonate.



Processed by Luminess, 75001 PARIS (FR)

(Cont. next page)

(52) Cooperative Patent Classification (CPC): (Cont.)

C-Sets
**C07C 37/0555, C07C 39/16;**
**C07C 68/06, C07C 69/96**

## Description

### Technical Field

The present invention relates to a method for degrading a polycarbonate resin. In addition, the present invention relates to a method for producing a bisphenol, a method for producing a dialkyl carbonate, and a method for producing an alkyl aryl carbonate, by using degradation of a polycarbonate resin. In addition, the present invention relates to a method for producing a dialkyl carbonate by using a dialkyl carbonate obtained by the method for producing a dialkyl carbonate or an alkyl aryl carbonate obtained by the method for producing an alkyl aryl carbonate. Further, the present invention relates to a method for producing a recycled polycarbonate resin by using a bisphenol obtained by the method for producing a bisphenol or a diaryl carbonate obtained by the method for producing a diaryl carbonate. In addition, the present invention relates to a method for producing an epoxy resin and a method for producing an epoxy resin cured product.

### Background Art

A plastic is easy to use, durable, and inexpensive, and thus is mass-produced not only in Japan but also all over the world. Many of such plastics are used as "disposable" ones and thus some thereof are not appropriately treated and released into the environment. Specifically, a plastic waste flows from a river into the sea and deteriorates because of a wave or an ultraviolet ray in the process to become 5 mm or less in size. Such a small plastic waste is referred to as a microplastic. An animal or fish accidentally swallows this microplastic. As described above, a plastic waste has a tremendous influence on the ecosystem, and, in recent years, has been regarded as a marine plastic problem all over the world.

A polycarbonate resin is used in a wide range of fields because of the transparency, mechanical physical properties, flame retardancy, dimensional stability, and electrical characteristics thereof, and this polycarbonate resin is also no exception.

As one of the methods for recycling a polycarbonate resin, there is chemical recycling involving chemically degrading a polycarbonate resin back to a bisphenol for reuse, and alcoholysis is known as one of the methods for degrading a polycarbonate resin.

For example, Patent Literature 1 discloses a method for continuously ring-opening an aromatic polycarbonate into a dihydroxy compound and dimethyl carbonate by subjecting the aromatic polycarbonate dissolved in a monohydroxy compound other than methanol to catalytic ester exchange with methanol in a distillation column.

In addition, Patent Literature 2 discloses a method for recovering a useful product from a waste plastic, comprising: a step of adding a specific tertiary amine as a catalyst into a solution containing a waste plastic and a monohydric alcohol or a monohydric phenol to chemically degrade the polycarbonate resin in the waste plastic; and a step of recovering a degradation product as a useful product.

Patent Literature 3 discloses a method for obtaining an aromatic dihydroxy compound from a waste aromatic polycarbonate, comprising subjecting a waste aromatic polycarbonate to a degradation reaction by an ester exchange reaction in the presence of an alcohol having 1 to 4 carbon atoms, a chlorinated compound organic solvent, and a metal hydroxide.

### Citation List

#### Patent Literature

Patent Literature 1: Japanese Patent Laid-Open No. 6-340591
Patent Literature 2: Japanese Patent Laid-Open No. 2004-51620
Patent Literature 3: Japanese Patent Laid-Open No. 2006-22029

### Summary of Invention

#### Technical Problem

An aliphatic monoalcohol is usually used in a degradation method using alcoholysis of a polycarbonate resin. However, an aliphatic monoalcohol having a small number of carbon atoms has a low boiling point, and thus when it is reacted at a high degradation temperature, a high pressure condition is required. Because of this, a pressure resistant

container needs to be used.

In addition, according to Example 1 of Patent Literature 1, a polycarbonate resin is dissolved in phenol at 150°C and brought into countercurrent contact with a vapor consisting of methanol and dimethyl carbonate to degrade the polycarbonate resin, and problems of carrying out this at normal pressure are that it is complicated to control the apparatus and it is difficult to control the degradation of the polycarbonate resin.

In addition, in the method of Patent Literature 2, it is difficult for a polycarbonate resin to dissolve in methanol, and thus an amine is used as a solvent and a catalyst. However, when the amount of the amine is small, a problem is that the reactivity is low and the reaction time is lengthened.

In a degradation method for degrading a polycarbonate resin with methanol at around room temperature, a chlorinated hydrocarbon solvent is used in order to dissolve the polycarbonate. For example, according to Example 1 of Patent Literature 3, methylene chloride is used as a solvent. A chlorinated hydrocarbon solvent such as methylene chloride is chemically stable and thus is a flame retardant compound. Because of this, a problem thereof is that a dioxin is generated if a disposal treatment is not appropriately carried out at a high temperature.

In any of the above methods for degrading a polycarbonate resin by using an aliphatic monoalcohol, a high temperature or high pressure degradation condition is required, the reactivity is insufficient, or a solvent that damages the environment is required, and these methods are required to be further improved.

The present invention has been made in view of such circumstances, and an object thereof is to provide a method for degrading a polycarbonate resin that can degrade a polycarbonate resin with high reactivity even under a condition that is mild and has a small environmental load.

In addition, another object of the present invention is to provide a method for producing a bisphenol, including producing a bisphenol by using the method for degrading a polycarbonate resin.

In addition, another object of the present invention is to provide a method for producing a recycled polycarbonate resin by using a bisphenol obtained by the method for producing a bisphenol.

In addition, another object of the present invention is to provide a method for producing an epoxy resin by using the bisphenol obtained by the method for producing a bisphenol, and a method for producing an epoxy resin cured product by using the obtained epoxy resin.

In addition, another object of the present invention is to provide a method for producing a dialkyl carbonate, including producing a dialkyl carbonate by using the method for degrading a polycarbonate resin, and a method for producing a diaryl carbonate by using a dialkyl carbonate obtained by the method for producing a dialkyl carbonate. Further, another object of the present invention is to provide a method for producing a recycled polycarbonate resin by using a diaryl carbonate obtained by the method for producing a diaryl carbonate.

On the other hand, as the method for producing a dialkyl carbonate by using a dialkyl carbonate, a method involving reacting a dialkyl carbonate with an aromatic monoalcohol to obtain an alkyl aryl carbonate, and then subjecting the alkyl aryl carbonate to a disproportionation reaction to obtain a diaryl carbonate is known. However, the reaction of reacting a dialkyl carbonate with an aromatic monoalcohol to obtain an alkyl aryl carbonate is an equilibrium reaction. In this reaction, the reaction rate is slow, and further, the equilibrium is extremely biased toward the raw material system, and a method for efficiently obtaining an alkyl aryl carbonate is required.

Therefore, another object of the present invention is to provide a method for producing an alkyl aryl carbonate that can degrade a polycarbonate resin even under a condition that is mild and has a small environmental load and can efficiently obtain an alkyl aryl carbonate. In addition, another object of the present invention is to provide a method for producing a diaryl carbonate by using an alkyl aryl carbonate obtained by the method for producing an alkyl aryl carbonate. Further, another object of the present invention is to provide a method for producing a recycled polycarbonate resin by using a diaryl carbonate obtained by the method for producing a diaryl carbonate.

## Solution to Problem

The present inventors have carried out a diligent study in order to solve the above problems and, as a result, found a degradation method for degrading a polycarbonate resin by using an aromatic monoalcohol and an aliphatic monoalcohol in combination. In addition, the present inventors have found that this degradation method can be used in a method for producing a bisphenol, a dialkyl carbonate, and/or an alkyl aryl carbonate.

That is, the present invention relates to the following invention.

<1> A method for degrading a polycarbonate resin, comprising, in a slurry-like reaction liquid comprising a polycarbonate resin, an aromatic monoalcohol, an aliphatic monoalcohol, and a catalyst, degrading the polycarbonate resin.

<2> A method for degrading a polycarbonate resin, comprising: a preparation step of preparing a slurry-like reaction liquid comprising a polycarbonate resin, an aromatic monoalcohol, an aliphatic monoalcohol, and a catalyst; and a degradation reaction step of degrading the polycarbonate resin in the slurry-like reaction liquid prepared in the preparation step.

<3> The method for degrading a polycarbonate resin according to the <1> or <2>, wherein the catalyst is any selected from the group consisting of an alkali metal hydroxide, an alkali metal carbonate, an alkylamine, and an acid.
<4> The method for degrading a polycarbonate resin according to the <3>, wherein the alkylamine is represented by the following formula (I):

[Formula 1]

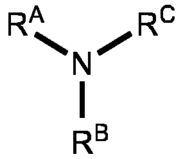

$\cdots$ (I)

wherein $R^A$ represents an alkyl group having 1 to 3 carbon atoms, and $R^B$ and $R^C$ each independently represent a hydrogen atom or an alkyl group having 1 to 3 carbon atoms.
<5> The method for degrading a polycarbonate resin according to the <3>, wherein the alkylamine is a tertiary amine.
<6> The method for degrading a polycarbonate resin according to any one of the <3> to <5>, wherein a mole ratio of the alkylamine to 1 mol of a repeating unit of the polycarbonate resin in the slurry-like reaction liquid is 4.5 or less.
<7> The method for degrading a polycarbonate resin according to the <3>, wherein the alkali metal hydroxide is sodium hydroxide or potassium hydroxide.
<8> The method for degrading a polycarbonate resin according to the <3>, wherein the acid is any selected from the group consisting of sulfuric acid, phosphoric acid, and a sulfonic acid.
<9> The method for degrading a polycarbonate resin according to any one of the <1> to <8>, wherein the aromatic monoalcohol is any selected from the group consisting of phenol, a cresol, and a xylenol.
<10> The method for degrading a polycarbonate resin according to any one of the <1> to <9>, wherein the aliphatic monoalcohol is any selected from the group consisting of methanol, ethanol, and n-butanol.
<11> The method for degrading a polycarbonate resin according to any one of the <1> to <10>, wherein a mole ratio of the aliphatic monoalcohol to the aromatic monoalcohol is 0.7 or less.
<12> The method for degrading a polycarbonate resin according to any one of the <1> to <11>, wherein a reaction temperature for degrading the polycarbonate resin is 120°C or less.
<13> A method for producing a bisphenol, comprising: a degradation step of degrading a polycarbonate resin by using the method for degrading a polycarbonate resin according to any one of the <1> to <12>; and a bisphenol recovery step of recovering a bisphenol generated by the degradation of the polycarbonate resin.
<14> The method for producing a bisphenol according to the <13>, wherein the bisphenol is 2,2-bis(4-hydroxyphenyl)propane.
<15> A method for producing a dialkyl carbonate, comprising: a degradation step of degrading a polycarbonate resin by using the method for degrading a polycarbonate resin according to any one of the <1> to <12>; and a dialkyl carbonate recovery step of recovering a dialkyl carbonate generated by the degradation of the polycarbonate resin.
<16> The method for degrading a dialkyl carbonate according to the <15>, wherein a mole ratio of the aliphatic monoalcohol to 1 mol of a repeating unit of the polycarbonate resin in the slurry-like reaction liquid is 2.0 or more and 6.0 or less.
<17> A method for producing an alkyl aryl carbonate, comprising: a polycarbonate resin degradation step of degrading a polycarbonate resin in the presence of an aromatic monoalcohol, an aliphatic monoalcohol, and a catalyst; and an alkyl aryl carbonate recovery step of recovering an alkyl aryl carbonate generated by the degradation of the polycarbonate.
<18> A method for producing an alkyl aryl carbonate, comprising: a degradation step of degrading a polycarbonate resin by using the method for degrading a polycarbonate resin according to any one of the <1> to <12>; and an alkyl aryl carbonate recovery step of recovering an alkyl aryl carbonate generated by the degradation of the polycarbonate resin.
<19> The method for producing an alkyl aryl carbonate according to the <17> or <18>, wherein the aromatic monoalcohol is phenol and the alkyl aryl carbonate is an alkyl phenyl carbonate.
<20> The method for producing an alkyl aryl carbonate according to any one of the <17> to <19>, wherein a mole ratio of the aliphatic monoalcohol to 1 mol of a repeating unit of the polycarbonate resin is 0.1 or more and less than 2.0.
<21> A method for producing a diaryl carbonate, comprising producing a diaryl carbonate by using a dialkyl carbonate obtained by the method for producing a dialkyl carbonate according to the <15> or <16>.
<22> A method for producing a diaryl carbonate, comprising producing a diaryl carbonate by using an alkyl aryl carbonate obtained by the method for producing an alkyl aryl carbonate according to any one of the <17> to <19>.

<23> A method for producing a recycled polycarbonate resin, comprising producing a recycled polycarbonate resin by using a bisphenol raw material comprising a bisphenol obtained by the method for producing a bisphenol according to the <13> or <14>.

<24> A method for producing a recycled polycarbonate resin, comprising producing a recycled polycarbonate resin by using a diaryl carbonate raw material comprising a diaryl carbonate obtained by the method for producing a diaryl carbonate according to the <21> or <22>.

<25> A method for producing a recycled epoxy resin, comprising producing a recycled epoxy resin by using a bisphenol obtained by the method for producing a bisphenol according to the <13> or <14>.

<26> The method for producing a recycled epoxy resin according to the <25>, wherein the recycled epoxy resin is further reacted with a polyhydroxy compound raw material.

<27> A method for producing a recycled epoxy resin cured product, comprising curing a recycled epoxy resin composition comprising a recycled epoxy resin obtained by the method for producing a recycled epoxy resin according to the <25> or <26> and a curing agent to obtain a recycled epoxy resin cured product.

Advantageous Effects of Invention

According to the present invention, a method for degrading a polycarbonate resin that can degrade a polycarbonate resin with high reactivity even under a condition that is mild and has a small environmental load is provided.

In addition, according to the present invention, a method for producing a bisphenol, including producing a bisphenol by using the method for degrading a polycarbonate resin is provided.

In addition, according to the present invention, a method for producing a recycled polycarbonate resin by using a bisphenol obtained by the method for producing a bisphenol is provided.

In addition, according to the present invention, a method for producing an epoxy resin by using the bisphenol obtained by the method for producing a bisphenol, and a method for producing an epoxy resin cured product by using the obtained epoxy resin are provided.

In addition, according to the present invention, a method for producing a dialkyl carbonate, including producing a dialkyl carbonate by using the method for degrading a polycarbonate resin, and a method for producing a diaryl carbonate by using a dialkyl carbonate obtained by the method for producing a dialkyl carbonate are provided. Further, a method for producing a recycled polycarbonate resin by using a diaryl carbonate obtained by the method for producing a diaryl carbonate is provided.

In addition, according to the present invention, a method for producing an alkyl aryl carbonate that can degrade a polycarbonate resin even under a condition that is mild and has a small environmental load and can efficiently obtain an alkyl aryl carbonate is provided. In addition, a method for producing a diaryl carbonate by using an alkyl aryl carbonate obtained by the method for producing an alkyl aryl carbonate is provided. Further, a method for producing a recycled polycarbonate resin by using a diaryl carbonate obtained by the method for producing a diaryl carbonate is provided.

Brief Description of Drawings

[Figure 1] Figure 1 shows a flow diagram of the degradation method of the present invention.

[Figure 2] Figure 2 shows a flow diagram for showing an example of the method for producing a bisphenol according to the present invention.

[Figure 3] Figure 3 shows a flow diagram for showing an example of the method for producing a bisphenol according to the present invention.

[Figure 4] Figure 4 shows a flow diagram showing an example of the method for producing a bisphenol according to the present invention.

[Figure 5] Figure 5 shows a flow diagram for describing a step (A3) of Figure 2.

[Figure 6] Figure 6 shows a flow diagram for describing a step (A3) of Figure 2.

[Figure 7] Figure 7 shows a flow diagram for describing a step (B2) of Figure 3.

[Figure 8] Figure 8 shows a flow diagram for describing a step (B2) of Figure 3.

[Figure 9] Figure 9 shows a flow diagram showing an example of the method for producing a dialkyl carbonate according to the present invention.

[Figure 10] Figure 10 shows a flow diagram showing an example of the method for producing a dialkyl carbonate according to the present invention.

[Figure 11] Figure 11 shows a flow diagram showing an example of the method for producing an alkyl aryl carbonate according to the present invention.

[Figure 12] Figure 12 shows a flow diagram showing an example of the method for producing an alkyl aryl carbonate

according to the present invention.

Description of Embodiment

Hereinafter, an embodiment of the present invention will be described in detail, but the description of the constituent requirements described below is an example of the embodiment of the present invention, and the present invention is not limited to the contents of the following description as long as they do not depart from the scope of the present invention. In addition, as used herein, an expression of a range including the term "to" with numerical values or physical property values provided before and after the term is used to mean that the values before and after the term are included in the range.

<Method for degrading polycarbonate resin>

The present invention is a method for degrading a polycarbonate resin, including a degradation step of, in a slurry-like reaction liquid including a polycarbonate resin, an aromatic monoalcohol, an aliphatic monoalcohol, and a catalyst, degrading the polycarbonate resin (hereinafter, sometimes referred to as the "degradation method of the present invention").

In addition, as shown in Figure 1, the degradation method of the present invention can be a method for degrading a polycarbonate resin, including: a preparation step (S1) of preparing a slurry-like reaction liquid including a polycarbonate resin, an aromatic monoalcohol, an aliphatic monoalcohol, and a catalyst; and a degradation reaction step (S2) of degrading the polycarbonate resin in the slurry-like reaction liquid prepared in the preparation step (S1).

The degradation method of the present invention degrades a polycarbonate resin in the presence of an aromatic monoalcohol, an aliphatic monoalcohol, and a catalyst. The aromatic monoalcohol and the aliphatic monoalcohol are each a poor solvent for a polycarbonate resin, and it is considered that by mixing and using these solvents, the recovery, the separation, or the like of the solvents is also complicated. Because of this, conventionally, it has been considered that it is not beneficial to mix and use these solvents. However, surprisingly, the present inventors have found that a polycarbonate resin can be degraded even under a mild condition such as normal pressure by reaction in a mixed solvent including an aromatic monoalcohol and an aliphatic monoalcohol in the presence of a catalyst. In addition, it has been found that the degradation reaction of a polycarbonate resin is caused at a high reaction rate by using an aromatic monoalcohol and an aliphatic monoalcohol in combination, without completely dissolving the polycarbonate resin by using a solvent having high solubility of a polycarbonate resin such as a halogen solvent.

It is considered that by using an aromatic monoalcohol and an aliphatic monoalcohol in combination, both reactions of solvolysis due to the aromatic monoalcohol (for example, phenolysis) and solvolysis due to the aliphatic monoalcohol (for example, methanolysis) are caused in the system, and thus the polycarbonate resin is easily degraded even under a mild condition.

In addition, it is difficult for a polycarbonate resin to dissolve in an aromatic monoalcohol and an aliphatic monoalcohol, and thus when carrying out the reaction in a slurry-like reaction liquid in which the polycarbonate resin is dispersed in a mixed solvent of these monoalcohols, only the polycarbonate resin dissolved only in an amount corresponding to the solubility is involved in the degradation reaction, and thus the reaction can be easily controlled and the polycarbonate resin can be stably degraded.

(Polycarbonate resin)

The polycarbonate resin used in the degradation method according to the present invention includes a polymerization composition including a carbonate bond (-O-C(=O)-O-). Specifically, the polycarbonate resin used in the degradation method of the present invention includes a polymer including a repeating unit derived from a bisphenol represented by the general formula (1).

[Formula 2]

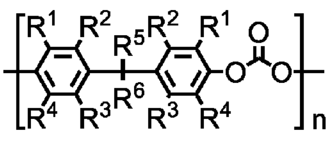

···(1)

The substituents $R^1$ to $R^4$ each independently represent a hydrogen atom, a halogen atom, an alkyl group, an alkoxy group, an aryl group, or the like. Examples thereof include a hydrogen atom, a fluoro group, a chloro group, a

bromo group, an iodine group, a methyl group, an ethyl group, a n-propyl group, an i-propyl group, a n-butyl group, an i-butyl group, a t-butyl group, a n-pentyl group, an i-pentyl group, a n-hexyl group, a n-heptyl group, a n-octyl group, a n-nonyl group, a n-decyl group, a n-undecyl group, a n-dodecyl group, a methoxy group, an ethoxy group, a n-propoxy group, an i-propoxy group, a n-butoxy group, an i-butoxy group, a t-butoxy group, a n-pentyloxy group, an i-pentyloxy group, a n-hexyloxy group, a n-heptyloxy group, a n-octyloxy group, a n-nonyloxy group, a n-decyloxy group, a n-undecyloxy group, a n-dodecyloxy group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, and 2,6-dimethylphenyl group.

The substituents $R^5$ and $R^6$ each independently represent a hydrogen atom, an alkyl group, an alkoxy group, an aryl group, or the like. Examples thereof include a hydrogen atom, a methyl group, an ethyl group, a n-propyl group, an i-propyl group, a n-butyl group, an i-butyl group, a t-butyl group, a n-pentyl group, an i-pentyl group, a n-hexyl group, a n-heptyl group, a n-octyl group, a 2-ethylhexyl group, a n-nonyl group, a n-decyl group, a n-undecyl group, a n-dodecyl group, a methoxy group, an ethoxy group, a n-propoxy group, an i-propoxy group, a n-butoxy group, an i-butoxy group, a t-butoxy group, a n-pentyloxy group, an i-pentyloxy group, a n-hexyloxy group, a n-heptyloxy group, a n-octyloxy group, a n-nonyloxy group, a n-decyloxy group, a n-undecyloxy group, a n-dodecyloxy group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, and 2,6-dimethylphenyl group.

$R^5$ and $R^6$ may be bonded or crosslinked to each other between the two groups to form a cycloalkylidene group. Examples thereof include cyclopropylidene, cyclobutylidene, cyclopentylidene, cyclohexylidene, 3,3,5-trimethylcyclohexylidene, cycloheptylidene, cyclooctylidene, cyclononylidene, cyclodecylidene, cycloundecylidene, cyclododecylidene, fluorenylidene, xanthonylidene, and thioxanthonylidene.

In the degradation method of the present invention, a polycarbonate resin wherein in the above general formula (1), $R^1$ to $R^4$ are each a hydrogen atom and $R^5$ and $R^6$ are each a methyl group (bisphenol A type polycarbonate resin) above all is suitably used as a raw material.

In the general formula (1), n is not particularly limited, and is, for example, 2 to 1000.

As the polycarbonate resin, which is a raw material, of the present invention, not only a polycarbonate resin alone but also a composition including a resin other than a polycarbonate resin such as a copolymer or a polymer alloy may be used. Examples of the composition including a resin other than a polycarbonate resin include a polycarbonate/polyester copolymer, a polycarbonate/polyester alloy, a polycarbonate/polyarylate copolymer, and a polycarbonate/polyarylate alloy. When a composition including a resin other than a polycarbonate resin is used, a composition including a polycarbonate resin as the main component (the composition includes 50% by mass or more of a polycarbonate resin) can be suitably used.

In addition, as the polycarbonate resin, two or more different polycarbonate resins can be mixed and used. The polycarbonate resin alone may be simply referred to as polycarbonate.

From the viewpoint of chemical recycling, the polycarbonate resin is preferably a polycarbonate resin included in a waste plastic. The polycarbonate resin is molded into various molded articles such as an optical member such as a headlamp or an optical recording medium such as an optical disk and used. As the waste plastic including a polycarbonate resin, a scrap or a defective product when molding and processing the polycarbonate resin into such a molded article, a used molded article, or the like can be used.

The waste plastic can be appropriately washed, crushed, ground, or the like before use. The methods for crushing the waste plastic include coarse crushing using a jaw crusher or a gyratory crusher for crushing to 20 cm or less, medium crushing using a gyratory crusher, a corn crusher, or a mill for crushing to 1 cm or less, grinding using a mill for crushing to 1 mm or less, or the like, and may be any method that can reduce the waste plastic to a size at which it can be supplied to a degradation tank. In addition, when the waste plastic is a thin plastic such as a CD or a DVD, the thin plastic can be cut by using a shredder or the like and supplied to a degradation tank. In addition, another resin of a copolymer or a polymer alloy, or a portion formed of a component other than a polycarbonate resin such as a layer on the front surface or the back surface of an optical disk may be removed in advance before use.

(Aromatic monoalcohol)

One of the features of the degradation method according to the present invention is that an aromatic monoalcohol is used. The aromatic monoalcohol is a compound in which one hydroxyl group is bonded to a carbon atom forming an aromatic ring, and is preferably any selected from the group consisting of phenol, a cresol, and a xylenol.

Examples of the cresol include orthocresol, metacresol, paracresol, and an isomer mixture including one or more thereof. If the cresol is a liquid at around 30°C, it is easy to supply it to a degradation tank, and thus the cresol is preferably orthocresol, metacresol, an isomer mixture of metacresol and paracresol, or an isomer mixture of orthocresol, metacresol, and paracresol.

Examples of the xylenol include 2,3-xylenol, 2,4-xylenol, 2,5-xylenol, 2,6-xylenol, 3,5-xylenol, 3,4-xylenol, and

an isomer mixture including one or more thereof. 2,5-Xylenol is preferable because it is industrially inexpensive to obtain.

When the amount of the aromatic monoalcohol used is small based on that of the polycarbonate resin used, the amount of the solid (polycarbonate resin) based on that of the liquid increases and the slurry concentration increases, which tends to result in poor mixing. Because of this, the mole ratio of the aromatic monoalcohol to 1 mol of the repeating unit of the polycarbonate resin (that is, the repeating unit represented by the above general formula (1)) ((mass [g] of aromatic monoalcohol used/molecular weight [g/mol] of aromatic monoalcohol)/(mass [g] of polycarbonate resin used/molecular weight [g/mol] of repeating unit)) is preferably 0.01 or more, and more preferably 0.03 or more. In addition, when the amount of the aromatic monoalcohol used is large based on that of the polycarbonate resin used, the production efficiency tends to deteriorate. Because of this, the mole ratio of the aromatic monoalcohol to 1 mol of the repeating unit of the polycarbonate resin is preferably 100 or less, more preferably 70 or less, and further preferably 50 or less.

(Aliphatic monoalcohol)

One of the features of the degradation method of the present invention is the use of an aliphatic monoalcohol. The aliphatic monoalcohol is a compound in which one hydroxyl group is bonded to an alkyl group, and is a compound represented by $R^7OH$ wherein $R^7$ represents an alkyl group. Examples of the aliphatic monoalcohol include methanol, ethanol, n-propanol, i-propanol, n-butanol, i-butanol, t-butanol, n-pentanol, i-pentanol, n-hexanol, n-heptanol, n-octanol, n-nonanol, n-decanol, n-undecanol, and n-dodecanol. Among these, the aliphatic monoalcohol is preferably a linear alcohol having 1 to 5 carbon atoms, and more preferably any selected from the group consisting of methanol, ethanol, and n-butanol.

When the amount of the aliphatic monoalcohol used is small based on that of the polycarbonate resin used, the polycarbonate resin is unlikely to be degraded or the degradation rate decreases, and thus the degradation time tends to be lengthened and the efficiency tends to deteriorate. Because of this, the mole ratio of the aliphatic monoalcohol to 1 mol of the repeating unit of the polycarbonate resin ((mass [g] of aliphatic monoalcohol used/molecular weight [g/mol] of aliphatic monoalcohol)/(mass [g] of polycarbonate resin used/molecular weight [g/mol] of repeating unit)) is preferably 0.1 or more, more preferably 0.5 or more, and further preferably 1.0 or more. In addition, when the amount of the aliphatic monoalcohol used is large based on that of the polycarbonate resin used, the production efficiency tends to deteriorate. Because of this, the mole ratio of the aliphatic monoalcohol to 1 mol of the repeating unit of the polycarbonate resin is preferably 6.0 or less, more preferably 5.5 or less, and further preferably 5.0 or less.

In addition, the structure of a carbonyl compound, which is a degradation product, can be controlled by adjusting the amount of the aliphatic monoalcohol, as will be described later. Therefore, it is preferable to control the amount of the aliphatic monoalcohol depending on the structure of the carbonyl compound to be obtained.

In order to efficiently produce a dialkyl carbonate, the mole ratio of the aliphatic monoalcohol to 1 mol of the repeating unit of the polycarbonate resin is preferably 2.0 or more, more preferably 2.1 or more, and further preferably 2.2 or more.

In addition, in order to suppress the generation of a dialkyl carbonate and efficiently produce an alkyl aryl carbonate, the mole ratio of the aliphatic monoalcohol to 1 mol of the repeating unit of the polycarbonate resin is preferably less than 2.0, and may be 1.95 or less, 1.9 or less, 1.85 or less, 1.8 or less, or the like.

In addition, the mole ratio of the aliphatic monoalcohol used to the aromatic monoalcohol used (number of moles of aliphatic monoalcohol used/number of moles of aromatic monoalcohol used) is preferably 0.01 or more, and more preferably 0.05 or more. In addition, the mole ratio is preferably 0.7 or less, more preferably 0.6 or less, more preferably 0.5 or less, and more preferably 0.3 or less. When the mole ratio of the aliphatic monoalcohol used to the aromatic monoalcohol used is small, the polycarbonate resin is unlikely to be degraded or the degradation rate decreases, and the degradation time is lengthened. In addition, when the mole ratio is large, the separation between the aliphatic monoalcohol and a dialkyl carbonate becomes complicated when recovering the dialkyl carbonate.

(Catalyst)

One of the features of the degradation method according to the present invention is that further a catalyst is used. The catalyst may be any as long as it can accelerate the degradation of the polycarbonate resin, and the catalyst is preferably any selected from the group consisting of an alkali metal hydroxide, an alkali metal carbonate, an alkylamine, and an acid.

[Alkali metal hydroxide]

The alkali metal hydroxide is a salt of an alkali metal ion ($M^+$) and a hydroxide ion ($OH^-$) , and is a compound represented by MOH where M represents an alkali metal atom. As the alkali metal hydroxide, sodium hydroxide or potassium hydroxide is preferable.

When the amount of the alkali metal hydroxide used is small based on that of the polycarbonate resin used, the degradation rate decreases, the degradation time is lengthened, and the efficiency tends to deteriorate. Because of this, the mole ratio of the alkali metal hydroxide to 1 mol of the repeating unit of the polycarbonate resin ((mass [g] of alkali metal hydroxide used/molecular weight [g/mol] of alkali metal hydroxide)/(mass [g] of polycarbonate resin used/molecular weight [g/mol] of repeating unit)) is preferably 0.0001 or more, more preferably 0.0005 or more, and further preferably 0.0007 or more. For example, the mole ratio thereof can be 0.001 or more, 0.01 or more, 0.1 or more, or the like. When the amount of the alkali metal hydroxide used is large based on that of the polycarbonate resin used, the amount of an acid required for neutralization after degradation increases, and the production efficiency tends to decrease. Because of this, the mole ratio of the alkali metal hydroxide to 1 mol of the repeating unit of the polycarbonate resin is preferably 1 or less, more preferably 0.9 or less, and further preferably 0.8 or less.

[Alkali metal carbonate]

The alkali metal carbonate is a salt of an alkali metal ion ($M^+$) and a carbonate ion ($CO_3^{2-}$), and is a compound represented by $M_2CO_3$ where M represents an alkali metal atom. As the alkali metal carbonate, sodium carbonate or potassium carbonate is preferable.

When the amount of the alkali metal carbonate used is small based on that of the polycarbonate resin used, the degradation rate decreases, the degradation time is lengthened, and the efficiency tends to deteriorate. Because of this, the mole ratio of the alkali metal carbonate to 1 mol of the repeating unit of the polycarbonate resin ((mass [g] of alkali metal carbonate used/molecular weight [g/mol] of alkali metal carbonate)/(mass [g] of polycarbonate resin used/molecular weight [g/mol] of repeating unit)) is preferably 0.0001 or more, more preferably 0.0005 or more, and further preferably 0.001 or more. For example, the mole ratio thereof can be 0.001 or more, 0.01 or more, 0.1 or more, or the like. When the amount of the alkali metal carbonate used is large based on that of the polycarbonate resin used, the amount of an acid required for neutralization after degradation increases, and the production efficiency tends to decrease. Because of this, the mole ratio of the alkali metal carbonate to 1 mol of the repeating unit of the polycarbonate resin is preferably 1 or less, more preferably 0.9 or less, and further preferably 0.8 or less.

[Alkylamine]

The alkylamine is a compound obtained by replacing at least one hydrogen atom of ammonia with an alkyl group. Among the alkylamines, a monoalkylamine, which is a primary amine, reacts with a carbonate bond moiety of the polycarbonate resin to form an isocyanate, and thus a dialkylamine, which is a secondary amine, and a trialkylamine, which is a tertiary amine, are more preferable.

The dialkylamine, which is a secondary amine, reacts with a carbonate bond moiety of the polycarbonate resin to form a tetraalkylurea, and thus a trialkylamine, which is a tertiary amine, is further preferable.

An alkylamine having a boiling point of 200°C or less is preferable, and an alkylamine having a boiling point of 160°C or less is more preferable. If an alkylamine has such a boiling point, the alkylamine can be removed by depressurization and/or heating together with an aromatic monoalcohol such as phenol. In addition, when the boiling point is too low, the alkylamine may volatilize during the degradation reaction and the degradation rate may decrease, and thus the boiling point of the alkylamine is preferably 10°C or more, and more preferably 30°C or more.

The alkylamine is preferably an alkylamine represented by the general formula (I).

[Formula 3]

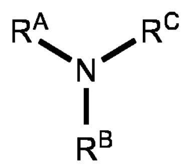

$\cdots$ (I)

wherein $R^A$ represents an alkyl group having 1 to 3 carbon atoms, and $R^B$ and $R^C$ each independently represent a hydrogen atom or an alkyl group having 1 to 3 carbon atoms.

$R^A$ is preferably a methyl group, an ethyl group, a n-propyl group, or an i-propyl group, and $R^B$ to $R^C$ is each independently preferably a hydrogen atom, a methyl group, an ethyl group, a n-propyl group, or an i-propyl group.

Specific examples of the alkylamine represented by the general formula (I) include methylamine, ethylamine, propylamine, dimethylamine, diethylamine, trimethylamine, and triethylamine.

When the amount of the alkylamine used is small based on that of the polycarbonate resin used, the degradation

rate decreases, the degradation time is lengthened, and the efficiency tends to deteriorate. Because of this, the mole ratio of the alkylamine to 1 mol of the repeating unit of the polycarbonate resin ((mass [g] of alkylamine used/molecular weight [g/mol] of alkylamine)/(mass [g] of polycarbonate resin used/molecular weight [g/mol] of repeating unit)) is preferably 0.0005 or more, more preferably 0.0007 or more, and further preferably 0.001 or more. For example, the mole ratio thereof can be 0.01 or more, 0.1 or more, or the like. When the amount of the alkylamine used is large based on that of the polycarbonate resin used, an amine odor is more likely to be generated, and a dialkyl carbonate and/or an alkyl aryl carbonate is unlikely to be generated. Because of this, the mole ratio of the alkylamine to 1 mol of the repeating unit of the polycarbonate resin is preferably 4.5 or less, and more preferably 4.0 or less, 3.0 or less, 2.0 or less, 1.0 or less, 0.9 or less, or 0.8 or less, where a smaller value is more preferable.

[Acid]

Examples of the acid include an inorganic acid such as hydrochloric acid, sulfuric acid, or phosphoric acid, and an organic acid such as carboxylic acid or a sulfonic acid. The acid is preferably any selected from the group consisting of sulfuric acid, phosphoric acid, and a sulfonic acid. Examples of the sulfonic acid include an alkylsulfonic acid such as methanesulfonic acid and an aromatic sulfonic acid such as toluenesulfonic acid.

When the amount of the acid used is small based on that of the polycarbonate resin used, the degradation rate decreases, the degradation time is lengthened, and the efficiency tends to deteriorate. Because of this, the mole ratio of the acid to 1 mol of the repeating unit of the polycarbonate resin ((mass [g] of acid used/molecular weight [g/mol] of acid)/(mass [g] of polycarbonate resin used/molecular weight [g/mol] of repeating unit)) is preferably 0.0001 or more, more preferably 0.0005 or more, and further preferably 0.007 or more. When the amount of the acid used is large based on that of the polycarbonate resin used, the amount of a base required for neutralization after degradation increases, and the production efficiency tends to decrease. Because of this, the mole ratio of the acid to 1 mol of the repeating unit of the polycarbonate resin is preferably 1 or less, more preferably 0.9 or less, and further preferably 0.8 or less.

(Reaction liquid)

The reaction liquid prepared is a slurry-like solution in which a polycarbonate resin is dispersed in a liquid component including an aromatic monoalcohol and an aliphatic monoalcohol. The slurry concentration of the reaction liquid (mass of solid in reaction liquid/mass of reaction liquid) is preferably 0.01 or more, and more preferably 0.05 or more. In addition, the slurry concentration is preferably 0.5 or less, and more preferably 0.3 or less. When the slurry concentration (concentration of solid) is too low, the degradation efficiency decreases, and when the slurry concentration is too high, poor mixing occurs.

The liquid components in the reaction liquid prepared include the aromatic monoalcohol and the aliphatic monoalcohol as the main components, and the total mass of the aromatic monoalcohol and the aliphatic monoalcohol based on the mass of all liquid components is 0.8 or more, 0.9 or more, 0.95 or more, or the like.

The total mass of the polycarbonate resin, the aromatic monoalcohol, the aliphatic monoalcohol, and a catalyst to the mass of the reaction liquid can be 0.9 or more, 0.95 or more, 0.98 or more, 0.99 or more, or the like. In addition, the reaction liquid may include a polycarbonate resin, an aromatic monoalcohol, an aliphatic monoalcohol, and a catalyst.

When a dialkyl carbonate and/or an alkyl aryl carbonate is to be obtained together with a bisphenol, if water is included in the reaction liquid, the generated dialkyl carbonate and/or alkyl aryl carbonate is easily degraded. Because of this, the content of water in the reaction liquid (mass of water/mass of reaction liquid) is usually 0.005 or less. The content of water in the reaction liquid is preferably 0.001 or less, and more preferably 0.0005 or less.

(Preparation of reaction liquid)

The reaction liquid is prepared preferably at 10°C or more, and more preferably at 20°C or more. In addition, the reaction liquid is prepared preferably at 40°C or less, and more preferably at 35°C or less. When the temperature at the time of preparing the reaction liquid is too low, the aromatic monoalcohol is likely to solidify depending on the type thereof, and it may be easy for poor mixing to occur or it may be difficult to carry out uniform mixing. In addition, when the temperature at the time of preparing the reaction liquid is too high, the catalyst is likely to volatilize depending on the type thereof, and it may also be difficult to prepare the reaction liquid to a predetermined concentration or control the degradation reaction.

The mixing order of a polycarbonate resin, an aromatic monoalcohol, an aliphatic monoalcohol, and a catalyst is not particularly limited, and for example, the aromatic monoalcohol, the aliphatic monoalcohol, and the catalyst may be sequentially supplied to the polycarbonate resin, or the polycarbonate resin, the aliphatic monoalcohol, and the catalyst may be sequentially supplied to the aromatic monoalcohol. In order to allow more uniform mixing, the polycarbonate resin is preferably supplied to a reaction tank after the aromatic monoalcohol and/or the aliphatic monoalcohol.

(Degradation reaction)

The presence of an aromatic monoalcohol, an aliphatic monoalcohol, and a catalyst cleaves a carbonate bond moiety of the polycarbonate resin to cause the degradation thereof. Thereby, a polycarbonate resin degradation product including a bisphenol and a carbonyl compound is generated. The generated carbonyl compound includes a dialkyl carbonate and/or an alkyl aryl carbonate. Depending on the amounts of the aromatic monoalcohol and the aliphatic monoalcohol in the slurry-like reaction liquid, the generated carbonyl compound may include the dialkyl carbonate as the main component or be a mixture of the dialkyl carbonate and the alkyl aryl carbonate.

For example, as described above, by setting the mole ratio of the aliphatic monoalcohol to 1 mol of the repeating unit of the polycarbonate resin to 2.0 or more, the reaction as shown in the following reaction equation (2) preferentially occurs. This can efficiently degrade the polycarbonate resin into a bisphenol and a dialkyl carbonate. In the reaction equation (2), $R^1$ to $R^6$ and n are defined as in the above general formula (1), and $R^7$ is an alkyl group.

[Formula 4]

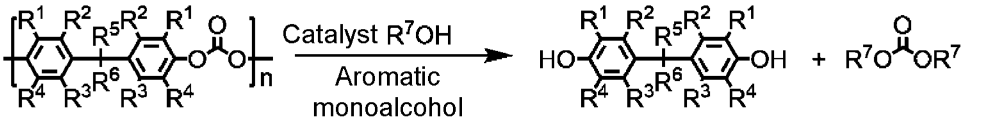


···(2)

In addition, by setting the mole ratio of the aliphatic monoalcohol to 1 mol of the repeating unit of the polycarbonate resin to less than 2.0 in the reaction liquid, a degradation product including a bisphenol and a dialkyl carbonate and/or an alkyl aryl carbonate may be obtained.

The degradation step can include: a preparation step of preparing a slurry-like reaction liquid including a polycarbonate resin, an aromatic monoalcohol, an aliphatic monoalcohol, and a catalyst; and a degradation reaction step of degrading the polycarbonate resin in the reaction liquid. At this time, by controlling the concentration of the polycarbonate resin, the temperature at the time of preparing the reaction liquid, or the like such that the degradation reaction does not proceed during the preparation of the reaction liquid (during the mixing of a polycarbonate resin, an aromatic monoalcohol, an aliphatic monoalcohol, and a catalyst), the preparation step and the degradation reaction step may be clearly separated, or the preparation step and the degradation step may not be clearly separated. During the reaction liquid preparation step, part of the polycarbonate resin may be dissolved, the degradation reaction may proceed, and part of the polycarbonate resin may be degraded. By degrading part of the polycarbonate resin during the of the reaction liquid preparation step, the degradation reaction can be caused to proceed more efficiently.

The degradation reaction may be carried out under normal pressure or under pressure, and the degradation reaction is preferably carried out under normal pressure because the reaction proceeds sufficiently even under normal pressure.

(Reaction temperature)

From the preparation of the reaction liquid to the stop of the degradation reaction, the temperature may be the same as the temperature at the time of preparing the reaction liquid, and after the reaction liquid is prepared (after a polycarbonate resin, an aromatic monoalcohol, an aliphatic monoalcohol, and a catalyst are mixed), it is preferable to raise the temperature to a predetermined reaction temperature. When the temperature at the time of preparing the reaction liquid is too high, it may be difficult to control the degradation reaction. It is preferable to raise the temperature after preparing the reaction liquid because the degradation reaction can proceed stably thereby.

The reaction temperature is appropriately selected depending on the type of the aromatic monoalcohol, the reaction time, or the like, and when the reaction temperature is high, aromatic monoalcohol in the reaction liquid evaporates and the alcoholysis stops. In addition, when the temperature is low, the aromatic monoalcohol solidifies, or the solvolysis is unlikely to proceed and the reaction rate decreases, and thus the time required for degradation is lengthened. Because of these, the reaction temperature is preferably 60°C or more, and more preferably 70°C or more, 75°C or more, or 80°C or more, where a larger value is more preferable. In addition, the reaction temperature is preferably 120°C or less, and more preferably 110°C or less, 100°C or less, or 95°C or less, where a smaller value is more preferable.

In particular, the degradation of the polycarbonate resin is preferably carried out at a reaction temperature of 60 to 120°C and under normal pressure, more preferably at a reaction temperature of 70 to 110°C and under normal pressure, and further preferably at a reaction temperature of 80 to 100°C and under normal pressure.

When the reaction is carried out at the same temperature as at the time of preparation of the reaction liquid, the reaction temperature is the average temperature from the time point when the mixing of a polycarbonate resin, an aromatic monoalcohol, an aliphatic monoalcohol, and a catalyst is completed to the time point when the operation of

neutralization or distillation for stopping the degradation reaction is started. In addition, when the reaction is carried out by raising the temperature after preparing the reaction liquid, the reaction temperature is the average temperature from the time point when the predetermined temperature is reached to the time point when the operation of neutralization or distillation operation for stopping the degradation reaction is started.

(Reaction time)

The reaction time is appropriately selected depending on the slurry concentration, the reaction temperature, or the like, and when the reaction time is long, the produced bisphenol tends to be degraded, and thus the reaction time is preferably 30 hours or less, and more preferably 25 hours or less, 20 hours or less, 15 hours or less, 10 hours or less, or 5 hours or less, where a shorter value is more preferable. In addition, when the reaction time is short, the degradation reaction may not proceed sufficiently, and thus the reaction time is preferably 0.1 hours or more, more preferably 0.5 hours or more, and further preferably 1 hour or more.

The reaction time is the time from the time point when the mixing of a polycarbonate resin, an aromatic monoalcohol, an aliphatic monoalcohol, and a catalyst is completed to the time point when the operation of neutralization or distillation for stopping the degradation reaction is started. The end point of the reaction time may be determined by tracking the degradation reaction by liquid chromatography or the like.

(Method for stopping degradation reaction of polycarbonate resin)

The method for stopping the degradation reaction of the polycarbonate resin is appropriately selected depending on the type of the catalyst used. When an alkylamine is used as the catalyst, the degradation reaction can be stopped by distilling off or neutralizing the alkylamine. In a method for removing an alkylamine by neutralization by supplying an acid, an ammonium salt is generated and also needs to be removed, and thus the alkylamine is removed preferably by a method for distilling off the same. In addition, when an alkali metal hydroxide, an alkali metal carbonate, or an acid is used as the catalyst, the degradation reaction can be stopped by neutralization or the like.

<Method for producing bisphenol>

The present invention relates to a method for producing a bisphenol, including: a degradation step of degrading a polycarbonate resin by using the degradation method of the present invention; and a bisphenol recovery step of recovering a bisphenol generated by the degradation of the polycarbonate resin (hereinafter, sometimes referred to as the "method for producing a bisphenol according to the present invention"). That is, the method for producing a bisphenol according to the present invention includes: a degradation step of, in a slurry-like reaction liquid including a polycarbonate resin, an aromatic monoalcohol, an aliphatic monoalcohol, and a catalyst, degrading the polycarbonate resin; and a bisphenol recovery step of recovering a bisphenol generated in the degradation step. In addition, the method for producing a bisphenol according to the present invention can include: a preparation step of preparing a slurry-like reaction liquid including a polycarbonate resin, an aromatic monoalcohol, an aliphatic monoalcohol, and a catalyst; a degradation reaction step of degrading the polycarbonate resin in the slurry-like reaction liquid prepared in the preparation step; and a bisphenol recovery step of recovering a bisphenol generated in the degradation reaction step.

As described above, a bisphenol is generated as a degradation product in the degradation method of the present invention, and thus the degradation method of the present invention can be used in the method for producing a bisphenol. The degradation step is as described in the degradation method of the present invention.

Above all, the method for producing a bisphenol according to the present invention is suitable as a method for producing 2,2-bis(4-hydroxyphenyl)propane (hereinafter, sometimes referred to as "bisphenol A").

<Bisphenol recovery step>

The method for producing a bisphenol according to the present invention includes a bisphenol recovery step of recovering a bisphenol obtained in the degradation step. The recovery of the bisphenol from the reaction liquid after the degradation reaction can be carried out by means such as crystallization or column chromatography after stopping the degradation reaction of the polycarbonate resin.

The bisphenol recovery step of the method for producing a bisphenol according to the present invention preferably includes a crystallization step of recovering a bisphenol by crystallization. Specifically, after the degradation reaction of the polycarbonate resin, the catalyst, the solvent, and the carbonyl compound are removed from the reaction liquid and an organic solvent is added and mixed, and the resulting organic phase is washed with water, saline, or the like, and further, if necessary, neutralized and washed with aqueous ammonium chloride or the like. Next, the washed organic phase is cooled and subjected to crystallization.

Examples of the organic solvent that can be used at the time of neutralization and crystallization include an aromatic hydrocarbon such as toluene, xylene, ethylbenzene, diethylbenzene, isopropylbenzene, or mesitylene, an aliphatic hydrocarbon such as hexane, heptane, octane, nonane, decane, undecane, or dodecane, and an aliphatic monoalcohol such as methanol, ethanol, n-propanol, i-propanol, n-butanol, i-butanol, t-butanol, n-pentanol, i-pentanol, n-hexanol, n-heptanol, n-octanol, n-nonanol, n-decanol, n-undecanol, n-dodecanol, ethylene glycol, diethylene glycol, or triethylene glycol.

Before the crystallization, the excess aromatic monoalcohol or the organic solvent may be distilled off by distillation before crystallization. In addition, bisphenol A forms a co-crystal with phenol when crystallized in the presence of phenol. When degrading a polycarbonate resin containing a repeating unit derived from bisphenol A (repeating unit wherein in the above general formula (1), $R^1$ to $R^4$ are each a hydrogen atom, and $R^5$ and $R^6$ are each a methyl group) by using phenol, it is necessary to distill off phenol before crystallization in order not to form a co-crystal.

In addition, as described above, in the degradation method of the present invention, a dialkyl carbonate and/or an alkyl aryl carbonate are generated in addition to a bisphenol as degradation products. The method for producing a bisphenol according to the present invention by using the degradation method of the present invention may include a step of recovering these degradation products.

That is, the method for producing a bisphenol according to the present invention may include a dialkyl carbonate recovery step of recovering a dialkyl carbonate obtained in the degradation step. The recovery of a dialkyl carbonate can be carried out in the same manner as the recovery of a dialkyl carbonate in the method for producing a dialkyl carbonate described later.

In addition, the method for producing a bisphenol according to the present invention may include an alkyl aryl carbonate recovery step of recovering an alkyl aryl carbonate obtained in the degradation step. The recovery of an alkyl aryl carbonate can be carried out in the same manner as the recovery of an alkyl aryl carbonate in the method for producing an alkyl aryl carbonate described later.

Hereinafter, the method for producing a bisphenol according to the present invention will be described more specifically by taking methods (A) to (C) for producing a bisphenol shown in Figure 2 to Figure 4 as examples. In the methods (A) to (C) for producing a bisphenol, as typical examples, phenol is used as the aromatic monoalcohol, methanol is used as the aliphatic monoalcohol, and a polycarbonate resin containing a repeating unit derived from bisphenol A (bisphenol A type polycarbonate resin) is used as the polycarbonate resin.

<Method (A) for producing bisphenol>

The method (A) for producing a bisphenol shown in Figure 2 includes: a step (A1) of, in a slurry-like reaction liquid including a bisphenol A polycarbonate resin, phenol, methanol, and an alkali metal hydroxide as a catalyst, degrading the polycarbonate resin; a step (A2) of neutralizing the reaction liquid after the step (A1) to obtain an organic phase in which bisphenol A is dissolved; and a step (A3) of depressurizing and/or heating the organic phase obtained in the step (A2) and then recovering bisphenol A by crystallization.

In the method (A) for producing a bisphenol according to the present invention, the step (A1) is a degradation step, and the step (A2) and the step (A3) are a bisphenol recovery step.

In the step (A2), the reaction liquid, an acid, and water are mixed and neutralized, then oil water separation is carried out, and the separated aqueous phase is removed. The alkali metal hydroxide and the like (the alkali metal hydroxide, the acid added for neutralization, a salt generated by the neutralization) are included in the aqueous phase, and thus if the aqueous phase is removed, the alkali metal hydroxide and the like can be removed. Thereby, an organic phase in which the generated bisphenol A is dissolved is obtained.

Examples of the acid used for neutralization include hydrochloric acid, sulfuric acid, and phosphoric acid. In the neutralization by mixing an acid, the pH of the reaction liquid may be less than 7 or more than pH 7, and when the pH is less than 7, the quality of bisphenol A isolated may decrease. Because of this, it is preferable to mix an acid such that the end point is where the pH of the reaction liquid is more than 7 (for example, pH 7.5 or more or pH 8.0 or more). On the other hand, when the pH of the reaction liquid is too high, dimethyl carbonate and/or methyl carbonate is likely to be hydrolyzed, and the yield of dimethyl carbonate and/or methyl carbonate when recovering the same decreases, and thus the acid is mixed such that the pH is 10 or less, and the pH is preferably 9.5 or less.

In addition, an organic solvent such as an aromatic hydrocarbon may be mixed before or after mixing an acid. An acid, water, and an organic solvent are mixed into the reaction liquid to neutralize the reaction liquid, then oil water separation thereof is carried out, and the aqueous phase is removed to obtain an organic phase in which bisphenol A is dissolved. By mixing an organic solvent, it becomes easy to carry out oil water separation, and thus it becomes easier to remove the aqueous phase in which the alkali metal hydroxide and the like are dissolved.

In the step (A3), the organic phase obtained in the step (A2) is depressurized and/or heated, and then bisphenol A is recovered by crystallization. When bisphenol A is present at the time of crystallization, bisphenol A forms a co-crystal with phenol and the co-crystal precipitates, and thus in order to obtain bisphenol A, phenol is removed before

crystallization in the step (A3). Specifically, the organic phase obtained in the step (A2) is depressurized and/or heated to distill off a liquid component such as phenol, methanol, or dimethyl carbonate to obtain a crude product of bisphenol A. Next, an organic solvent such as an aromatic hydrocarbon is added to the crude product of bisphenol A to prepare a solution for crystallization in which bisphenol A is dissolved, and then this is cooled to precipitate bisphenol A. The precipitated bisphenol A is recovered by solid liquid separation.

When methanol is used such that the mole ratio of methanol to 1 mol of the repeating unit of the polycarbonate resin is 2.0 or more, a reaction that degrades the bisphenol A type polycarbonate resin into 2,2-bis(4-hydroxyphenyl)propane (bisphenol A) and dimethyl carbonate preferentially occurs. In this case, as shown in Figure 5, in the step (A3), first, an azeotropic mixture of dimethyl carbonate and methanol is distilled off from the organic phase obtained in the step (A2). Next, phenol is distilled off. For example, under normal pressure (101 kPa), the organic phase obtained in step (A2) is transferred to a distillation apparatus, then the temperature is raised to 65 to 250°C (preferably 90 to 200°C) and/or the pressure is reduced to 0.1 to 100 kPa (preferably 10 to 100 kPa), and thereby an azeotropic mixture of methanol and dimethyl carbonate is distilled off, and next, phenol is distilled off. Phenol is distilled off, and an organic solvent is added to the resulting crude product of bisphenol A to carry out crystallization to obtain bisphenol A.

When methanol is used such that the mole ratio of methanol to 1 mol of the repeating unit of the polycarbonate resin is less than 2.0, a large amount of methyl phenyl carbonate is generated as a degradation product. In this case, as shown in Figure 6, in the step (A3), first, an azeotropic mixture of dimethyl carbonate and methanol is distilled off from the organic phase obtained in the step (A2). Next, phenol is distilled off, and further, methyl phenyl carbonate is distilled off. For example, under normal pressure (101 kPa), the organic phase obtained in step (A2) is transferred to a distillation apparatus, then the temperature is raised to 65 to 200°C and/or the pressure is reduced to 0.1 to 50 kPa, and thereby first, an azeotropic mixture of methanol and dimethyl carbonate is distilled off, next, phenol is distilled off, and further, methyl phenyl carbonate is distilled off. An organic solvent is added to the resulting crude product of bisphenol A to carry out crystallization to obtain bisphenol A.

In addition, even when an alkali metal carbonate is used as the catalyst, it is possible to carry out the same method as the method (A) for producing a bisphenol.

<Method (B) for producing bisphenol>

The method (B) for producing a bisphenol shown in Figure 3 includes: a step (B1) of, in a slurry-like reaction liquid including a bisphenol A type polycarbonate resin, phenol, methanol, and an alkylamine as a catalyst, degrading the polycarbonate resin; and a step (B2) of depressurizing and/or heating the reaction liquid after the step (B1) and then recovering bisphenol A by crystallization.

In the method (B) for producing a bisphenol according to the present invention, the step (B1) is a degradation step, and the step (B2) is a bisphenol recovery step.

In the step (B2), specifically, the reaction liquid after the step (B1) is depressurized and/or heated to distill off a liquid component such as the alkylamine, phenol, methanol, or dimethyl carbonate to obtain a crude product of bisphenol A. Next, an organic solvent such as an aromatic hydrocarbon is added to the crude product of bisphenol A to prepare a solution for crystallization in which bisphenol A is dissolved, and then this is cooled to precipitate bisphenol A. The precipitated bisphenol A is recovered by solid liquid separation.

In the step (B2), the pressure and the temperature at which the liquid component is distilled off are controlled in the same manner as in the step (A3) of the method (A) for producing a bisphenol, depending on the amount of methanol based on that of the polycarbonate resin.

When methanol is used such that the mole ratio of methanol to 1 mol of the repeating unit of the polycarbonate resin is 2.0 or more, an azeotropic mixture of dimethyl carbonate and methanol, the alkylamine, and phenol are distilled off from the reaction liquid after the step (B1) to obtain a crude product of bisphenol A. The azeotropic mixture of dimethyl carbonate and methanol, the alkylamine, and phenol are distilled off in ascending order of boiling point. For example, when the boiling point of the azeotropic mixture of dimethyl carbonate and methanol < the boiling point of the alkylamine < the boiling point of phenol, first the azeotropic mixture of dimethyl carbonate and methanol is distilled off, next the alkylamine is distilled off, and finally phenol is distilled off, as shown in Figure 7.

When methanol is used such that the mole ratio of methanol to 1 mol of the repeating unit of the polycarbonate resin is less than 2.0, an azeotropic mixture of dimethyl carbonate and methanol, the alkylamine, phenol, and methyl phenyl carbonate are distilled off from the reaction liquid after the step (B1) to obtain a crude product of bisphenol A. The azeotropic mixture of dimethyl carbonate and methanol, the alkylamine, phenol, and methyl phenyl carbonate are distilled off in ascending order of boiling point. For example, when the boiling point of the azeotropic mixture of dimethyl carbonate and methanol < the boiling point of the alkylamine < the boiling point of phenol < methyl phenyl carbonate, first the azeotropic mixture of dimethyl carbonate and methanol is distilled off, next the alkylamine is distilled off, next, phenol is distilled off, and finally methyl phenyl carbonate is distilled off, as shown in Figure 8.

When an alkylamine is used as the catalyst, the alkylamine may be removed by a method involving supplying

an acid for neutralization. In this case, as in the step (A2) of the method (A) for producing a bisphenol, an acid and water are mixed into the reaction liquid after the degradation reaction to neutralize the reaction liquid, and then oil water separation thereof is carried out, and the aqueous phase is removed to obtain an organic phase in which bisphenol A is dissolved. Next, as in the step (A3) of the method (A) for producing a bisphenol, the obtained organic phase is depressurized and/or heated, and bisphenol A can be recovered by crystallization.

As described above, examples of the method for removing the alkylamine from the degradation reaction liquid of the polycarbonate resin include a method involving distilling off the alkylamine and a method involving supplying an acid for neutralization, however, in the method involving supplying an acid for neutralization, an ammonium salt is generated and also needs to be removed, and thus the method involving distilling off the alkylamine is preferable. By using an alkylamine as the catalyst, the alkylamine can be removed together with phenol by depressurization and/or heating, and the neutralization is not essential, and thus the purification can be simplified.

<Method (C) for producing bisphenol>

The method (C) for producing a bisphenol shown in Figure 4 includes: a step (C1) of, in a slurry-like reaction liquid including a bisphenol A polycarbonate resin, phenol, methanol, and an acid as a catalyst, degrading the polycarbonate resin; a step (C2) of neutralizing the reaction liquid after the step (C1) to obtain an organic phase in which bisphenol A is dissolved; and a step (C3) of depressurizing and/or heating the organic phase obtained in the step (C2) and then recovering bisphenol A by crystallization.

In the method (C) for producing a bisphenol according to the present invention, the step (C1) is a degradation step, and the step (C2) and the step (C3) are a bisphenol recovery step.

In the step (C2), the reaction liquid, a base, and water are mixed and neutralized, then oil water separation is carried out, and the separated aqueous phase is removed to obtain an organic phase in which bisphenol A is dissolved. In addition, an organic phase in which bisphenol A is dissolved may be obtained by carrying out oil water separation of a mixed liquid of the reaction liquid, a base, water, and an organic solvent and removing the aqueous phase.

Examples of the base used for neutralization include sodium carbonate and sodium hydroxide. As in the step (A2) of the method (A) for producing a bisphenol, the neutralization is preferably carried out such that the end point is where the pH of the reaction liquid is more than 7. For example, it is preferable to mix a base such that the pH is 7.5 or more or the pH is 8.0 or more. In addition, it is preferable to mix a base such that the pH is 10 or less or pH 9.5 or less.

In the step (C3), bisphenol A is recovered from the organic phase in which bisphenol A is dissolved obtained in the step (C2). As in the step (A3) of the method (A) for producing a bisphenol, the organic phase obtained in the step (C2) is depressurized and/or heated, and then bisphenol A can be recovered by crystallization.

In addition, as does the organic phase obtained in the step (A2), the organic phase obtained in the step (C2) includes bisphenol A, methanol, phenol, and dimethyl carbonate and/or methyl phenyl carbonate, depending on the amount of methanol used in the step (C1). The method for distilling off methanol, phenol, and dimethyl carbonate and/or methyl phenyl carbonate from this organic phase can be the same as in the step (A3).

The methods (A) to (C) for producing a bisphenol are examples in which phenol is used as the aromatic monoalcohol, and when an aromatic monoalcohol other than phenol such as a cresol or a xylenol is used as the aromatic monoalcohol, bisphenol A does not form a co-crystal, and thus it is not essential to remove the aromatic monoalcohol by depressurization and/or heating in the step (A3), the step (B2), and the step (C3). In this case, bisphenol A can be recovered by cooling the reaction liquid after the step (B1) or the organic phase obtained in the step (A2) or the step (C2) to precipitate bisphenol A. Purification of bisphenol A can be simplified by using a cresol or a xylenol.

In addition, bisphenol A may be recovered as a co-crystal of bisphenol A and phenol. In this case, bisphenol A is recovered by cooling the reaction liquid after the step (B1) or the organic phase obtained in the step (A2) or the step (C2) without distilling off phenol to precipitate a co-crystal of bisphenol A and phenol.

In addition, as described above, the polycarbonate resin used in the method for producing a bisphenol according to the present invention is not limited to the bisphenol A type polycarbonate resin. The method for producing a bisphenol according to the present invention by using a polycarbonate resin containing a repeating unit derived from a bisphenol other than bisphenol A can also be appropriately carried out in the same manner as in the above methods (A) to (C) for producing a bisphenol.

<Application of bisphenol>

The bisphenol obtained by the method for producing a bisphenol according to the present invention (hereinafter, sometimes referred to as a "recycled bisphenol") can be used as a constituent component of various thermoplastic resins such as a polyether resin, a polyester resin, a polyarylate resin, a polycarbonate resin, a polyurethane resin, or an acrylic resin, various thermosetting resins such as an epoxy resin, an unsaturated polyester resin, a phenol resin, a polybenzoxazine resin, or a cyanate resin, which are used in various applications such as an optical material, a recording material,

an insulating material, a transparent material, an electronic material, an adhesive material, or a heat resistant material, or the like, a curing agent, an additive, a precursor thereof, or the like. In addition, the bisphenol is also useful as an additive such as a color developer for a heat sensitive recording material or the like, a fading inhibitor, a microbicidal agent, or an antimicrobial or antifungal agent.

The bisphenol is preferably used as a raw material (monomer) for a thermoplastic resin or a thermosetting resin among these because it can impart a good mechanical physical property, and more preferably used as a raw material for a polycarbonate resin or an epoxy resin among such resins. In addition, the bisphenol is also preferably used as a color developer, and is more preferably used particularly in combination with a leuco dye or a discoloration temperature adjusting agent.

<Method for producing dialkyl carbonate>

The present invention relates to a method for producing a dialkyl carbonate, including: a degradation step of degrading a polycarbonate resin by using the degradation method of the present invention; and a dialkyl carbonate recovery step of recovering a dialkyl carbonate generated by the degradation of the polycarbonate resin (hereinafter, sometimes referred to as the "method for producing a dialkyl carbonate according to the present invention"). That is, the method for producing a dialkyl carbonate according to the present invention includes: a degradation step of, in a slurry-like reaction liquid including a polycarbonate resin, an aromatic monoalcohol, an aliphatic monoalcohol, and a catalyst, degrading the polycarbonate resin; and a dialkyl carbonate recovery step of recovering a dialkyl carbonate generated in the degradation step. In addition, the method for producing a dialkyl carbonate according to the present invention can include: a preparation step of preparing a slurry-like reaction liquid including a polycarbonate resin, an aromatic monoalcohol, an aliphatic monoalcohol, and a catalyst; a degradation reaction step of degrading the polycarbonate resin in the slurry-like reaction liquid prepared in the preparation step; and a dialkyl carbonate recovery step of recovering a dialkyl carbonate generated in the degradation reaction step.

As described above, a dialkyl carbonate can be generated as a degradation product in the degradation method of the present invention, and thus the degradation method of the present invention can be used in the method for producing a dialkyl carbonate. The degradation step is as described in the degradation method of the present invention.

Above all, the method for producing a dialkyl carbonate according to the present invention is suitable as a method for producing dimethyl carbonate, diethyl carbonate, or dibutyl carbonate.

In order to more efficiently produce a dialkyl carbonate, the mole ratio of the aliphatic monoalcohol to 1 mol of the repeating unit of the polycarbonate resin is preferably 2.0 or more, more preferably 2.1 or more, and further preferably 2.2 or more. In addition, the upper limit thereof is preferably 6.0 or less, more preferably 5.5 or less, and further preferably 5.0 or less from the viewpoint of production efficiency.

<Dialkyl carbonate recovery step>

The recovery (isolation, purification) of a dialkyl carbonate can be carried out by a conventional method. Examples thereof include a method involving neutralizing the reaction liquid or the reaction liquid and next heating and/or depressurizing and distilling an organic phase after neutralization. The distillation can be carried out, for example, under the condition(s) of reducing the pressure to 50 to 100 kPa and/or raising the temperature to 65 to 120°C.

Dimethyl carbonate and methanol form an azeotrope, and thus as a method for recovering dimethyl carbonate, for example, a method involving distilling the reaction liquid or an organic phase after neutralizing the reaction liquid to obtain a mixture of dimethyl carbonate and methanol, and then adding water to this mixture of dimethyl carbonate and methanol to extract methanol into an aqueous phase and remove the aqueous phase is preferable.

Figures 9 and 10 are each a flow diagram showing an example of the method for producing a diaryl carbonate according to the present invention. In the methods (D) and (E) for producing a dialkyl carbonate shown in Figures 9 and 10, as typical examples, phenol is used as the aromatic monoalcohol, methanol is used as the aliphatic monoalcohol, and a bisphenol A type polycarbonate resin is used as the polycarbonate resin.

The method (D) for producing a dialkyl carbonate shown in Figure 9 includes: a step (D1) of, in a slurry-like reaction liquid including a bisphenol A type polycarbonate resin, phenol, methanol, and an alkali metal hydroxide as a catalyst, degrading the polycarbonate resin; a step (D2) of neutralizing the reaction liquid after the step (D1) to obtain an organic phase including bisphenol A, dimethyl carbonate, methanol, and phenol; a step (D3) of distilling the organic phase obtained in the step (D2) to obtain an azeotropic mixture of dimethyl carbonate and methanol (D3); and a step (D4) of adding water to the azeotropic mixture to extract methanol into an aqueous phase and recovering dimethyl carbonate.

In the method (D) for producing a dialkyl carbonate shown in Figure 9, the step (D4) is applied to the azeotropic mixture of dimethyl carbonate and methanol distilled out in the step (A3) of the method (A) for producing a bisphenol shown in Figure 5 and Figure 6. The azeotropic mixture of dimethyl carbonate and methanol obtained in the step (D3)

of Figure 9 is the same as the azeotropic mixture of dimethyl carbonate and methanol distilled out in the step (A3) of the method (A) for producing a bisphenol shown in Figure 5 and Figure 6. In addition, further, phenol and the like are distilled off from the bottoms remaining without evaporation in the step (D3), and crystallization is carried out to obtain bisphenol A. The steps (D1) and (D2) of Figure 9 can be carried out in the same manner as the steps (A1) and (A2) of the method (A) for producing a bisphenol shown in Figure 2.

In addition, in the method (D) for producing a dialkyl carbonate, an acid may be used as the catalyst and a base may be used for neutralization. In this case, the azeotropic mixture of dimethyl carbonate and methanol obtained in the step (D3) is the same as the azeotropic mixture of dimethyl carbonate and methanol distilled out in the step (C3) of the method (C) for producing a bisphenol.

The method (E) for producing a dialkyl carbonate shown in Figure 10 includes: a step (E1) of, in a slurry-like reaction liquid including a bisphenol A type polycarbonate resin, phenol, methanol, and an alkylamine as a catalyst, degrading the polycarbonate resin; a step (E2) of distilling the reaction liquid after the step (E1) to obtain an azeotropic mixture of dimethyl carbonate and methanol; and a step (E3) of adding water to the azeotropic mixture to extract methanol into an aqueous phase and recovering dimethyl carbonate.

In the method (E) for producing a dialkyl carbonate shown in Figure 10, the step (E3) is applied to the azeotropic mixture of dimethyl carbonate and methanol distilled out in the step (B2) of the method (B) for producing a bisphenol shown in Figure 7 and Figure 8. The azeotropic mixture of dimethyl carbonate and methanol obtained in the step (E2) of Figure 10 is the same as the azeotropic mixture of dimethyl carbonate and methanol distilled out in the step (B2) of the method (B) for producing a bisphenol shown in Figure 7 and Figure 8. In addition, further, phenol and the like are distilled off from the bottoms remaining without evaporation in the step (E2), and crystallization is carried out to obtain bisphenol A. The step (E1) of Figure 10 can be carried out in the same manner as the step (B1) of the method (B) for producing a bisphenol shown in Figure 3.

<Method for producing alkyl aryl carbonate>

The present invention relates to a method for producing an alkyl aryl carbonate, including: a degradation step of degrading a polycarbonate resin in the presence of an aromatic monoalcohol, an aliphatic monoalcohol, and a catalyst; and an alkyl aryl carbonate recovery step of recovering an alkyl aryl carbonate generated by the degradation of the polycarbonate resin (hereinafter, sometimes referred to as the "method for producing an alkyl aryl carbonate according to the present invention").

In the method for producing an alkyl aryl carbonate according to the present invention, in a reaction liquid including a polycarbonate resin, an aromatic monoalcohol, an aliphatic monoalcohol, and a catalyst the polycarbonate resin may be degraded in the degradation step, and this is the same as in the degradation method of the present invention except that it is not essential for the reaction liquid to be slurry-like.

The reaction liquid may be slurry-like, and the degradation step may be a step of degrading the polycarbonate resin by using the degradation method of the present invention. That is, the method for producing an alkyl aryl carbonate according to the present invention may include: a degradation step of, in a slurry-like reaction liquid including a polycarbonate resin, an aromatic monoalcohol, an aliphatic monoalcohol, and a catalyst, degrading the polycarbonate resin; and an alkyl aryl carbonate recovery step of recovering an alkyl aryl carbonate generated in the degradation step. In addition, the method for producing an alkyl aryl carbonate according to the present invention may include: a preparation step of preparing a slurry-like reaction liquid including a polycarbonate resin, an aromatic monoalcohol, an aliphatic monoalcohol, and a catalyst; a degradation reaction step of degrading the polycarbonate resin in the slurry-like reaction liquid prepared in the preparation step; and an alkyl aryl carbonate recovery step of recovering an alkyl aryl carbonate generated in the degradation reaction step.

By controlling the amount of the aliphatic monoalcohol based on that of the polycarbonate resin, an alkyl aryl carbonate can be generated as a degradation product. The degradation step is as described in the degradation method of the present invention.

Above all, the method for producing an alkyl aryl carbonate according to the present invention is suitable as a method for producing an alkyl phenyl carbonate. That is, it is preferable to use phenol as the aromatic monoalcohol used for degrading the polycarbonate resin.

In order to more efficiently produce an alkyl aryl carbonate, the mole ratio of the aliphatic monoalcohol to 1 mol of the repeating unit of the polycarbonate resin is preferably less than 2.0, and may be 1.95 or less, 1.9 or less, 1.85 or less, 1.8 or less, or the like. In addition, the lower limit thereof is preferably 0.1 or more, more preferably 0.5 or more, and further preferably 1.0 or more from the viewpoint of production efficiency.

<Alkyl aryl carbonate recovery step>

The recovery (isolation, purification) of an alkyl aryl carbonate can be carried out by a conventional method.

Examples thereof include a method involving neutralizing the reaction liquid or the reaction liquid and next heating and/or depressurizing and distilling an organic phase after neutralization. The distillation can be carried out, for example, under the condition(s) of reducing the pressure to 0.1 to 50 kPa and/or raising the temperature to 100 to 200°C.

Figures 11 and 12 are each a flow diagram showing an example of the method for producing an alkyl aryl carbonate according to the present invention. In the methods (F) and (G) for producing an alkyl aryl carbonate shown in Figures 11 and 12, as typical examples, phenol is used as the aromatic monoalcohol, methanol is used as the aliphatic monoalcohol, and a bisphenol A type polycarbonate resin is used as the polycarbonate resin.

The method (F) for producing an alkyl aryl carbonate shown in Figure 11 includes: a step (F1) of, in a slurry-like reaction liquid including a bisphenol A type polycarbonate resin, phenol, methanol, and an alkali metal hydroxide as a catalyst, degrading the polycarbonate resin; a step (F2) of neutralizing the reaction liquid after the step (F1) to obtain an organic phase including bisphenol A, dimethyl carbonate, methyl phenyl carbonate, methanol, and phenol; and a step (F3) of distilling the organic phase obtained in the step (F2) to obtain methyl phenyl carbonate.

The methyl phenyl carbonate obtained in the step (F3) of Figure 11 is the same as the methyl phenyl carbonate distilled out in the step (A3) of the method (A) for producing a bisphenol shown in Figure 6. In addition, further, the bottoms remaining without evaporation in the step (F3) are subjected to crystallization to obtain bisphenol A. The steps (F1) to (F2) of Figure 11 can be carried out in the same manner as the steps (A1) and (A2) of the method (A) for producing a bisphenol shown in Figure 2.

In addition, in the method (F) for producing an alkyl aryl carbonate, an acid may be used as the catalyst and a base may be used for neutralization. In this case, the methyl phenyl carbonate obtained in the step (F3) is the same as the methyl phenyl carbonate distilled out in the step (C3) of the method (C) for producing a bisphenol.

The method (G) for producing an alkyl aryl carbonate shown in Figure 12 includes: a step (G1) of, in a slurry-like reaction liquid including a bisphenol A type polycarbonate resin, phenol, methanol, and an alkylamine as a catalyst, degrading the polycarbonate resin; and a step (G2) of distilling the reaction liquid after the step (G1) to obtain methyl phenyl carbonate.

The methyl phenyl carbonate obtained in the step (G2) of the method (G) for producing a dialkyl carbonate shown in Figure 12 is the same as the methyl phenyl carbonate distilled out in the step (B2) of the method (B) for producing a bisphenol shown in Figure 8. In addition, further, the bottoms remaining without evaporation in the step (G2) are subjected to crystallization to obtain bisphenol A. The step (G1) of Figure 12 can be carried out in the same manner as the step (B1) of the method (B) for producing a bisphenol shown in Figure 3.

The methods (F) and (G) for producing an alkyl aryl carbonate degrade the polycarbonate resin in a slurry-like reaction liquid, and in the method for producing an alkyl aryl carbonate according to the present invention, the polycarbonate resin may be able to be degraded in the presence of an aromatic monoalcohol, an aliphatic monoalcohol, and a catalyst, and it is not essential for the reaction liquid to be slurry-like.

<Method for producing diaryl carbonate according to the present invention>

The method for producing a diaryl carbonate according to the present invention is a method for producing a diaryl carbonate by using a dialkyl carbonate obtained by the method for producing a dialkyl carbonate according to the present invention (hereinafter, sometimes referred to as a "recycled dialkyl carbonate") or an alkyl aryl carbonate obtained by the method for producing an alkyl aryl carbonate according to the present invention (hereinafter, sometimes referred to as a "recycled alkyl aryl carbonate"), as at least part of a raw material. For example, the method for producing a diaryl carbonate according to the present invention can be a method for producing diphenyl carbonate.

Hereinafter, the method for producing a diaryl carbonate by using a recycled dialkyl carbonate will be described as a "first method for producing a diaryl carbonate," and the method for producing a diaryl carbonate by using a recycled diaryl carbonate will be described as a "second method for producing a diaryl carbonate." In addition, the "first method for producing a diaryl carbonate" and the "second method for producing a diaryl carbonate" are collectively referred as "the method for producing a diaryl carbonate according to the present invention."

<First method for producing diaryl carbonate>

The first method for producing a diaryl carbonate is a method for producing a diaryl carbonate, including producing a diaryl carbonate by using a dialkyl carbonate obtained by the method for producing a dialkyl carbonate according to the present invention (recycled dialkyl carbonate).

The first method for producing a diaryl carbonate may be any method therefor involving using a dialkyl carbonate including a recycled dialkyl carbonate as a raw material. For the production of a diaryl carbonate, a known method for producing a diaryl carbonate from a dialkyl carbonate (for example, Japanese Patent Laid-Open No. 3-291257) can be used.

For example, a diaryl carbonate can be produced by a method involving using a dialkyl carbonate and an

aromatic monoalcohol as raw materials, subjecting these to an ester exchange reaction to obtain an alkyl aryl carbonate (the following reaction equation (3a)), and then subjecting the alkyl aryl carbonate to a disproportionation reaction to obtain a diaryl carbonate (the following reaction equation (3b)).

[Formula 5]

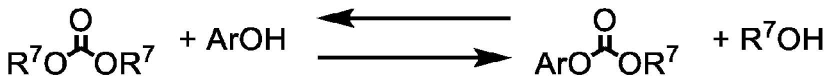

···(3a)

[Formula 6]

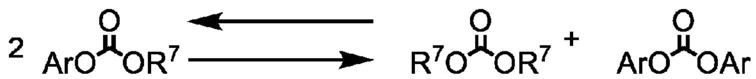

···(3b)

In the equation (3a) and the equation (3b), $R^7$ represents an alkyl group and Ar represents an aryl group.

As an aromatic monoalcohol (ArOH) that can be used in the reaction of the reaction equation (3a), the same aromatic monoalcohol as that used in the degradation reaction of the present invention can be used. The aromatic monoalcohol used in the above reaction equation (3a) is preferably phenol.

As a catalyst used in the reactions of the reaction equation (3a) and the reaction equation (3b), a known catalyst used for producing a diaryl carbonate can be used. For example, an organotitanium catalyst such as tetraphenoxytitanium can be used.

As the raw material dialkyl carbonate, at least part thereof may be a recycled dialkyl carbonate, only a recycled dialkyl carbonate may be used, or a mixture obtained by mixing a recycled dialkyl carbonate with a general dialkyl carbonate that is not a recycled dialkyl carbonate may be used. The amount of the recycled dialkyl carbonate is not particularly limited. From the viewpoint of consideration for the environment, the amount of the recycled dialkyl carbonate in the raw material dialkyl carbonate is preferably 50% by mass or more, and more preferably 70% by mass or more, 80% by mass or more, or 90% by mass or more, where a larger value is more preferable.

<Second method for producing diaryl carbonate>

The second method for producing a diaryl carbonate is a method for producing a diaryl carbonate, including producing a diaryl carbonate by using an alkyl aryl carbonate obtained by the method for producing an alkyl aryl carbonate according to the present invention (recycled alkyl aryl carbonate).

The second method for producing a diaryl carbonate may be any method therefor involving using an alkyl aryl carbonate including a recycled alkyl aryl carbonate as a raw material. For the production of a diaryl carbonate, a known method for producing a diaryl carbonate from an alkyl aryl carbonate can be used.

For example, a diaryl carbonate can be obtained by the reaction of the reaction equation (3b) in the first method for producing a diaryl carbonate.

As the raw material alkyl aryl carbonate, at least part thereof may be a recycled alkyl aryl carbonate, only a recycled alkyl aryl carbonate may be used, or a mixture obtained by mixing a recycled alkyl aryl carbonate with a general alkyl aryl carbonate that is not a recycled alkyl aryl carbonate may be used. The amount of the recycled alkyl aryl carbonate is not particularly limited. From the viewpoint of consideration for the environment, the amount of the recycled alkyl aryl carbonate in the raw material alkyl aryl carbonate is preferably 50% by mass or more, and more preferably 70% by mass or more, 80% by mass or more, or 90% by mass or more, where a larger value is more preferable.

The reaction of giving an alkyl aryl carbonate and an aliphatic monoalcohol from a dialkyl carbonate and an aromatic monoalcohol is an equilibrium reaction, and the equilibrium thereof is extremely biased toward the raw material system. Further, this reaction has a slow reaction rate. Because of these, it is preferable to produce a diaryl carbonate by using the second method for producing a diaryl carbonate by using an alkyl aryl carbonate obtained by the method for producing an alkyl aryl carbonate according to the present invention.

<Method for producing recycled polycarbonate resin according to the present invention>

The method for producing a recycled polycarbonate resin according to the present invention is a method for producing a recycled polycarbonate resin by using a diaryl carbonate raw material including a bisphenol raw material including a bisphenol (recycled bisphenol) obtained by the method for producing a bisphenol according to the present

invention, or a diaryl carbonate obtained by the method for producing a diaryl carbonate according to the present invention (recycled diaryl carbonate ).

Hereinafter, the method for producing a recycled polycarbonate resin by using a bisphenol raw material including a recycled bisphenol will be described as a "first method for producing a recycled polycarbonate resin," and the method for producing a recycled polycarbonate resin by using a diaryl carbonate raw material including a recycled diaryl carbonate will be described as a "second method for producing a recycled polycarbonate resin." In addition, the "first method for producing a recycled polycarbonate resin" and the "second method for producing a recycled polycarbonate resin" are collectively referred as "the method for producing a recycled polycarbonate resin according to the present invention."

<First method for producing recycled polycarbonate resin>

The first method for producing a recycled polycarbonate resin is a method for producing a recycled polycarbonate resin, including producing a recycled polycarbonate resin by using a bisphenol raw material including a bisphenol (recycled bisphenol) obtained by the method for producing a bisphenol according to the present invention. The first method for producing a recycled polycarbonate resin uses a chemical recycling method involving producing a polycarbonate resin by using, as a raw material, a recycled bisphenol obtained by degrading a polycarbonate resin included in a waste plastic or the like into a bisphenol as a monomer.

As the first method for producing a polycarbonate resin, a known method for polymerizing a polycarbonate resin can be appropriately selected and carried out except that a bisphenol raw material including a recycled bisphenol is used as the bisphenol. A polycarbonate resin is generally produced by polymerizing a bisphenol and a carbonic acid diester in the presence of a catalyst.

In the first method for producing a recycled polycarbonate resin, the recycled polycarbonate resin can be obtained, for example, by polymerizing a bisphenol raw material including a recycled bisphenol (bisphenol obtained by the method for producing a bisphenol according to the present invention) and a carbonic acid diester raw material.

The recycled polycarbonate resin can be produced, for example, by a method involving subjecting a bisphenol raw material including a recycled bisphenol and a carbonic acid diester raw material such as diphenyl carbonate to an ester exchange reaction in the presence of an alkali metal compound and/or an alkaline earth metal compound.

The recycled bisphenol may be used as the whole of the bisphenol raw material, or may be mixed with a general bisphenol that is not a recycled bisphenol and used as part of the bisphenol raw material. The amount of the recycled bisphenol is not particularly limited, and is any amount such as 0.1% by mass or more, 1% by mass or more, 10% by mass or more, 20% by mass or more, 30% by mass or more, 40% by mass or more, 50% by mass or more, 70% by mass or more, 80% by mass or more, or 90% by mass or more. A larger proportion of the recycled bisphenol is more environmentally friendly, and thus, from the viewpoint of consideration for the environment, the amount of the recycled bisphenol based on that of the bisphenol raw material is preferably large.

As the carbonic acid diester raw material, a diaryl carbonate can be used. This diaryl carbonate may include a recycled diaryl carbonate, or may use only a general diaryl carbonate and include no recycled diaryl carbonate.

The ester exchange reaction can be carried out by appropriately selecting a known method, and an example of a method using diphenyl carbonate as the carbonic acid diester raw material will be described below.

In the first method for producing a recycled polycarbonate resin, it is preferable to use an excess amount of diphenyl carbonate based on that of the bisphenol raw material. The amount of diphenyl carbonate used based on that of the bisphenol raw material is preferably large from the viewpoint of the produced recycled polycarbonate resin having few terminal hydroxyl groups and being excellent in thermal stability of the polymer, and is preferably small from the viewpoint of the ester exchange reaction rate being fast and it being easy to produce a recycled polycarbonate resin having a desired molecular weight. Because of these, the amount of diphenyl carbonate used per mol of the bisphenol raw material is usually 1.001 mol or more, preferably 1.002 mol or more, and usually 1.3 mol or less, preferably 1.2 mol or less.

As a method for supplying a raw material, a bisphenol raw material and diphenyl carbonate can also be supplied in a solid state, and it is preferable to melt one or both thereof and supply the same in a liquid state.

When producing a recycled polycarbonate resin by an ester exchange reaction between diphenyl carbonate and a bisphenol raw material, an ester exchange catalyst is usually used. As this ester exchange catalyst, an alkali metal compound and/or an alkaline earth metal compound is preferably used. One of these may be used, or two or more thereof may be used in any combination and at any ratio. Practically, it is desirable to use an alkali metal compound.

The amount of the catalyst used per mol of the bisphenol raw material or diphenyl carbonate is usually 0.05 μmol or more, preferably 0.08 μmol or more, and further preferably 0.10 μmol or more, in addition, the amount thereof is usually 100 μmol or less, preferably 50 μmol or less, and further preferably 20 μmol or less.

When the amount of the catalyst used is within such a range, it is easy to obtain a polymerization activity required for producing a recycled polycarbonate resin having a desired molecular weight, and it is easy to obtain a polycarbonate resin having an excellent polymer color hue, a low level of excessive polymer branching, and excellent

fluidity during the molding.

In order to produce a recycled polycarbonate resin by the above method, it is preferable to continuously supply both of the above raw materials to a raw material mixing tank and continuously supply the resulting mixture and an ester exchange catalyst to a polymerization tank.

In the production of a recycled polycarbonate resin by the ester exchange method, both of the raw materials supplied to the raw material mixing tank are usually stirred uniformly and then supplied to the polymerization tank to which a catalyst is added, to produce a polymer.

<Second method for producing recycled polycarbonate resin>

The second method for producing a recycled polycarbonate resin is a method for producing a recycled polycarbonate resin, including producing a recycled polycarbonate resin by using a diaryl carbonate raw material including a diaryl carbonate (recycled diaryl carbonate) obtained by the method for producing a diaryl carbonate according to the present invention.

The second method for producing a recycled polycarbonate resin uses a diaryl carbonate raw material including a diaryl carbonate (recycled diaryl carbonate) produced by using a dialkyl carbonate and/or an alkyl aryl carbonate generated by degradation of a polycarbonate resin included in a waste plastic or the like. As described above, a polycarbonate resin is generally produced by polymerizing a bisphenol raw material and a carbonic acid diester raw material in the presence of a catalyst. As the second method for producing a polycarbonate resin, a known method for polymerizing a polycarbonate resin can be appropriately selected and carried out except that a diaryl carbonate raw material including a recycled diaryl carbonate is used as the carbonic acid diester raw material.

The recycled diaryl carbonate may be used as the whole of the diaryl carbonate raw material, or may be mixed with a general diaryl carbonate that is not a diaryl carbonate and used as part of the diaryl carbonate raw material. The amount of the recycled diaryl carbonate is not particularly limited, and is any amount such as 0.1% by mass or more, 1% by mass or more, 10% by mass or more, 20% by mass or more, 30% by mass or more, 40% by mass or more, 50% by mass or more, 70% by mass or more, 80% by mass or more, or 90% by mass or more. A larger proportion of the recycled diaryl carbonate is more environmentally friendly, and thus, from the viewpoint of consideration for the environment, the amount of the recycled diaryl carbonate based on that of the diaryl carbonate raw material is preferably large.

Specifically, the recycled polycarbonate resin can be obtained by polymerizing a diaryl carbonate raw material including a recycled diaryl carbonate (recycled diaryl carbonate obtained by the method for producing a diaryl carbonate according to the present invention) and a bisphenol raw material. In addition, the bisphenol raw material may include a recycled bisphenol, or may use only a general bisphenol and include no recycled bisphenol.

The polymerization method, the mixing ratio of the raw materials, the amount of the catalyst, the supply method, and the like are the same as in the first method for producing a recycled polycarbonate resin. In the first method for producing a recycled polycarbonate resin, a bisphenol raw material including a recycled bisphenol is used as the bisphenol raw material, and as the bisphenol raw material used in the second method for producing a recycled polycarbonate resin, a bisphenol raw material including a recycled bisphenol raw material may be used, or a general bisphenol raw material including no recycled bisphenol raw material may be used.

(Recycled polycarbonate resin and composition thereof)

The recycled polycarbonate resin obtained by the method for producing a recycled polycarbonate resin according to the present invention may be used as it is, or may be used as a recycled polycarbonate resin composition including an unused polycarbonate resin and the recycled polycarbonate resin. The recycled polycarbonate resin composition can be obtained by appropriately selecting a known kneading method or the like and mixing an unused polycarbonate resin and the recycled polycarbonate resin. When the recycled polycarbonate resin composition including an unused polycarbonate resin and the recycled polycarbonate resin is prepared, the amount of the recycled polycarbonate resin is not particularly limited, and a larger proportion of the recycled polycarbonate resin is more environmentally friendly. Because of this, from the viewpoint of consideration for the environment, the amount of the recycled polycarbonate resin based on that of the recycled polycarbonate resin composition is preferably 50% by mass or more, and more preferably 70% by mass or more, 80% by mass or more, or 90% by mass or more, where a larger value is more preferable.

The obtained recycled polycarbonate resin or composition thereof can be molded and processed into various molded articles such as an optical member or an optical recording medium as can an unused polycarbonate resin.

<Method for producing epoxy resin>

The present invention relates to a method for producing an epoxy resin, including producing an epoxy resin by

using a bisphenol obtained by the method for producing a bisphenol according to the present invention. In addition, the obtained epoxy resin may be further reacted with a polyhydroxy compound raw material to produce an epoxy resin.

As described above, the method for producing an epoxy resin according to the present invention is a method for producing an epoxy resin by using a recycled bisphenol and/or an epoxy resin produced by using a recycled bisphenol, as at least part of a raw material. The method for producing an epoxy resin according to the present invention is not particularly limited except that a recycled bisphenol (bisphenol obtained by the method for producing a bisphenol according to the present invention) and/or an epoxy resin produced by using a recycled bisphenol is used as a raw material, and a known method for producing an epoxy resin can be used. For example, the recycled bisphenol can be used as at least part of a polyhydroxy compound raw material when an epoxy resin is produced by using a one-stage method, an oxidation method, or a two-stage method, as will be described later. The obtained epoxy resin can also be used as at least part of the epoxy resin raw material when an epoxy resin is produced by using a two-stage method.

The "epoxy resin raw material" means an epoxy resin used as a raw material for an epoxy resin obtained by the method for producing an epoxy resin according to the present invention (hereinafter, sometimes referred to as a "recycled epoxy resin"). The "polyhydroxy compound" is a general term for a dihydric or higher polyhydric phenol compound and a dihydric or higher polyhydric alcohol compound, and the "polyhydroxy compound raw material" means a polyhydroxy compound used as a raw material for a recycled epoxy resin.

As the method for producing an epoxy resin according to the present invention, a one-stage method, an oxidation method, a two-stage method, or the like can be used.

The method for producing an epoxy resin by the one-stage method is a method involving using a recycled bisphenol (bisphenol obtained by the method for producing a bisphenol according to the present invention) and reacting the same with an epihalohydrin to obtain an epoxy resin.

The method for producing an epoxy resin by the oxidation method is a method involving allylating a recycled bisphenol by using an allyl halide (allyl chloride, allyl bromide, or the like) and then subjecting the resulting allylated product to an oxidation reaction to obtain an epoxy resin.

The method for producing an epoxy resin by the two-stage method is a method involving reacting an epoxy resin raw material with a polyhydroxy compound raw material, and a recycled bisphenol and/or an epoxy resin produced by using a recycled bisphenol is used as a raw material.

Hereinafter, the methods for producing an epoxy resin by using the one-stage method, the oxidation method, and the two-stage method will be described.

(Method for producing epoxy resin by one-stage method)

The method for producing an epoxy resin by the one-stage method is not particularly limited as long as it is a known production method, and will be described in detail below.

In the method for producing an epoxy resin by the one-stage method, an epoxy resin may be produced by using a recycled bisphenol in combination with a polyhydroxy compound other than the recycled bisphenol (hereinafter, sometimes referred to as a "further polyhydroxy compound"). That is, the method for producing an epoxy resin by the one-stage method is a method involving reacting a polyhydroxy compound raw material with an epihalohydrin to obtain an epoxy resin, and can be a method in which at least part of the polyhydroxy compound raw material is a recycled bisphenol.

The content of the recycled bisphenol in the polyhydroxy compound raw material is not particularly limited, and is preferably 1 to 100% by mass, and more preferably 10 to 100% by mass because a high content of the recycled bisphenol is environmentally friendly.

Here, the "further polyhydroxy compound" is a general term for a dihydric or higher polyhydric phenol compound and a dihydric or higher polyhydric alcohol compound excluding the recycled bisphenol. In the method for producing an epoxy resin by the one-stage method, the "polyhydroxy compound raw material" refers to all polyhydroxy compounds that collectively include a recycled bisphenol and a further polyhydroxy compound used if necessary.

Examples of the further polyhydroxy compound include various polyhydric phenols such as bisphenol A, tetramethyl bisphenol A, bisphenol F, tetramethyl bisphenol F, bisphenol S, bisphenol C, bisphenol AD, bisphenol AF, hydroquinone, resorcin, methylresorcin, biphenol, tetramethyl biphenol, dihydroxynaphthalene, dihydroxydiphenyl ether, a thiodiphenol, a phenol novolac resin, a cresol novolac resin, a phenol aralkyl resin, a biphenyl aralkyl resin, a naphthol aralkyl resin, a terpene phenol resin, a dicyclopentadiene phenol resin, a bisphenol A novolac resin, a naphthol novolac resin, brominated bisphenol A, or a brominated phenol novolac resin, polyhydric phenol resins obtained by condensation reactions between various phenols and various aldehydes such as benzaldehyde, hydroxybenzaldehyde, crotonaldehyde, or glyoxal, polyhydric phenol resins obtained by condensation reactions between xylene resins and phenols, various phenol resins such as a cocondensed resin of heavy oil or a pitch, a phenol, and a formaldehyde, a chain aliphatic diol such as ethylene glycol, trimethylene glycol, propylene glycol, 1,3-butanediol, 1,4-butanediol, 1,3-pentanediol, 1,4-pentanediol, 1,5-pentanediol, or 1,6-hexanediol, a cyclic aliphatic diol such as cyclohexanediol or cyclodecanediol, and

a polyalkylene ether glycol such as polyethylene ether glycol, polyoxytrimethylene ether glycol, or polypropylene ether glycol.

At the time of reaction, the polyhydroxy compound raw material is dissolved in an epihalohydrin to form a uniform solution. As the epihalohydrin, epichlorohydrin or epibromohydrin is usually used, and in the present invention, epichlorohydrin is preferable.

The amount of the epihalohydrin used is usually an amount corresponding to 1.0 to 14.0 equivalents, and particularly preferably an amount corresponding to 2.0 to 10.0 equivalents, per equivalent of the hydroxyl group of the polyhydroxy compound raw material (all polyhydroxy compounds). When the amount of the epihalohydrin is equal to or greater than the above lower limit, it is easy to control the molecular weight increasing reaction, and the epoxy resin obtained can have an appropriate epoxy equivalent, and thus such an amount is preferable. On the other hand, when the amount of the epihalohydrin is less than or equal to the above upper limit, the production efficiency tends to be improved, and thus such an amount is preferable.

Next, while stirring the above solution, an alkali metal hydroxide in an amount corresponding to usually 0.1 to 3.0 equivalents, preferably 0.8 to 2.0 equivalents, per equivalent of the hydroxyl group of the polyhydroxy compound raw material is added in the form of a solid or an aqueous solution to cause a reaction. When the amount of the alkali metal hydroxide added is equal to or greater than the above lower limit, it is difficult for the unreacted hydroxyl group and the generated epoxy resin to react with each other and it is easy to control the molecular weight increasing reaction, and thus such an amount is preferable. In addition, when the amount of the alkali metal hydroxide added is less than or equal to the above upper limit, it is difficult for an impurity due to a side reaction to be generated, and thus such an amount is preferable. Examples of the alkali metal hydroxide used here usually include sodium hydroxide or potassium hydroxide.

This reaction can be carried out under normal pressure or under reduced pressure, and the reaction temperature is preferably 20 to 200°C, and more preferably 40 to 150°C. When the reaction temperature is equal to or greater than the above lower limit, it is easy to allow the reaction to proceed and it is easy to control the reaction, and thus such a reaction temperature is preferable. In addition, when the reaction temperature is less than or equal to the above upper limit, it is difficult for a side reaction to proceed and it is particularly easy to reduce the amount of the polymer, and thus such a reaction temperature is preferable.

In addition, this reaction is carried out while carrying out dehydration by a method involving azeotroping the reaction liquid while holding a predetermined temperature if necessary, cooling the volatilized vapor, carrying out oil/water separation of the obtained condensate liquid, and returning the oil resulting from the removal of water to the reaction system. The alkali metal hydroxide is added intermittently or continuously in small amounts over preferably 0.1 to 24 hours, more preferably 0.5 to 10 hours, in order to suppress a rapid reaction. When the addition time of the alkali metal hydroxide is equal to or greater than the above lower limit, it is possible to prevent rapid progress of the reaction, and it is easy to control the reaction temperature, and thus such an addition time is preferable. When the addition time is less than or equal to the above upper limit, it is easy to reduce the amount of the polymer, and thus such an addition time is preferable.

After the reaction is completed, the insoluble by-produced salt can be removed by filtration or removed by washing with water, and then the unreacted epihalohydrin can be distilled off and removed by heating and/or distilling off under reduced pressure.

In addition, in this reaction, a catalyst such as a quaternary ammonium salt such as tetramethylammonium chloride or tetraethylammonium bromide, a tertiary amine such as benzyldimethylamine or 2,4,6-tris(dimethylaminomethyl) phenol, an imidazole such as 2-ethyl-4-methylimidazole or 2-phenylimidazole, a phosphonium salt such as ethyltriphenylphosphonium iodide, or a phosphine such as triphenylphosphine may be used.

Further, in this reaction, an inert organic solvent such as an alcohol such as ethanol or isopropanol, a ketone such as acetone, methyl ethyl ketone, or methyl isobutyl ketone, an ether such as dioxane or ethylene glycol dimethyl ether, a glycol ether such as methoxypropanol, or an aprotic polar solvent such as dimethyl sulfoxide or dimethylformamide may be used.

[Production of epoxy resin having reduced total chlorine content]

When it is necessary to reduce the total chlorine content of the epoxy resin thus obtained, it is possible to produce an epoxy resin having a total chlorine content reduced by reaction with an alkali.

An organic solvent for dissolving the epoxy resin may be used for the reaction with an alkali. The organic solvent used for the reaction is not particularly limited, and it is preferable to use a ketone-based organic solvent from the viewpoint of production efficiency, handleability, workability, or the like. In addition, from the viewpoint of further reducing the amount of hydrolyzable chlorine, an aprotic polar solvent may be used.

Examples of the ketone-based organic solvent include a ketone-based solvent such as methyl ethyl ketone, methyl isobutyl ketone, or cyclohexanone. Methyl isobutyl ketone is particularly preferable because of the effect, ease

of a post-treatment, or the like. These may be used singly or as a mixture of two or more.

Examples of the aprotic polar solvent include dimethyl sulfoxide, diethyl sulfoxide, dimethyl sulfone, sulfolane, dimethylformamide, dimethylacetamide, and hexamethylphosphoramide. These may be used singly or as a mixture of two or more. Among these aprotic polar solvents, dimethyl sulfoxide is preferable because it is easily available and has an excellent effect.

The amount of such a solvent used is an amount that allows the concentration of the epoxy resin in a liquid to be treated with an alkali to be usually 1 to 95% by mass, and preferably 5 to 80% by mass.

As the alkali, a solid or a solution of an alkali metal hydroxide can be used. Examples of the alkali metal hydroxide include potassium hydroxide and sodium hydroxide, and sodium hydroxide is preferable. In addition, as the alkali metal hydroxide, one dissolved in an organic solvent or water may be used. Preferably, the alkali metal hydroxide is used as a solution obtained by dissolving the same in an aqueous solvent or an organic solvent.

The amount of the alkali metal hydroxide used is preferably 0.01 to 20.0 parts by mass or less per 100 parts by mass of the epoxy resin in terms of the solid content of the alkali metal hydroxide. The amount thereof is more preferably 0.10 to 10.0 parts by mass. When the amount of the alkali metal hydroxide used is less than or equal to the above lower limit, the effect of reducing the total chlorine content is low, and when the amount thereof is equal to or greater than the above upper limit, a large amount of the polymer is generated and thus the yield is lowered.

The reaction temperature is preferably 20 to 200°C, and more preferably 40 to 150°C, and the reaction time is preferably 0.1 to 24 hours, and more preferably 0.5 to 10 hours.

After the reaction, the excess alkali metal hydroxide and the by-produced salt can be removed by a method such as washing with water, and further the organic solvent can be removed by distilling off under heating and/or reduced pressure and/or steam distillation.

(Method for producing epoxy resin by oxidation method)

The method for producing an epoxy resin by the oxidation method is not particularly limited as long as it is a known production method, and can be carried out, for example, according to a method disclosed in Japanese Patent Laid-Open No. 2011-225711, Japanese Patent Laid-Open No. 2012-092247, Japanese Patent Laid-Open No. 2012-111858, or the like.

Even in the method for producing an epoxy resin by the oxidation method, as for the one-stage method, an epoxy resin may be produced by using a recycled bisphenol in combination with a further polyhydroxy compound other than the recycled bisphenol. That is, the method for producing an epoxy resin by the oxidation method is a method involving allylating a polyhydroxy compound raw material by using an allyl halide and then subjecting the resulting allylated product to an oxidation reaction to obtain an epoxy resin, and can be a method in which at least part of the polyhydroxy compound raw material is a recycled bisphenol.

In the method for producing an epoxy resin by the oxidation method, the "polyhydroxy compound raw material" refers to all polyhydroxy compounds that collectively include a recycled bisphenol and a further polyhydroxy compound used if necessary, and examples of the further polyhydroxy compound include the same ones as for the one-stage method. The content of the recycled bisphenol in the polyhydroxy compound raw material is not particularly limited, and is preferably 1 to 100% by mass, and more preferably 10 to 100% by mass because a high content of the recycled bisphenol is environmentally friendly.

(Method for producing epoxy resin by two-stage method)

The method for producing an epoxy resin by the two-stage method is not particularly limited as long as it is a known production method, and will be described in detail below.

The method for producing an epoxy resin by the two-stage method can be a method including a step of reacting an epoxy resin raw material with a polyhydroxy compound raw material, wherein at least part of the epoxy resin raw material is an epoxy resin produced by using a recycled bisphenol, and/or at least part of the polyhydroxy compound raw material is a recycled bisphenol.

That is, the method for producing an epoxy resin by the two-stage method is any of the following methods (i) to (iii).

Method (i): A method involving reacting a further epoxy resin other than an epoxy resin produced by using a recycled bisphenol with a polyhydroxy compound raw material including a recycled bisphenol

In the method (i), the epoxy resin raw material is a further epoxy resin other than an epoxy resin produced by using a recycled bisphenol. In addition, the "polyhydroxy compound raw material" refers to all polyhydroxy compounds that collectively include a recycled bisphenol and a further polyhydroxy compound used if necessary.

Method (ii): A method involving reacting an epoxy resin raw material including an epoxy resin produced by using a recycled bisphenol with a polyhydroxy compound raw material including a recycled bisphenol

In the method (ii), the epoxy resin raw material is all epoxy resins that collectively include an epoxy resin

produced by using a recycled bisphenol and a further epoxy resin used if necessary. In addition, the "polyhydroxy compound raw material" refers to all polyhydroxy compounds that collectively include a recycled bisphenol and a further polyhydroxy compound used if necessary.

Method (iii): A method involving reacting an epoxy resin raw material including an epoxy resin produced using a recycled bisphenol with a further polyhydroxy compound raw material other than a recycled bisphenol

In the method (iii), the epoxy resin raw material is all epoxy resins that collectively include an epoxy resin produced by using a recycled bisphenol and a further epoxy resin used if necessary. The polyhydroxy compound raw material is a further polyhydroxy compound other than a recycled bisphenol.

The epoxy resin produced by using a recycled bisphenol used in the method (ii) and the method (iii) can be obtained by the method for producing an epoxy resin by the one-stage method or the method for producing an epoxy resin by the oxidation method. In addition, the epoxy resin obtained by the method (i) may be used. The further epoxy resin other than an epoxy resin produced by using a recycled bisphenol is the same as the further epoxy resin described later in the method for producing an epoxy resin cured product, and the further polyhydroxy compound is the same as for the one-stage method.

In the method (i) and the method (ii), in the polyhydroxy compound raw material including a recycled bisphenol, the content of the recycled bisphenol is not particularly limited, and is preferably 1 to 100% by mass, and more preferably 10 to 100% by mass because a high content of the recycled bisphenol is environmentally friendly.

In addition, in the method (ii) and the method (iii), in the epoxy resin raw material including an epoxy resin produced by using a recycled bisphenol, the content of the epoxy resin produced by using a recycled bisphenol is not particularly limited, and is preferably 1 to 100% by mass, and more preferably 10 to 100% by mass because a high content of the epoxy resin produced by using a recycled bisphenol is environmentally friendly.

In the reaction by the two-stage method, the amounts of the epoxy resin raw material and the polyhydroxy compound raw material used are preferably such that the blending equivalent ratio is (epoxy group equivalent):(hydroxyl group equivalent) = 1:0.1 to 2.0. The blending equivalent ratio is more preferably 1:0.2 to 1.2. When this equivalent ratio is within such a range, it is easy to increase the molecular weight, and it is possible to leave more epoxy group ends, and thus such an equivalent ratio is preferable.

In addition, a catalyst may be used in the reaction by the two-stage method, and the catalyst may be any compound as long as the compound has a catalytic ability to promote the reaction between an epoxy group and a phenolic hydroxyl group or an alcoholic hydroxyl group. Examples thereof include an alkali metal compound, an organophosphorus compound, a tertiary amine, a quaternary ammonium salt, a cyclic amine, and an imidazole. Among these, a quaternary ammonium salt is preferable. In addition, only one catalyst can be used, or two or more can be used in combination. The amount of the catalyst used is usually 0.001 to 10% by mass based on that of the epoxy resin raw material.

In addition, in the reaction by the two-stage method, a solvent may be used, and the solvent may be any solvent as long as this solvent dissolves the epoxy resin raw material. Examples thereof include an aromatic solvent, a ketone-based solvent, an amide-based solvent, and a glycol ether-based solvent. Only one solvent may be used, or two or more can be used in combination. In addition, the resin concentration of the solvent is preferably 10 to 95% by mass. The resin concentration is more preferably 20 to 80% by mass. In addition, when a highly viscous product is generated in the middle of the reaction, the solvent can also be additionally added to continue the reaction. After completion of the reaction, the solvent can, if necessary, be removed or further added.

In the reaction by the two-stage method, the reaction temperature is preferably 20 to 250°C, and more preferably 50 to 200°C. When the reaction temperature is equal to or greater than the above upper limit, the epoxy resin generated may deteriorate. In addition, when the reaction temperature is less than or equal to the above lower limit, the reaction may not proceed sufficiently. In addition, the reaction time is usually 0.1 to 24 hours, and preferably 0.5 to 12 hours.

<Method for producing epoxy resin cured product>

The present invention relates to a method for producing an epoxy resin cured product, including curing an epoxy resin composition including an epoxy resin obtained by the method for producing an epoxy resin according to the present invention and a curing agent to obtain an epoxy resin cured product. In the method for producing an epoxy resin cured product according to the present invention, an epoxy resin obtained by the above method for producing an epoxy resin according to the present invention is mixed with a curing agent to obtain a composition including the epoxy resin and the curing agent (hereinafter, sometimes referred to as an "epoxy resin composition"), and then the epoxy resin composition is cured to obtain an epoxy resin cured product.

In addition, if necessary, a further epoxy resin other than the epoxy resin obtained by the method for producing an epoxy resin according to the present invention (hereinafter, sometimes simply referred to as a "further epoxy resin"),

a curing agent, a curing accelerator, an inorganic filler, a coupling agent, or the like can be appropriately blended into the epoxy resin composition.

The content of the recycled epoxy resin in the epoxy resin composition is not particularly limited. A high content of the recycled epoxy resin is environmentally friendly, and thus the recycled epoxy resin is preferably 40 parts by mass or more, and more preferably 60 parts by mass or more per 100 parts by mass of all epoxy resin components in the recycled epoxy resin composition. When a further epoxy resin is included, the recycled epoxy resin can be 40 to 99 parts by mass, 60 to 99 parts by mass, or the like per 100 parts by mass of all epoxy resin components in the epoxy resin composition. "All epoxy resin components" correspond to the amount of all epoxy resins included in the epoxy resin composition, and is the total amount of the recycled epoxy resin and a further epoxy resin used if needed.

(Curing agent)

In the present invention, the curing agent refers to a substance that contributes to the crosslinking reaction and/or the chain length extension reaction between the epoxy groups of an epoxy resin. In the present invention, even if a substance is usually referred to as a "curing accelerator," when the substance is a substance that contributes to the crosslinking reaction and/or the chain length extension reaction between the epoxy groups of an epoxy resin, the substance is regarded as a curing agent.

In the epoxy resin composition, the content of the curing agent is preferably 0.1 to 1000 parts by mass per 100 parts by mass of all epoxy resin components. In addition, the content thereof is more preferably 500 parts by mass or less.

The curing agent is not particularly limited, and all generally known epoxy resin curing agents can be used. Examples thereof include a phenol-based curing agent, an amine-based curing agent such an aliphatic amine, a polyether amine, an alicyclic amine, or an aromatic amine, an acid anhydride-based curing agent, an amide-based curing agent, a tertiary amine, and an imidazole. Such curing agents may be used singly or in combinations of two or more. When two or more curing agents are used in combination, they may be mixed in advance to prepare a mixed curing agent before use, or when mixing each component of an epoxy resin obtained by the method for producing an epoxy resin according to the present invention and a further epoxy resin, each component of the curing agent may be added separately to mix these components at the same time.

[Phenol-based curing agent]

Specific examples of the phenol-based curing agent include various polyhydric phenols such as a recycled bisphenol, bisphenol A, tetramethyl bisphenol A, bisphenol F, tetramethyl bisphenol F, bisphenol C, bisphenol S, bisphenol AD, bisphenol AF, hydroquinone, resorcin, methylresorcin, biphenol, tetramethyl biphenol, dihydroxynaphthalene, dihydroxydiphenyl ether, a thiodiphenol, a phenol novolac resin, a cresol novolac resin, a phenol aralkyl resin, a biphenyl aralkyl resin, a naphthol aralkyl resin, terpene phenol resin, a dicyclopentadiene phenol resin, a bisphenol A novolac resin, a trisphenol methane type resin, a naphthol novolac resin, brominated bisphenol A, or a brominated phenol novolac resin, polyhydric phenol resins obtained by condensation reactions between various phenols and various aldehydes such as benzaldehyde, hydroxybenzaldehyde, crotonaldehyde, or glyoxal, polyhydric phenol resins obtained by condensation reactions between xylene resins and phenols, and various phenol resins such as a cocondensed resin of heavy oil or a pitch, a phenol, and a formaldehyde, a phenol/benzaldehyde/xylylene dimethoxide polycondensate, a phenol/benzaldehyde/xylylene dihalide polycondensate, a phenol/benzaldehyde/4,4'-dimethoxide biphenyl polycondensate, or a phenol/benzaldehyde/4,4'-dihalide biphenyl polycondensate.

Only one of these phenol-based curing agents may be used, or two or more thereof may be used in any combination and at any blending ratio.

The amount of the phenol-based curing agent blended is preferably 0.1 to 1000 parts by mass, and more preferably 500 parts by mass or less per 100 parts by mass of all epoxy resin components in the epoxy resin composition.

[Amine-based curing agent]

Examples of the amine-based curing agent (excluding a tertiary amine) include an aliphatic amine, a polyether amine, an alicyclic amine, and an aromatic amine.

Examples of the aliphatic amine include ethylenediamine, 1,3-diaminopropane, 1,4-diaminopropane, hexamethylenediamine, 2,5-dimethylhexamethylenediamine, trimethylhexamethylenediamine, diethylenetriamine, iminobispropylamine, bis(hexamethylene)triamine, triethylenetetramine, tetraethylenepentamine, pentaethylenehexamine, N-hydroxyethylethylenediamine, and tetra(hydroxyethyl)ethylenediamine.

Examples of the polyether amine include triethylene glycol diamine, tetraethylene glycol diamine, diethylene glycol bis(propylamine), polyoxypropylenediamine, and a polyoxypropylenetriamine.

Examples of the alicyclic amine include isophoronediamine, metasendiamine, N-aminoethylpiperazine, bis(4-amino-3-methyldicyclohexyl)methane, bis(aminomethyl)cyclohexane, 3,9-bis(3-aminopropyl)-2,4,8,10-tetraoxaspiro(5,5)undecane, and norbornenediamine.

Examples of the aromatic amine include tetrachloro-p-xylenediamine, m-xylenediamine, p-xylenediamine, m-phenylenediamine, o-phenylenediamine, p-phenylenediamine, 2,4-diaminoanisol, 2,4-toluenediamine, 2,4-diaminodiphenylmethane, 4,4'-diaminodiphenylmethane, 4,4'-diamino-1,2-diphenylethane, 2,4-diaminodiphenylsulfone, 4,4'-diaminodiphenylsulfone, m-aminophenol, m-aminobenzylamine, benzyldimethylamine, 2-(dimethylaminomethyl)phenol, triethanolamine, methylbenzylamine, α-(m-aminophenyl)ethylamine, α-(p-aminophenyl)ethylamine, diaminodiethyldimethyldiphenylmethane, and α,α'-bis(4-aminophenyl)-p-diisopropylbenzene.

Only one of the amine-based curing agents listed above may be used, or two or more thereof may be used in any combination and at any blending ratio.

It is preferable to use such an amine-based curing agent such that the equivalent ratio of the functional group in the curing agent to the epoxy group in all epoxy resin components included in the epoxy resin composition is in the range of 0.1 to 2.0. The equivalent ratio is more preferably in the range of 0.8 to 1.2. When the equivalent ratio is within such a range, an unreacted epoxy group and a functional group of the curing agent are unlikely to remain, and thus such an equivalent ratio is preferable.

[Tertiary amine]

Examples of the tertiary amine include 1,8-diazabicyclo(5,4,0)undecene-7, triethylenediamine, benzyldimethylamine, triethanolamine, dimethylaminoethanol, and tris(dimethylaminomethyl)phenol.

Only one of the tertiary amines listed above may be used, or two or more thereof may be used in any combination and at any blending ratio.

It is preferable to use such a tertiary amine such that the equivalent ratio of the functional group in the curing agent to the epoxy group in all epoxy resin components included in the epoxy resin composition is in the range of 0.1 to 2.0. The equivalent ratio is more preferably in the range of 0.8 to 1.2. When the equivalent ratio is within such a range, an unreacted epoxy group and a functional group of the curing agent are unlikely to remain, and thus such an equivalent ratio is preferable.

[Acid anhydride-based curing agent]

Examples of the acid anhydride-based curing agent include an acid anhydride and a modified product of an acid anhydride.

Examples of the acid anhydride include phthalic anhydride, trimellitic anhydride, pyromellitic anhydride, benzophenonetetracarboxylic anhydride, dodecenylsuccinic anhydride, polyadipic anhydride, polyazelaic anhydride, polysebacic anhydride, poly(ethyloctadecanedioic) anhydride, poly(phenylhexadecanedioic) anhydride, tetrahydrophthalic anhydride, methyltetrahydrophthalic anhydride, methylhexahydrophthalic anhydride, hexahydrophthalic anhydride, methylhimic anhydride, trialkyltetrahydrophthalic anhydride, methylcyclohexenedicarboxylic anhydride, methylcyclohexenetetracarboxylic anhydride, ethylene glycol bistrimellitate dianhydride, HET anhydride, nadic anhydride, methylnadic anhydride, 5-(2,5-dioxotetrahydro-3-furanyl)-3-methyl-3-cyclohexane-1,2-dicarboxylic anhydride, 3,4-dicarboxy-1,2,3,4-tetrahydro-1-naphthalenesuccinic dianhydride, and 1-methyl-dicarboxy-1,2,3,4-tetrahydro-1-naphthalenesuccinic dianhydride.

Examples of the modified product of an acid anhydride include products obtained by modifying the above acid anhydrides with glycol. Here, examples of the glycol that can be used for the modification include an alkylene glycol such as ethylene glycol, propylene glycol, or neopentyl glycol, and a polyether glycol such as polyethylene glycol, polypropylene glycol, or polytetramethylene ether glycol. Further, a copolymerized polyether glycol of two or more of these glycols and/or a polyether glycol can also be used.

Only one of the acid anhydride-based curing agents listed above may be used, or two or more thereof may be used in any combination and at any blending ratio.

When an acid anhydride-based curing agent is used, it is preferable to use the acid anhydride-based curing agent such that the equivalent ratio of the functional group in the curing agent to the epoxy group in all epoxy resin components in the epoxy resin composition is in the range of 0.1 to 2.0. The equivalent ratio is more preferably in the range of 0.8 to 1.2. When the equivalent ratio is within such a range, an unreacted epoxy group and a functional group of the curing agent are unlikely to remain, and thus such an equivalent ratio is preferable.

[Amide-based curing agent]

Examples of the amide-based curing agent include dicyandiamide and a derivative thereof, and a polyamide resin.

Only one of the amide-based curing agents may be used, or two or more thereof may be mixed and used in any combination and at any ratio.

When an amide-based curing agent is used, it is preferable to use the amide-based curing agent such that the amount of the amide-based curing agent is 0.1 to 20% by mass based on the total amount of all epoxy resin components in the epoxy resin composition and the amide-based curing agent.

[Imidazole]

Examples of the imidazole include 2-phenylimidazole, 2-ethyl-4(5)-methylimidazole, 2-phenyl-4-methylimidazole, 1-benzyl-2-methylimidazole, 1-benzyl-2-phenylimidazole, 1-cyanoethyl-2-undecylimidazole, 1-cyano-2-phenylimidazole, 1-cyanoethyl-2-undecylimidazole trimellitate, 1-cyanoethyl-2-phenylimidazolium trimellitate, 2,4-diamino-6-[2'-methylimidazolyl-(1')]-ethyl-s-triazine, 2,4-diamino-6-[2'-ethyl-4'-methylimidazolyl-(1')]-ethyl-s-triazine, a 2,4-diamino-6-[2'-methylimidazolyl-(1')]-ethyl-s-triazine isocyanuric acid adduct, a 2-phenylimidazole isocyanuric acid adduct, 2-phenyl-4,5-dihydroxymethylimidazole, 2-phenyl-4-methyl-5-hydroxymethylimidazole, and adducts of an epoxy resin and the above imidazoles. An imidazole has a catalytic ability, and thus can also be generally classified as a curing accelerator, but in the present invention, an imidazole is classified as a curing agent.

Only one of the imidazoles listed above may be used, or two or more thereof may be mixed and used in any combination and at any ratio.

When an imidazole is used, it is preferable to use the imidazole such that the amount of the imidazole is 0.1 to 20% by mass based on the total amount of all epoxy resin components in the epoxy resin composition and the imidazole.

[Further curing agent]

In the epoxy resin composition, a further curing agent can be used in addition to the curing agent. The further curing agent that can be used in the epoxy resin composition is not particularly limited, and all generally known curing agents for an epoxy resin can be used.

Only one of these further curing agents may be used, or two or more thereof may be used in combination.

(Further epoxy resin)

The epoxy resin composition can include a further epoxy resin other than the epoxy resin obtained by the method for producing an epoxy resin according to the present invention. By including a further epoxy resin, various physical properties can be improved.

The further epoxy resin that can be used in the epoxy resin composition corresponds to all epoxy resins other than the epoxy resin obtained by the method for producing an epoxy resin according to the present invention. Specific examples include a bisphenol A type epoxy resin, a bisphenol C type epoxy resin, a trisphenol methane type epoxy resin, an anthracene type epoxy resin, a phenol-modified xylene resin type epoxy resin, a bisphenol cyclododecyl type epoxy resin, a bisphenol diisopropylidene resorcin type epoxy resin, a bisphenol F type epoxy resin, a bisphenol AD type epoxy resin, a bisphenol AF type epoxy resin, a hydroquinone type epoxy resin, a methylhydroquinone type epoxy resin, a dibutylhydroquinone type epoxy resin, a resorcin type epoxy resin, a methylresorcin type epoxy resin, a biphenol type epoxy resin, a tetramethyl biphenol type epoxy resin, a tetramethyl bisphenol F type epoxy resin, a dihydroxydiphenyl ether type epoxy resin, an epoxy resin derived from a thiodiphenol, a dihydroxynaphthalene type epoxy resin, a dihydroxyanthracene type epoxy resin, a dihydroxydihydroanthracene type epoxy resin, a dicyclopentadiene type epoxy resin, an epoxy resin derived from a dihydroxystilbene, a phenol novolac type epoxy resin, a cresol novolac type epoxy resin, a bisphenol A novolac type epoxy resin, a naphthol novolac type epoxy resin, a phenol aralkyl type epoxy resin, a naphthol aralkyl type epoxy resin, a biphenyl aralkyl type epoxy resin, a terpene phenol type epoxy resin, a dicyclopentadiene phenol type epoxy resin, an epoxy resin derived from a condensate of phenol/hydroxybenzaldehyde, an epoxy resin derived from a condensate of phenol/crotonaldehyde, an epoxy resin derived from a condensate of phenol/glyoxal, an epoxy resin derived from a cocondensed resin of heavy oil or a pitch, a phenol, and a formaldehyde, an epoxy resin derived from diaminodiphenylmethane, an epoxy resin derived from aminophenol, an epoxy resin derived from xylenediamine, an epoxy resin derived from methylhexahydrophthalic acid, and an epoxy resin derived from a dimer acid. Only one of these may be used, or two or more thereof may be used in any combination and at any blending ratio.

When the epoxy resin composition includes a further epoxy resin as described above, the content thereof is preferably 1 to 60 parts by mass, and more preferably 40 parts by mass or less per 100 parts by mass of all epoxy resin components in the composition.

(Curing accelerator)

The epoxy resin composition preferably includes a curing accelerator. By including a curing accelerator, it is possible to shorten the curing time and lower the curing temperature, and it is possible to easily obtain a desired cured product.

The curing accelerator is not particularly limited, and specific examples thereof include an organic phosphine, a phosphorus-based compound such as a phosphonium salt, a tetraphenylboron salt, an organic acid dihydrazide, and a boron halide amine complex.

Examples of the phosphorus-based compound that can be used as a curing accelerator include an organic phosphine such as triphenylphosphine, diphenyl(p-tolyl)phosphine, a tris(alkylphenyl)phosphine, a tris(alkoxyphenyl)phosphine, a tris(alkyl/alkoxyphenyl)phosphine, a tris(dialkylphenyl)phosphine, a tris(trialkylphenyl)phosphine, a tris(tetraalkylphenyl)phosphine, a tris(dialkoxyphenyl)phosphine, a tris(trialkoxyphenyl)phosphine, a tris(tetraalkoxyphenyl)phosphine, a trialkylphosphine, a dialkylarylphosphine, or an alkyldiarylphosphine, complexes of these organic phosphines and an organic boron compound, and compounds obtained by adding, to these organic phosphines, a compound such as maleic anhydride, a quinone compound such as 1,4-benzoquinone, 2,5-toluquinone, 1,4-naphthoquinone, 2,3-dimethylbenzoquinone, 2,6-dimethylbenzoquinone, 2,3-dimethoxy-5-methyl-1,4-benzoquinone, 2,3-dimethoxy-1,4-benzoquinone, or phenyl-1,4-benzoquinone, or diazophenylmethane.

Among the curing accelerators listed above, an organic phosphine and a phosphonium salt are preferable, and an organic phosphine is most preferable. In addition, only one of the curing accelerators listed above may be used, or two or more thereof may be mixed and used in any combination and at any ratio.

The curing accelerator is preferably used in the range of 0.1 parts by mass or more and 20 parts by mass or less per 100 parts by mass of all epoxy resin components in the epoxy resin composition. When the content of the curing accelerator is equal to or greater than the above lower limit value, it is possible to obtain a good curing acceleration effect, and on the other hand, when the content thereof is less than or equal to the above upper limit value, it is easy to obtain a desired cured physical property, and thus such a content is preferable.

(Inorganic filler)

An inorganic filler can be blended into the epoxy resin composition. Examples of the inorganic filler include fused silica, crystalline silica, a glass powder, alumina, calcium carbonate, calcium sulfate, talc, and boron nitride. Only one of these may be used, or two or more thereof may be used in any combination and at any blending ratio. The amount of the inorganic filler blended is preferably 10 to 95% by mass based on the total amount of the epoxy resin composition.

(Release agent)

A release agent can be blended into the epoxy resin composition. As the release agent, for example, a natural wax such as carnauba wax, a synthetic wax such as polyethylene wax, a higher fatty acid and a metal salt thereof such as stearic acid or zinc stearate, or a hydrocarbon-based release agent such as paraffin can be used. Only one of these may be used, or two or more thereof may be used in any combination and at any blending ratio.

The content of the release agent blended is preferably 0.001 to 10.0 parts by mass per 100 parts by mass of all epoxy resin components in the epoxy resin composition. When the amount of the release agent blended is within the above range, good releasability can be developed while maintaining a curing characteristic.

(Coupling agent)

A coupling agent can be blended into the epoxy resin composition. The coupling agent is preferably used in combination with an inorganic filler, and by blending a coupling agent, the adhesiveness between an epoxy resin as a matrix and an inorganic filler can be improved. Examples of the coupling agent include a silane coupling agent and a titanate coupling agent.

Examples of the silane coupling agent include an epoxysilane such as γ-glycidoxypropyltrimethoxysilane, γ-glycidoxypropyltriethoxysilane, or β-(3,4-epoxycyclohexyl)ethyltrimethoxysilane, an aminosilane such as γ-aminopropyltriethoxysilane, N-β(aminoethyl)γ-aminopropyltrimethoxysilane, N-β(aminoethyl)γ-aminopropylmethyldimethoxysilane, γ-aminopropyltrimethoxysilane, or γ-ureidopropyltriethoxysilane, a mercaptosilane such as 3-mercaptopropyltrimethoxysilane, a vinylsilane such as p-styryltrimethoxysilane, vinyltrichlorosilane, vinyltris(β-methoxyethoxy)silane, vinyltrimethoxysilane, vinyltriethoxysilane, or γ-methacryloxypropyltrimethoxysilane, and further, an epoxy-based, amino-based, or vinyl-based polymer type silane.

Examples of the titanate coupling agent include isopropyltriisostearoyl titanate, isopropyltri(N-aminoethyl/aminoethyl) titanate, diisopropylbis(dioctyl phosphate) titanate, tetraisopropylbis(dioctyl phosphite) titanate, tetraoctyl-

bis(ditridecyl phosphite) titanate, tetra(2,2-diallyloxymethyl-1-butyl)bis(ditridecyl) phosphite titanate, bis(dioctyl pyrophosphate) oxyacetate titanate, and bis(dioctyl pyrophosphate) ethylene titanate.

Only one of these coupling agents may be used, or two or more thereof may be mixed and used in any combination and at any ratio.

When a coupling agent is used in the epoxy resin composition, the amount thereof blended is preferably 0.001 to 10.0 parts by mass per 100 parts by mass of all epoxy resin components. When the amount of the coupling agent blended is equal to or greater than the above lower limit value, the effect of improving the adhesiveness between an epoxy resin as a matrix and an inorganic filler due to the blending of the coupling agent tends to be improved, and on the other hand, when the amount of the agent blended is less than or equal to the above upper limit value, the coupling agent is unlikely to bleed out from the cured product obtained, and thus such an amount thereof is preferable.

(Further blending component)

A component other than those described above can be blended into the epoxy resin composition. Examples of the further blending component include a flame retardant, a plasticizer, a reactive diluent, and a pigment, and these can be appropriately blended if necessary. However, this does not prevent the blending of a component other than those listed above.

Examples of the flame retardant include a halogen-based flame retardant such as a brominated epoxy resin or a brominated phenol resin, an antimony compound such as antimony trioxide, a phosphorus-based flame retardant such as red phosphorus, a phosphoric acid ester, or a phosphine, a nitrogen-based flame retardant such as a melamine derivative, and an inorganic flame retardant such as aluminum hydroxide or magnesium hydroxide.

(Curing method)

An epoxy resin cured product can be obtained by curing the epoxy resin composition. The curing method is not particularly limited, and usually, a cured product can be obtained by a thermal curing reaction by heating. At the time of the thermal curing reaction, it is preferable to appropriately select the curing temperature depending on the type of the curing agent used. For example, when a phenol-based curing agent is used, the curing temperature is usually 80 to 250°C. In addition, it is also possible to lower the curing temperature by adding a curing accelerator to these curing agents. The reaction time is preferably 0.01 to 20 hours. When the reaction time is equal to or greater than the above lower limit value, the curing reaction tends to easily proceed sufficiently, and thus such a reaction time is preferable. On the other hand, when the reaction time is less than or equal to the above upper limit value, it is easy to reduce deterioration due to heating and energy loss during the heating, and thus such a reaction time is preferable.

(Applications)

The epoxy resin cured product obtained by curing the epoxy resin composition has a low linear expansion coefficient, and a cured product having excellent thermal cracking resistance can be obtained.

Therefore, the epoxy resin cured product can be effectively used in any application as long as the application requires these physical properties. For example, the epoxy resin cured product can be suitably used for any of applications such as a coating material field such as an electrodeposition coating material for an automobile, a heavy-duty anticorrosion coating material for a ship/bridge, or a coating material for inner surface coating of a beverage can; an electrical or electronic field such as a laminated plate, a semiconductor encapsulant, an insulating powder coating material, or coil impregnation; or a civil engineering/construction/adhesive field such as seismic reinforcement of a bridge, concrete reinforcement, a floor material of a building, lining of a waterworks facility, drainage/water permeable pavement, or an adhesive for a vehicle/aircraft.

The epoxy resin composition may be used after being cured for the above applications, or may be cured in a production step in the above applications.

Examples

Hereinafter, the present invention will be described more specifically with reference to Examples and Comparative Examples, but the present invention is not limited by the following Examples as long as they do not depart from the scope of the present invention.

[Raw material and reagents]

As the polycarbonate resin, the polycarbonate resin "NOVAREX (registered trademark) M7027BF" manufactured by Mitsubishi Engineering-Plastics Corporation was used.

As phenol, toluene, sodium hydroxide, paratoluenesulfonic acid, methanol, ethanol, n-butanol, triethylamine, acetonitrile, and cesium carbonate, reagents of FUJIFILM Wako Pure Chemical Corporation were used.

As diphenyl carbonate, a product of Mitsubishi Chemical Corporation was used.

Tetraphenoxytitanium was synthesized according to the following procedure and used.

200 g (2.1 mol) of phenol and 100 mL of toluene were placed in a 500 mL three-necked flask equipped with a receiver and a distilling-out tube, and the inside of the flask was purged with a nitrogen flow. The flask was immersed in a 100°C oil bath to obtain a uniform solution. 57 g (0.2 mol) of tetraisopropoxytitanium was added thereto. When the internal temperature of the bottom of the flask was held at 100°C, distilling-out of generated i-propyl alcohol started. After that, the internal temperature was gradually raised to 116°C to distill out 80 mL of a distillate, which was a mixture of i-propyl alcohol and toluene. 50 mL of hexane was added to the obtained still residue, and then the resulting mixture was cooled to room temperature and crystallized. The precipitated red crystal was obtained by filtration and dried on a rotary evaporator equipped with an oil bath at an oil bath temperature of 140°C and a pressure of 50 Torr to obtain 60 g (0.1 mol) of tetraphenoxytitanium.

[Analysis]

The generation and purity of a bisphenol were checked by high performance liquid chromatography under the following procedure and conditions.

- Apparatus: LC-2010A manufactured by Shimadzu Corporation, Waters Corporation 5 μm 150 mm × 4.6 mm ID
- Method: Low pressure gradient method
- Analysis temperature: 40°C
- Eluent composition:

  Liquid A acetonitrile
  Liquid B a solution of 85% phosphoric acid:water = 1 mL:999 mL

At minute 0 of the analysis time, the eluent composition was liquid A:liquid B = 35:65 (volume ratio, the same applies hereinafter); at minute 0 to minute 5 of the analysis time, the eluent composition was liquid A:liquid B = 35:65; and then at minute 5 to minute 40 of the analysis time, the eluent composition was gradually changed to liquid A:liquid B = 90:10.

- Flow rate: 0.85 mL/min
- Detection wavelength: 280 nm

Analysis of dimethyl carbonate, diethyl carbonate, and dibutyl carbonate was carried out by gas chromatography under the following procedure and conditions.

- Apparatus: GC-2014 manufactured by Shimadzu Corporation Agilent DB-1 0.530 mm × 30 m 1.5 μm
- Detection method: FID
- Vaporization chamber temperature: 230°C
- Detector temperature: 300°C
- At minute 0 to minute 5 of the analysis time, the column temperature was kept at 50°C; at minute 5 to minute 30 of the analysis time, the column temperature was gradually raised to 280°C; and at minute 30 to minute 40 of the analysis time, the column temperature was maintained at 280°C.
- Quantification method: Internal standard method using biphenyl as the internal standard

[Viscosity average molecular weight (Mv)]

The viscosity average molecular weight (Mv) was calculated by dissolving a polycarbonate resin in methylene chloride (concentration of 6.0 g/L), measuring the specific viscosity (ηsp) at 20°C by using an Ubbelohde viscosity tube, and using the following expression to measure the viscosity average molecular weight (Mv).

$$\eta sp/C = [\eta]\ (1 + 0.28\ \eta sp)$$

$$[\eta] = 1.23 \times 10^{-4} Mv^{0.83}$$

[Molten color of bisphenol]

For the molten color of a bisphenol, 20 g of the bisphenol was placed in a test tube "P-24" (2 mmcp × 200 mm) manufactured by Nichiden-Rika Glass Co., Ltd., melted at 174°C for 30 minutes, and the Hazen color value thereof was measured by using "OME7700" manufactured by Nippon Denshoku Industries Co., Ltd.

[Measurement of pH]

The pH was measured by using a pH meter "pH METER ES-73" manufactured by HORIBA, Ltd. for an aqueous phase at 25°C taken out from a flask.

[Example 1]

(Degradation step)

In a jacket-type separable flask equipped with a Dimroth condenser tube, a stirring blade, and a thermometer, 80 g of the polycarbonate resin (the molecular weight of the repeating unit of the polycarbonate resin was 254 g/mol, and thus the number of moles of the repeating unit = 80 g / 254 g/mol = 0.31 mol), 240 g of phenol, 23 g of methanol (23 g / 32 g/mol = 0.72 mol, the mole ratio of methanol to 1 mol of the repeating unit of the polycarbonate resin = 0.72 mol / 0.31 mol = 2.32), and 15 g of triethylamine (15 g / 101 g/mol = 0.15 mol, the mole ratio of triethylamine to 1 mol of the repeating unit of the polycarbonate resin = 0.15 mol / 0.31 mol = 0.48) were placed at room temperature in a nitrogen atmosphere (the amount of the liquid was 80 g + 240 g + 23 g + 15 g = 358 g).

After that, the internal temperature was raised to 85°C. An undissolved polycarbonate resin was observed (it was slurry-like) in the reaction liquid when the temperature reached 85°C. The reaction was carried out for 4 hours while maintaining 85°C as it was, to obtain a uniform reaction liquid.

When the composition of part of the obtained reaction liquid was checked by high performance liquid chromatography, bisphenol A was found to have been generated in an amount of 19.5% by mass (the generation rate was 19.5 / 100 × 358 g / 228 g/mol / 0.31 mol = 99 mol%).

In addition, when the composition of part of the obtained reaction liquid was checked by gas chromatography, dimethyl carbonate was found to have been generated in an amount of 6.5% by mass (the generation rate was 6.5 / 100 × 358 g / 90 g/mol / 0.31 mol = 83 mol%).

Triethylamine was used slightly, in an amount of 15 g, for 80 g of the polycarbonate resin, and thus no amine odor was observed when part of the obtained reaction liquid was withdrawn.

[Example 2]

The same procedure as in Example 1 was carried out except that in Example 1, 32 g of triethylamine (32 g / 101 g/mol = 0.32 mol, the mole ratio of triethylamine to 1 mol of the repeating unit of the polycarbonate resin = 0.32 mol / 0.31 mol = 1.03) was used instead of 15 g of triethylamine.

When the composition of part of the obtained reaction liquid was checked by high performance liquid chromatography, bisphenol A was found to have been generated in an amount of 18.9% by mass (the generation rate was 18.9 / 100 × 375 g / 228 g/mol / 0.31 mol = 100 mol%).

In addition, when the composition of part of the obtained reaction liquid was checked by gas chromatography, dimethyl carbonate was found to have been generated in an amount of 7.2% by mass (the generation rate was 7.2 / 100 × 375 g / 90 g/mol / 0.31 mol = 97 mol%).

Triethylamine was used excessively, in an amount of 32 g, for 80 g of the polycarbonate resin, and the amine odor when part of the obtained reaction liquid was withdrawn was not a remarkable odor probably because of the strong interaction with phenol, which is an acidic substance.

[Example 3]

The same procedure as in Example 1 was carried out except that in Example 1, 63 g of triethylamine (63 g / 101 g/mol = 0.62 mol, the mole ratio of triethylamine to 1 mol of the repeating unit of the polycarbonate resin = 0.62 mol / 0.31 mol = 2.00) was used instead of 15 g of triethylamine.

When the composition of part of the obtained reaction liquid was checked by high performance liquid chromatography, bisphenol A was found to have been generated in an amount of 17.4% by mass (the generation rate was 17.4 / 100 × 406 g / 228 g/mol / 0.31 mol = 100 mol%).

In addition, when the composition of part of the obtained reaction liquid was checked by gas chromatography, dimethyl carbonate was found to have been generated in an amount of 6.6% by mass (the generation rate was 6.6 / 100 × 406 g / 90 g/mol / 0.31 mol = 96 mol%).

Triethylamine was used excessively, in an amount of 63 g, for 80 g of the polycarbonate resin, and the amine odor when part of the obtained reaction liquid was withdrawn was not a remarkable odor probably because of the strong interaction with phenol, which is an acidic substance.

[Example 4]

The same procedure as in Example 1 was carried out except that in Example 1, 110 g of triethylamine (110 g / 101 g/mol = 1.01 mol, the mole ratio of triethylamine to 1 mol of the repeating unit of the polycarbonate resin = 1.01 mol / 0.31 mol = 3.26) was used instead of 15 g of triethylamine.

When the composition of part of the obtained reaction liquid was checked by high performance liquid chromatography, bisphenol A was found to have been generated in an amount of 15.5% by mass (the generation rate was 15.5 / 100 × 453 g / 228 g/mol / 0.31 mol = 99 mol%).

In addition, when the composition of part of the obtained reaction liquid was checked by gas chromatography, dimethyl carbonate was found to have been generated in an amount of 5.9% by mass (the generation rate was 5.9 / 100 × 453 g / 90 g/mol / 0.31 mol = 96 mol%).

Triethylamine was used excessively, in an amount of 110 g, for 80 g of the polycarbonate resin, and the amine odor when part of the obtained reaction liquid was withdrawn was not as remarkable an odor as in Comparative Example 2 described later, probably because of the strong interaction with phenol, which is an acidic substance.

[Example 5]

The same procedure as in Example 1 was carried out except that in Example 1, 128 g of triethylamine (128 g / 101 g/mol = 1.27 mol, the mole ratio of triethylamine to 1 mol of the repeating unit of the polycarbonate resin = 1.27 mol / 0.31 mol = 4.10) was used instead of 15 g of triethylamine.

When the composition of part of the obtained reaction liquid was checked by high performance liquid chromatography, bisphenol A was found to have been generated in an amount of 14.9% by mass (the generation rate was 14.9 / 100 × 471 g / 228 g/mol / 0.31 mol = 99 mol%).

In addition, when the composition of part of the obtained reaction liquid was checked by gas chromatography, dimethyl carbonate was found to have been generated in an amount of 5.8% by mass (the generation rate was 5.8 / 100 × 471 g / 90 g/mol / 0.31 mol = 98 mol%).

Triethylamine was used excessively, in an amount of 128 g, for 80 g of the polycarbonate resin, and the amine odor when part of the obtained reaction liquid was withdrawn was not as remarkable an odor as in Comparative Example 2 described later, probably because of the strong interaction with phenol, which is an acidic substance.

[Example 6]

The same procedure as in Example 1 was carried out except that in Example 1, 33 g (0.72 mol) of ethanol was used instead of 23 g of methanol.

When the composition of part of the obtained reaction liquid was checked by high performance liquid chromatography, bisphenol A was found to have been generated in an amount of 17.5% by mass (the generation rate was 17.5 / 100 × 368 g / 228 g/mol / 0.31 mol = 91 mol%).

In addition, when the composition of part of the obtained reaction liquid was checked by gas chromatography, dimethyl carbonate was found to have been generated in an amount of 8.7% by mass (the generation rate was 8.7 / 100 × 368 g / 118 g/mol / 0.31 mol = 88 mol%).

[Example 7]

The same procedure as in Example 1 was carried out except that in Example 1, 53 g (0.72 mol) of n-butanol was used instead of 23 g of methanol.

When the composition of part of the obtained reaction liquid was checked by high performance liquid chromatography, bisphenol A was found to have been generated in an amount of 14.5% by mass (the generation rate was 14.5 / 100 × 388 g / 228 g/mol / 0.31 mol = 80 mol%).

In addition, when the composition of part of the obtained reaction liquid was checked by gas chromatography,

dimethyl carbonate was found to have been generated in an amount of 10.7% by mass (the generation rate was 10.7 / 100 × 388 g / 174 g/mol / 0.31 mol = 77 mol%).

[Example 8]

The same procedure as in Example 1 was carried out except that in Example 1, 2 g of sodium hydroxide (2 g / 40 g/mol = 0.05 mol, the mole ratio of sodium hydroxide to 1 mol of the repeating unit of the polycarbonate resin = 0.05 mol / 0.31 mol = 0.16) was used instead of 15 g of triethylamine.

When the composition of part of the obtained reaction liquid was checked by high performance liquid chromatography, bisphenol A was found to have been generated in an amount of 20.4% by mass (the generation rate was 20.4 / 100 × 345 g / 228 g/mol / 0.31 mol = 100 mol%). In addition, the generation of dimethyl carbonate was also found.

[Example 9]

The same procedure as in Example 1 was carried out except that in Example 1, 16 g of paratoluenesulfonic acid (16 g / 172 g/mol = 0.09 mol, the mole ratio of sodium hydroxide to 1 mol of the repeating unit of the polycarbonate resin = 0.09 mol / 0.31 mol = 0.29) was added instead of 15 g of triethylamine.

When the composition of part of the obtained reaction liquid was checked by high performance liquid chromatography, bisphenol A was found to have been generated in an amount of 10.7% by mass (the generation rate was 10.7 / 100 × 359 g / 228 g/mol / 0.31 mol = 54 mol%). In addition, the generation of dimethyl carbonate was also found.

[Comparative Example 1]

In a jacket-type separable flask equipped with a Dimroth condenser tube, a stirring blade, and a thermometer, 80 g of the polycarbonate resin, 201 g of methanol (201 g / 32 g/mol = 6.28 mol, the mole ratio of methanol to 1 mol of the repeating unit of the polycarbonate resin = 6.28 mol / 0.31 mol = 20.25), and 15 g of triethylamine were placed at room temperature in a nitrogen atmosphere. Phenol was not added.

Next, when an attempt was made to raise the internal temperature to 85°C, reflux of methanol occurred at around 64°C, and thus the reflux was maintained as it was, to carry out the reaction. When the reaction was carried out for 4 hours, the polycarbonate resin was observed as a solid in the reaction liquid, and the degradation was found not to proceed.

[Comparative Example 2]

In a jacket-type separable flask equipped with a Dimroth condenser tube, a stirring blade, and a thermometer, 80 g of the polycarbonate resin, 201 g of methanol, and 127 g of triethylamine (127 g / 101 g/mol = 1.26 mol, the mole ratio of triethylamine to 1 mol of the repeating unit of the polycarbonate resin = 1.26 mol / 0.31 mol = 4.06) were placed at room temperature in a nitrogen atmosphere. Phenol was not added.

After that, the internal temperature was raised to 64°C. The reaction was carried out for 4 hours while maintaining 64°C as it was, to obtain a uniform reaction liquid.

When the composition of part of the obtained reaction liquid was checked by high performance liquid chromatography, bisphenol A was found to have been generated in an amount of 17.3% by mass (the generation rate was 17.3 / 100 × 408 g / 228 g/mol / 0.31 mol = 100 mol%). Triethylamine was used in as large an amount as 127 g for 80 g of the polycarbonate resin, and thus a remarkable amine odor was observed when part of the obtained reaction liquid was withdrawn.

[Comparative Example 3]

The same procedure as in Example 1 was carried out except that in Example 1, methanol was not supplied at all instead of 23 g of methanol.

When the composition of part of the obtained reaction liquid was checked by high performance liquid chromatography, bisphenol A was found to have been generated in an amount of 8.5% by mass (the generation rate was 8.5 / 100 × 335 g / 228 g/mol / 0.31 mol = 40 mol%).

No amine odor was felt when part of the obtained reaction liquid was withdrawn.

The types of the aromatic monoalcohols, the types of the aliphatic monoalcohols, and the types of the catalysts used in Examples 1 to 5 and Comparative Examples 1 to 3 are summarized in Table 1. In addition, the generation rates (%) of bisphenol A (BPA) and the amine odors after the reaction in Examples 1 to 5 and Comparative Examples 1 to 3 are summarized in Table 2. From Table 2, it was able to be found that even when the amount of the amine used is small,

the state after the reaction becomes a uniform solution by using phenol and methanol in combination, and thus the polycarbonate resin can be degraded and the amine odor can be suppressed. In addition, it was able to be found that even when the amount of the amine used was large, the odor can be suppressed by using phenol and methanol in combination as compared with the system using methanol alone.

[Table 1]

| | Type of aromatic monoalcohol | Amount of aromatic monoalcohol (g) | Type of aliphatic monoalcohol | Amount of aliphatic monoalcohol (g) | Mole ratio of aliphatic monoalcohol to 1 mol of repeating unit of PC | Type of catalyst | Amount of catalyst (g) | Mole ratio of catalyst to 1 mol of repeating unit of PC |
|---|---|---|---|---|---|---|---|---|
| Example 1 | Phenol | 240 | Methanol | 23 | 2.32 | Triethylamine | 15 | 0.48 |
| Example 2 | Phenol | 240 | Methanol | 23 | 2.32 | Triethylamine | 32 | 1.03 |
| Example 3 | Phenol | 240 | Methanol | 23 | 2.32 | Triethylamine | 63 | 2.00 |
| Example 4 | Phenol | 240 | Methanol | 23 | 2.32 | Triethylamine | 110 | 3.26 |
| Example 5 | Phenol | 240 | Methanol | 23 | 2.32 | Triethylamine | 128 | 4.10 |
| Comparative Example 1 | No | 0 | Methanol | 201 | 20.32 | Triethylamine | 15 | 0.48 |
| Comparative Example 2 | No | 0 | Methanol | 201 | 20.32 | Triethylamine | 127 | 4.10 |
| Comparative Example 3 | Phenol | 240 | No | 0 | - | Triethylamine | 15 | 0.48 |

[Table 2]

| | Type of aromatic monoalcohol | Type of aliphatic monoalcohol | Type of catalyst | Mole ratio of catalyst to 1 mol of repeating unit of PC | State after reaction | Generation rate of BPA (%) | Amine odor after reaction | |
|---|---|---|---|---|---|---|---|---|
| Example 1 | Phenol | Methanol | Triethylamine | 0.48 | Uniform solution | 99 | ○ | Slight odor |
| Example 2 | Phenol | Methanol | Triethylamine | 1.03 | Uniform solution | 100 | Δ | Odor observed |
| Example 3 | Phenol | Methanol | Triethylamine | 2.00 | Uniform solution | 100 | Δ | Odor observed |
| Example 4 | Phenol | Methanol | Triethylamine | 3.26 | Uniform solution | 99 | ×Δ | Strong odor observed. No odor as compared with Comparative Examples 1 and 2 |
| Example 5 | Phenol | Methanol | Triethylamine | 4.10 | Uniform solution | 99 | ×Δ | Strong odor observed. No odor as compared with Comparative Examples 1 and 2 |
| Comparative Example 1 | No | Methanol | Triethylamine | 0.48 | Solid observed | Degradation did not proceed | ○ | Slight odor |
| Comparative Example 2 | No | Methanol | Triethylamine | 4.10 | Uniform solution | 99 | × | Remarkable odor |
| Comparative Example 3 | Phenol | No | Triethylamine | 0.48 | Uniform solution | 40 | ○ | Slight odor |

The types of the aromatic monoalcohols, the types of the aliphatic monoalcohols, the types of the catalysts, and the generation rates (%) of bisphenol A (BPA) in Examples 1 and 6 to 9 are summarized in Table 3. From Table 3, it can be seen that the polycarbonate resin can be degraded by any catalyst of an alkali metal hydroxide, an alkali metal carbonate, an alkylamine, and an acid. In addition, it can be seen that the polycarbonate resin can be degraded even when the type of the aliphatic monoalcohol is changed. Especially, it can be seen that the bisphenol can be obtained at a high generation rate by the combination of phenol and methanol.

[Table 3]

| | Type of aromatic monoalcohol | Type of aliphatic monoalcohol | Type of catalyst | Generation rate of BPA (%) |
|---|---|---|---|---|
| Example 1 | Phenol | Methanol | Triethylamine | 99 |
| Example 6 | Phenol | Ethanol | Triethylamine | 91 |
| Example 7 | Phenol | n-Butanol | Triethylamine | 80 |
| Example 8 | Phenol | Methanol | Sodium hydroxide | 100 |
| Example 9 | Phenol | Methanol | Paratoluenesulfonic acid | 54 |

[Example 10]

(Bisphenol recovery step)

The reaction liquid obtained in Example 1 was transferred to a distillation apparatus equipped with a thermometer, a stirring blade, a distilling-out tube, and a pressure regulator, and while observing the amount distilled out, the internal temperature was gradually raised to 180°C and the internal pressure was gradually lowered from normal pressure to 20 kPa to distill off an azeotropic mixture of methanol and dimethyl carbonate, triethylamine, and phenol.

After that, the pressure inside the flask was restored with nitrogen, the internal temperature was lowered to 80°C, and 200 g of toluene was added to obtain an organic phase 1. The obtained organic phase 1 was washed 5 times with 50 g of desalinated water to obtain an organic phase 2.

The temperature of the obtained organic phase 2 was lowered to 20°C to obtain a slurry. The obtained slurry was filtered to obtain a cake. The obtained cake was dried on a rotary evaporator to obtain 30 g of bisphenol A.

The purity of the obtained bisphenol A was 99.8% by mass, and the molten color thereof was APHA 192.

(Dimethyl carbonate recovery step)

The azeotropic mixture of methanol and dimethyl carbonate distilled off in the bisphenol recovery step was supplied to a separatory funnel, then water was added, oil water separation was carried out, and the oil phase was recovered to obtain 17 g of dimethyl carbonate.

[Example 11]

In a glass reaction tank having an internal volume of 45 mL equipped with a stirrer and a distilling-out tube, 10.00 g (0.04 mol) of bisphenol A obtained in Example 10, 9.95 g (0.05 mol) of diphenyl carbonate, and 18 μL of a 400 ppm by mass cesium carbonate aqueous solution were placed. The operation of reducing the pressure of the glass reaction tank to about 100 Pa and subsequently restoring the pressure to atmospheric pressure with nitrogen was repeated three times to purge the inside of the reaction tank with nitrogen. After that, the reaction tank was immersed in an oil bath at 220°C to dissolve the contents thereof.

The rotation speed of the stirrer was set to 100 revolutions per minute, and the pressure inside the reaction tank was reduced from 101.3 kPa to 13.3 kPa in absolute pressure over 40 minutes while distilling off phenol by-produced by the oligomerization reaction of bisphenol A and diphenyl carbonate in the reaction tank.

Subsequently, an ester exchange reaction was carried out for 80 minutes while holding the pressure inside the

reaction tank at 13.3 kPa and further distilling off phenol.

After that, the temperature outside the reaction tank was raised to 290°C, and the pressure inside the reaction tank was reduced from 13.3 kPa to 399 Pa in absolute pressure over 40 minutes to remove the distilled-out phenol outside the system.

After that, the absolute pressure of the reaction tank was reduced to 30 Pa, and a polycondensation reaction was carried out. The polycondensation reaction was completed when the stirrer in the reaction tank had a predetermined stirring power. The time from the temperature raising to 290°C to the completion of the polymerization was 120 minutes.

Next, the pressure of the reaction tank was restored to 101.3 kPa in absolute pressure with nitrogen, then the pressure was increased to 0.2 MPa in gauge pressure, and the polycarbonate resin was withdrawn from the reaction tank to obtain a polycarbonate resin. The viscosity average molecular weight (Mv) of the obtained polycarbonate resin was 27100.

[Example 12]

(Degradation step)

In a jacket-type separable flask equipped with a Dimroth condenser tube, a stirring blade, and a thermometer, 80 g of the polycarbonate resin (the molecular weight of the repeating unit of the polycarbonate resin was 254 g/mol, and thus the number of moles of the repeating unit = 80 g / 254 g/mol = 0.31 mol), 240 g of phenol, 1 g of methanol (1 g / 32 g/mol = 0.031 mol, the mole ratio of methanol to 1 mol of the repeating unit of the polycarbonate resin = 0.031 mol / 0.31 mol = 0.1), and 0.3 g of sodium hydroxide (0.3 g / 40 g/mol = 7.5 mmol, the mole ratio of sodium hydroxide to 1 mol of the repeating unit of the polycarbonate resin = 7.5 mmol / 0.31 mol = 0.02) were placed at room temperature in a nitrogen atmosphere (the amount of the liquid was 80 g + 240 g + 1 g + 0.3 g = 321 g).

After that, the internal temperature was raised to 85°C. An undissolved polycarbonate resin was observed (it was slurry-like) in the reaction liquid when the temperature reached 85°C. The reaction was carried out for 3 hours while maintaining 85°C as it was, to obtain a uniform reaction liquid.

When the composition of part of the obtained reaction liquid was checked by gas chromatography, methyl phenyl carbonate was found to have been generated in an amount of 0.7% by mass (the generation rate was 0.7 / 100 × 321 g / 152 g/mol / 0.31 mol = 5 mol%). No generation of dimethyl carbonate was observed. In addition, the generation of bisphenol A was found.

[Example 13]

The same procedure as in Example 12 was carried out except that in Example 12, 17 g of methanol (17 g / 32 g/mol = 0.53 mol, the mole ratio of methanol to 1 mol of the repeating unit of the polycarbonate resin = 0.53 mol / 0.31 mol = 1.7) was used instead of 1 g of methanol (the amount of the liquid was 80 g + 240 g + 17 g + 0.3 g = 337 g).

When the composition of part of the obtained reaction liquid was checked by high performance liquid chromatography, methyl phenyl carbonate was found to be generated in an amount of 3.7% by mass (the generation rate was 3.7 / 100 × 337 g / 152 g/mol / 0.31 mol = 26 mol%), and dimethyl carbonate was found to be generated in an amount of 1.9% by mass (the generation rate was 1.9 / 100 × 337 g / 90 g/mol / 0.31 mol = 23 mol%). In addition, the generation of bisphenol A was found.

[Example 14]

The same procedure as in Example 12 was carried out except that in Example 12, 17 g of methanol (17 g / 32 g/mol = 0.53 mol, the mole ratio of methanol to 1 mol of the repeating unit of the polycarbonate resin = 0.53 mol / 0.31 mol = 1.7) was used instead of 1 g of methanol, and 4.8 g of triethylamine (4.8 g / 101 g/mol = 0.05 mol, the mole ratio of triethylamine to 1 mol of the repeating unit of the polycarbonate resin = 0.05 mol / 0.31 mol = 0.16) was used instead of 0.3 g of sodium hydroxide (the amount of the liquid was 80 g + 240 g + 17 g + 4.8 g = 342 g).

When the composition of part of the obtained reaction liquid was checked by high performance liquid chromatography, methyl phenyl carbonate was found to be generated in an amount of 10.2% by mass (the generation rate was 10.2 / 100 × 342 g / 152 g/mol / 0.31 mol = 74 mol%), and dimethyl carbonate was found to be generated in an amount of 1.6% by mass (the generation rate was 1.6 / 100 × 342 g / 90 g/mol / 0.31 mol = 20 mol%). In addition, the generation of bisphenol A was found.

[Example 15]

The same procedure as in Example 12 was carried out except that in Example 12, 40 g of methanol (40 g / 32

g/mol = 1.25 mol, the mole ratio of methanol to 1 mol of the repeating unit of the polycarbonate resin = 1.25 mol / 0.31 mol = 4.0) was used instead of 1 g of methanol (the amount of the liquid was 80 g + 240 g + 40 g + 0.3 g = 360 g).

When the composition of part of the obtained reaction liquid was checked by high performance liquid chromatography, no generation of methyl phenyl carbonate was observed. Dimethyl carbonate was found to have been generated in an amount of 3.7% by mass (the generation rate was 3.7 / 100 × 360 g / 90 g/mol / 0.31 mol = 48 mol%). In addition, the generation of bisphenol A was found.

The mole ratios of methanol to 1 mol of the repeating unit of the PC (polycarbonate resin), the types of the catalysts used, and the amounts of methyl phenyl carbonate generated in Examples 12 to 15 are summarized in Table 4. From Table 4, it can be seen that a large amount of methyl phenyl carbonate can be obtained when the mole ratio of methanol to 1 mol of the repeating unit of the polycarbonate resin is less than 2.

[Table 4]

| | Mole ratio of methanol to 1 mol of repeating unit of PC | Type of catalyst used | Generation rate of methyl phenyl carbonate (mol%) |
|---|---|---|---|
| Example 12 | 0.1 | Sodium hydroxide | 5 |
| Example 13 | 1.7 | Sodium hydroxide | 26 |
| Example 14 | 1.7 | Triethylamine | 74 |
| Example 15 | 4.0 | Sodium hydroxide | Not generated |

[Example 16]

The same procedure as in Example 12 was carried out except that in Example 12, 43 g of butanol (43 g / 74 g/mol = 0.58 mol, the mole ratio of butanol to 1 mol of the repeating unit of the polycarbonate resin = 0.58 mol / 0.31 mol = 1.9) was used instead of 1 g of methanol (the amount of the liquid was 80 g + 240 g + 43 g + 0.3 g = 363 g).

When the composition of part of the obtained reaction liquid was checked by high performance liquid chromatography, butyl phenyl carbonate was found to be generated in an amount of 8.4% by mass (the generation rate was 8.4 / 100 × 363 g / 194 g/mol / 0.31 mol = 51 mol%), and dibutyl carbonate was found to be generated in an amount of 0.7% by mass (the generation rate was 0.7 / 100 × 363 g / 174 g/mol / 0.31 mol = 5 mol%). In addition, the generation of bisphenol A was found.

[Example 17]

In a jacket-type separable flask equipped with a Dimroth condenser tube, a stirring blade, and a thermometer, 200 g of the polycarbonate resin (the molecular weight of the repeating unit of the polycarbonate resin was 254 g/mol, and thus the number of moles of the repeating unit = 200 g / 254 g/mol = 0.79 mol), 600 g of phenol, 45 g of methanol (45 g / 32 g/mol = 1.41 mol, the mole ratio of methanol to 1 mol of the repeating unit of the polycarbonate resin = 1.41 mol / 0.79 mol = 1.8), and 2.4 g of sodium hydroxide (2.4 g / 40 g/mol = 0.06 mol, the mole ratio of sodium hydroxide to 1 mol of the repeating unit of the polycarbonate resin = 0.06 mol / 0.79 mol = 0.08) were placed at room temperature in a nitrogen atmosphere (the amount of the liquid was 200 g + 600 g + 45 g + 2.4 g = 847 g).

After that, the internal temperature was raised to 85°C. An undissolved polycarbonate resin was observed (it was slurry-like) in the reaction liquid when the temperature reached 85°C. The reaction was carried out for 3 hours while maintaining 85°C as it was, to obtain a uniform reaction liquid.

When the composition of part of the obtained reaction liquid was checked by gas chromatography, methyl phenyl carbonate was found to have been generated in an amount of 4.2% by mass (the generation rate was 4.2 / 100 × 847 g / 152 g/mol / 0.79 mol = 30 mol%). In addition, the generation of dimethyl carbonate and bisphenol A was found.

10 g of 10% by mass hydrochloric acid was added to the obtained reaction liquid to neutralize sodium hydroxide, and thereby the reaction was stopped to obtain a mixed liquid.

The obtained mixed liquid was filtered, then the filtrate was placed in a flask equipped with a depressurization apparatus, a thermometer, and a distilling-out tube, and the flask was immersed in an oil bath. The oil bath was set to 120°C under normal pressure to distill off unreacted methanol, dimethyl carbonate, and water.

After that, the pressure was set to 1 kPa, and then the temperature of the oil bath was raised to 160°C to distill off phenol.

Further, the temperature of the oil bath was raised to 175°C to obtain 28 g of methyl phenyl carbonate.

[Example 18]

28 g of methyl phenyl carbonate obtained in Example 17 and 1 g of tetraphenoxytitanium were added to a flask equipped with a depressurization apparatus, a thermometer, and a distilling-out tube, and the flask was immersed in an oil bath at 40°C. The pressure was set to 1 kPa, the temperature was gradually raised to 185°C, and the reaction was carried out while distilling off dimethyl carbonate. After that, the oil bath was set to 210°C to obtain 11 g of diphenyl carbonate.

[Example 19]

In a glass reaction tank having an internal volume of 45 mL equipped with a stirrer and a distilling-out tube, 10.00 g (0.04 mol) of bisphenol A, 9.95 g (0.05 mol) of diphenyl carbonate obtained in Example 18, and 18 μL of a 400 ppm by mass cesium carbonate aqueous solution were placed. The operation of reducing the pressure of the glass reaction tank to about 100 Pa and subsequently restoring the pressure to atmospheric pressure with nitrogen was repeated three times to purge the inside of the reaction tank with nitrogen. After that, the reaction tank was immersed in an oil bath at 220°C to dissolve the contents thereof.

The rotation speed of the stirrer was set to 100 revolutions per minute, and the pressure inside the reaction tank was reduced from 101.3 kPa to 13.3 kPa in absolute pressure over 40 minutes while distilling off phenol by-produced by the oligomerization reaction of bisphenol A and diphenyl carbonate in the reaction tank.

Subsequently, an ester exchange reaction was carried out for 80 minutes while holding the pressure inside the reaction tank at 13.3 kPa and further distilling off phenol.

After that, the temperature outside the reaction tank was raised to 290°C, and the pressure inside the reaction tank was reduced from 13.3 kPa to 399 Pa in absolute pressure over 40 minutes to remove the distilled-out phenol outside the system.

After that, the absolute pressure of the reaction tank was reduced to 30 Pa, and a polycondensation reaction was carried out. The polycondensation reaction was completed when the stirrer in the reaction tank had a predetermined stirring power. The time from the temperature raising to 290°C to the completion of the polymerization was 120 minutes.

Next, the pressure of the reaction tank was restored to 101.3 kPa in absolute pressure with nitrogen, then the pressure was increased to 0.2 MPa in gauge pressure, and the polycarbonate resin was withdrawn from the reaction tank to obtain a polycarbonate resin. The viscosity average molecular weight (Mv) of the obtained polycarbonate resin was 25800.

Industrial Applicability

According to the degradation method of the present invention, a useful compound such as a bisphenol can be obtained from a waste plastic or the like by using chemical recycling. Further, by using such a compound, a polycarbonate resin can be produced again, which is industrially useful.

## Claims

**1.** A method for degrading a polycarbonate resin, comprising, in a slurry-like reaction liquid comprising a polycarbonate resin, an aromatic monoalcohol, an aliphatic monoalcohol, and a catalyst, degrading the polycarbonate resin.

**2.** A method for degrading a polycarbonate resin, comprising:

a preparation step of preparing a slurry-like reaction liquid comprising a polycarbonate resin, an aromatic monoalcohol, an aliphatic monoalcohol, and a catalyst; and
a degradation reaction step of degrading the polycarbonate resin in the slurry-like reaction liquid prepared in the preparation step.

**3.** The method for degrading a polycarbonate resin according to claim 1 or 2, wherein the catalyst is any selected from the group consisting of an alkali metal hydroxide, an alkali metal carbonate, an alkylamine, and an acid.

**4.** The method for degrading a polycarbonate resin according to claim 3, wherein the alkylamine is represented by the following formula (I):

[Formula 1]

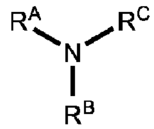

...(I)

wherein $R^A$ represents an alkyl group having 1 to 3 carbon atoms, and $R^B$ and $R^C$ each independently represent a hydrogen atom or an alkyl group having 1 to 3 carbon atoms.

**5.** The method for degrading a polycarbonate resin according to claim 3, wherein the alkylamine is a tertiary amine.

**6.** The method for degrading a polycarbonate resin according to any one of claims 3 to 5, wherein a mole ratio of the alkylamine to 1 mol of a repeating unit of the polycarbonate resin in the slurry-like reaction liquid is 4.5 or less.

**7.** The method for degrading a polycarbonate resin according to claim 3, wherein the alkali metal hydroxide is sodium hydroxide or potassium hydroxide.

**8.** The method for degrading a polycarbonate resin according to claim 3, wherein the acid is any selected from the group consisting of sulfuric acid, phosphoric acid, and a sulfonic acid.

**9.** The method for degrading a polycarbonate resin according to any one of claims 1 to 8, wherein the aromatic monoalcohol is any selected from the group consisting of phenol, a cresol, and a xylenol.

**10.** The method for degrading a polycarbonate resin according to any one of claims 1 to 9, wherein the aliphatic monoalcohol is any selected from the group consisting of methanol, ethanol, and n-butanol.

**11.** The method for degrading a polycarbonate resin according to any one of claims 1 to 10, wherein a mole ratio of the aliphatic monoalcohol to the aromatic monoalcohol is 0.7 or less.

**12.** The method for degrading a polycarbonate resin according to any one of claims 1 to 11, wherein a reaction temperature for degrading the polycarbonate resin is 120°C or less.

**13.** A method for producing a bisphenol, comprising:

a degradation step of degrading a polycarbonate resin by using the method for degrading a polycarbonate resin according to any one of claims 1 to 12; and
a bisphenol recovery step of recovering a bisphenol generated by the degradation of the polycarbonate resin.

**14.** The method for producing a bisphenol according to claim 13, wherein the bisphenol is 2,2-bis(4-hydroxyphenyl)propane.

**15.** A method for producing a dialkyl carbonate, comprising:

a degradation step of degrading a polycarbonate resin by using the method for degrading a polycarbonate resin according to any one of claims 1 to 12; and
a dialkyl carbonate recovery step of recovering a dialkyl carbonate generated by the degradation of the polycarbonate resin.

**16.** The method for degrading a dialkyl carbonate according to claim 15, wherein a mole ratio of the aliphatic monoalcohol to 1 mol of a repeating unit of the polycarbonate resin in the slurry-like reaction liquid is 2.0 or more and 6.0 or less.

**17.** A method for producing an alkyl aryl carbonate, comprising:

a polycarbonate resin degradation step of degrading a polycarbonate resin in the presence of an aromatic monoalcohol, an aliphatic monoalcohol, and a catalyst; and
an alkyl aryl carbonate recovery step of recovering an alkyl aryl carbonate generated by the degradation of the

polycarbonate.

**18.** A method for producing an alkyl aryl carbonate, comprising:

a degradation step of degrading a polycarbonate resin by using the method for degrading a polycarbonate resin according to any one of claims 1 to 12; and
an alkyl aryl carbonate recovery step of recovering an alkyl aryl carbonate generated by the degradation of the polycarbonate resin.

**19.** The method for producing an alkyl aryl carbonate according to claim 17 or 18, wherein the aromatic monoalcohol is phenol and the alkyl aryl carbonate is an alkyl phenyl carbonate.

**20.** The method for producing an alkyl aryl carbonate according to any one of claims 17 to 19, wherein a mole ratio of the aliphatic monoalcohol to 1 mol of a repeating unit of the polycarbonate resin is 0.1 or more and less than 2.0.

**21.** A method for producing a diaryl carbonate, comprising producing a diaryl carbonate by using a dialkyl carbonate obtained by the method for producing a dialkyl carbonate according to claim 15 or 16.

**22.** A method for producing a diaryl carbonate, comprising producing a diaryl carbonate by using an alkyl aryl carbonate obtained by the method for producing an alkyl aryl carbonate according to any one of claims 17 to 20.

**23.** A method for producing a recycled polycarbonate resin, comprising producing a recycled polycarbonate resin by using a bisphenol raw material comprising a bisphenol obtained by the method for producing a bisphenol according to claim 13 or 14.

**24.** A method for producing a recycled polycarbonate resin, comprising producing a recycled polycarbonate resin by using a diaryl carbonate raw material comprising a diaryl carbonate obtained by the method for producing a diaryl carbonate according to claim 21 or 22.

**25.** A method for producing an epoxy resin, comprising producing an epoxy resin by using a bisphenol obtained by the method for producing a bisphenol according to claim 13 or 14.

**26.** The method for producing an epoxy resin according to claim 25, wherein the epoxy resin is further reacted with a polyhydroxy compound raw material.

**27.** A method for producing an epoxy resin cured product, comprising curing an epoxy resin composition comprising an epoxy resin obtained by the method for producing an epoxy resin according to claim 25 or 26 and a curing agent to obtain an epoxy resin cured product.

Fig. 1

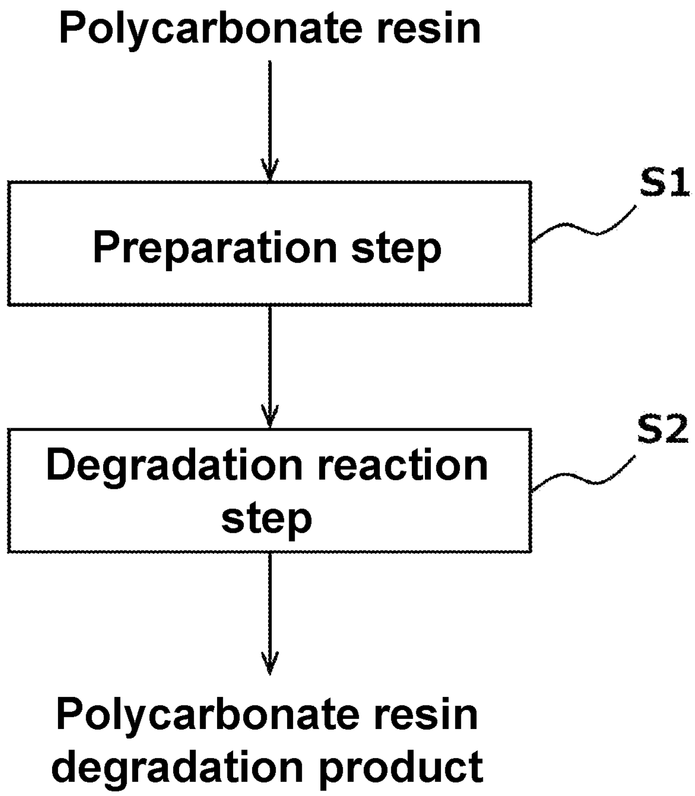
Polycarbonate resin
Preparation step
S1
Degradation reaction step
S2
Polycarbonate resin degradation product

Fig. 2

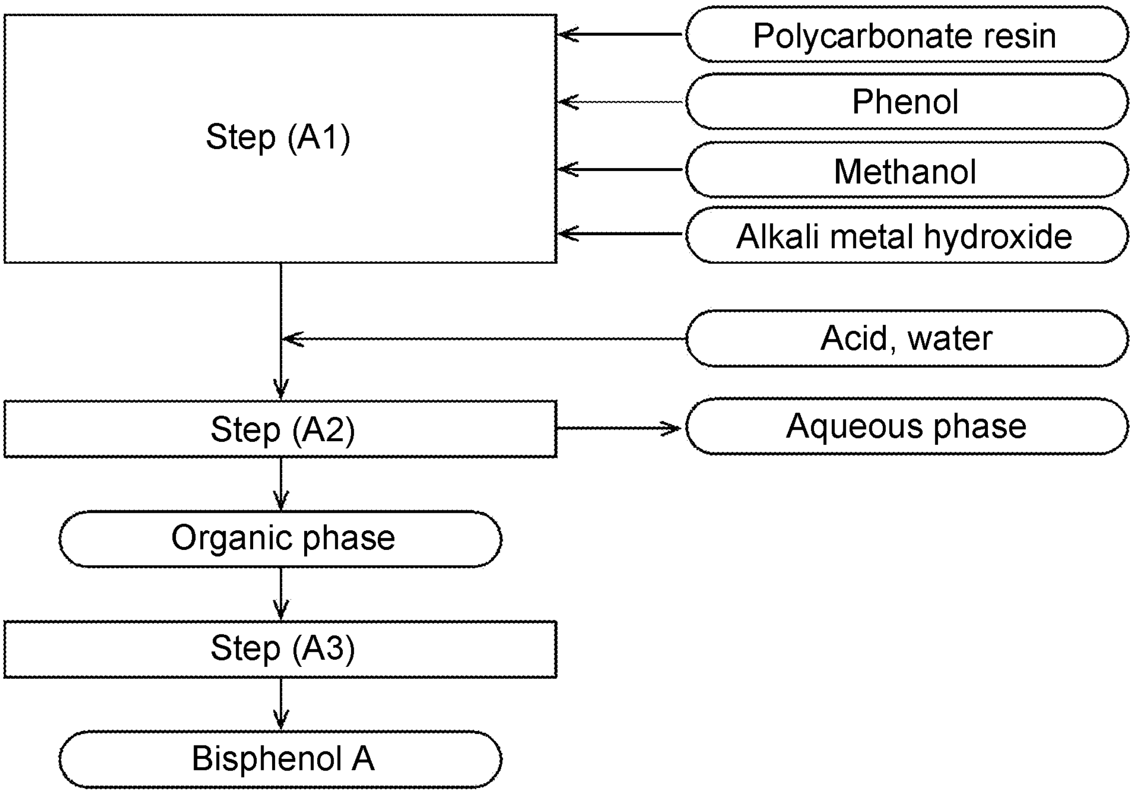
Step (A1)
Polycarbonate resin
Phenol
Methanol
Alkali metal hydroxide
Acid, water
Step (A2)
Aqueous phase
Organic phase
Step (A3)
Bisphenol A

Fig. 3

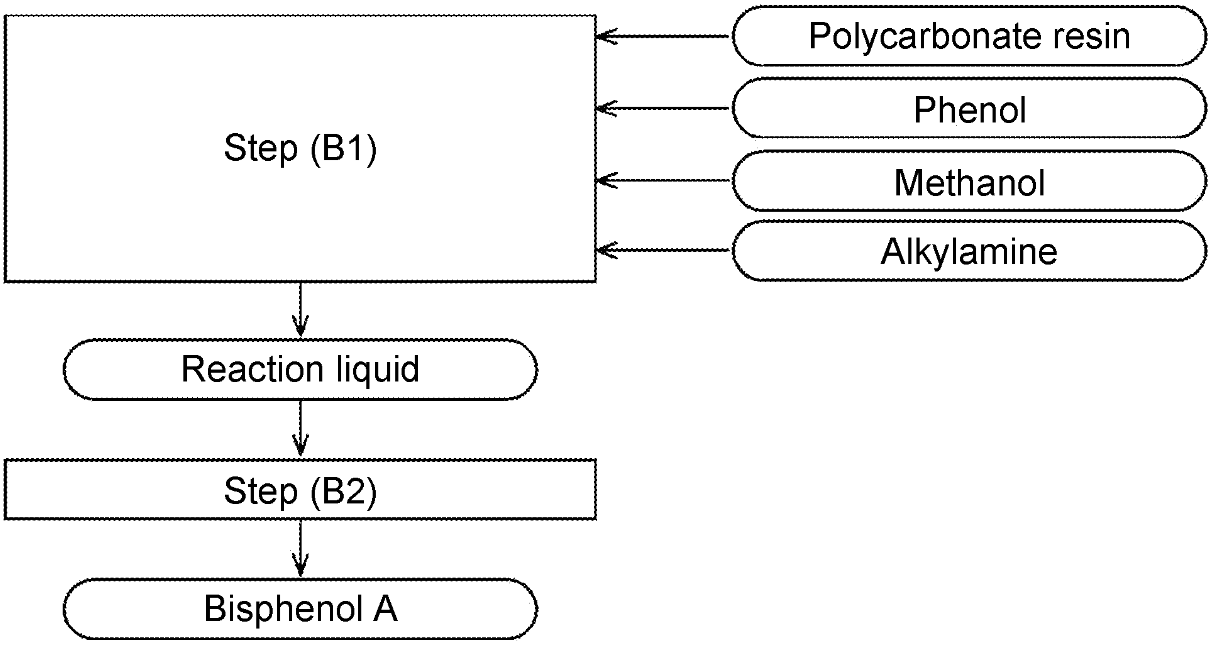
Step (B1)
Polycarbonate resin
Phenol
Methanol
Alkylamine
Reaction liquid
Step (B2)
Bisphenol A

Fig. 4

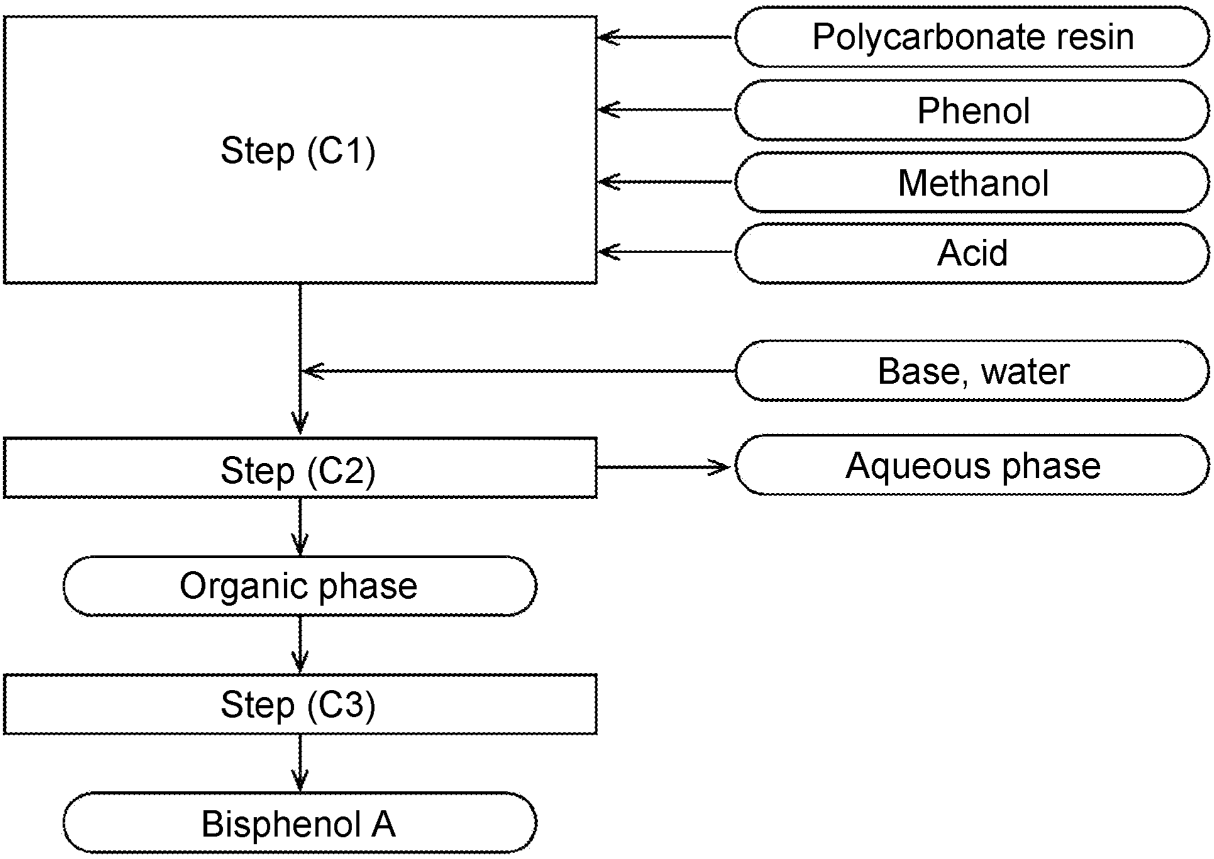
Step (C1)
Polycarbonate resin
Phenol
Methanol
Acid
Base, water
Step (C2)
Aqueous phase
Organic phase
Step (C3)
Bisphenol A

Fig. 5

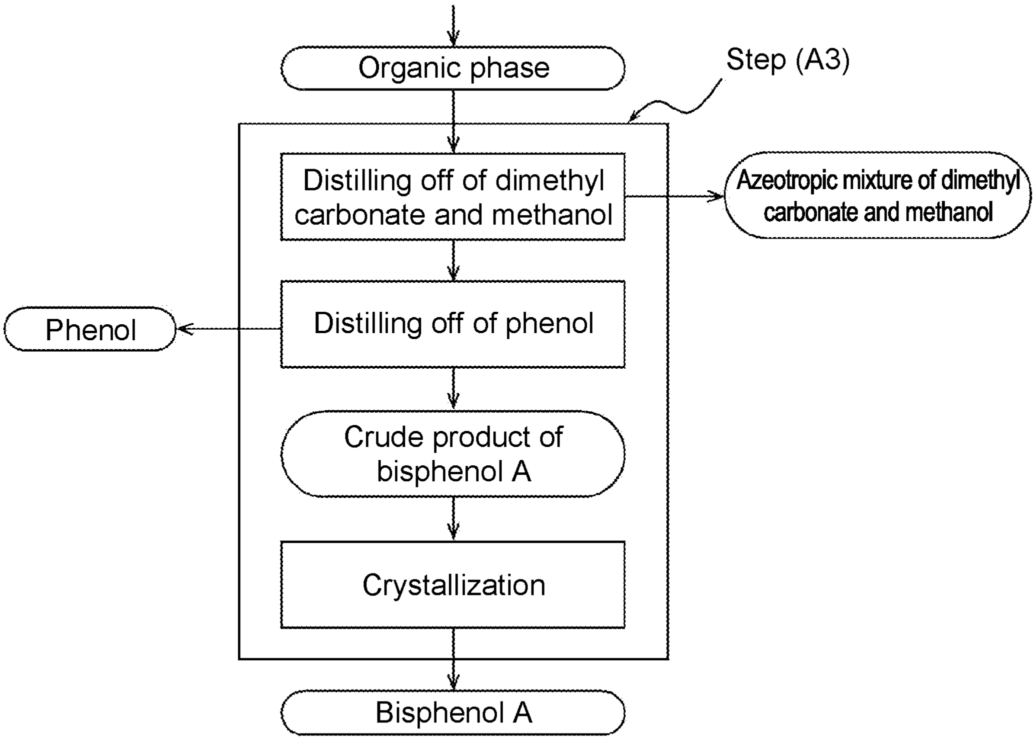
Organic phase
Step (A3)
Distilling off of dimethyl carbonate and methanol
Azeotropic mixture of dimethyl carbonate and methanol
Phenol
Distilling off of phenol
Crude product of bisphenol A
Crystallization
Bisphenol A

Fig. 6

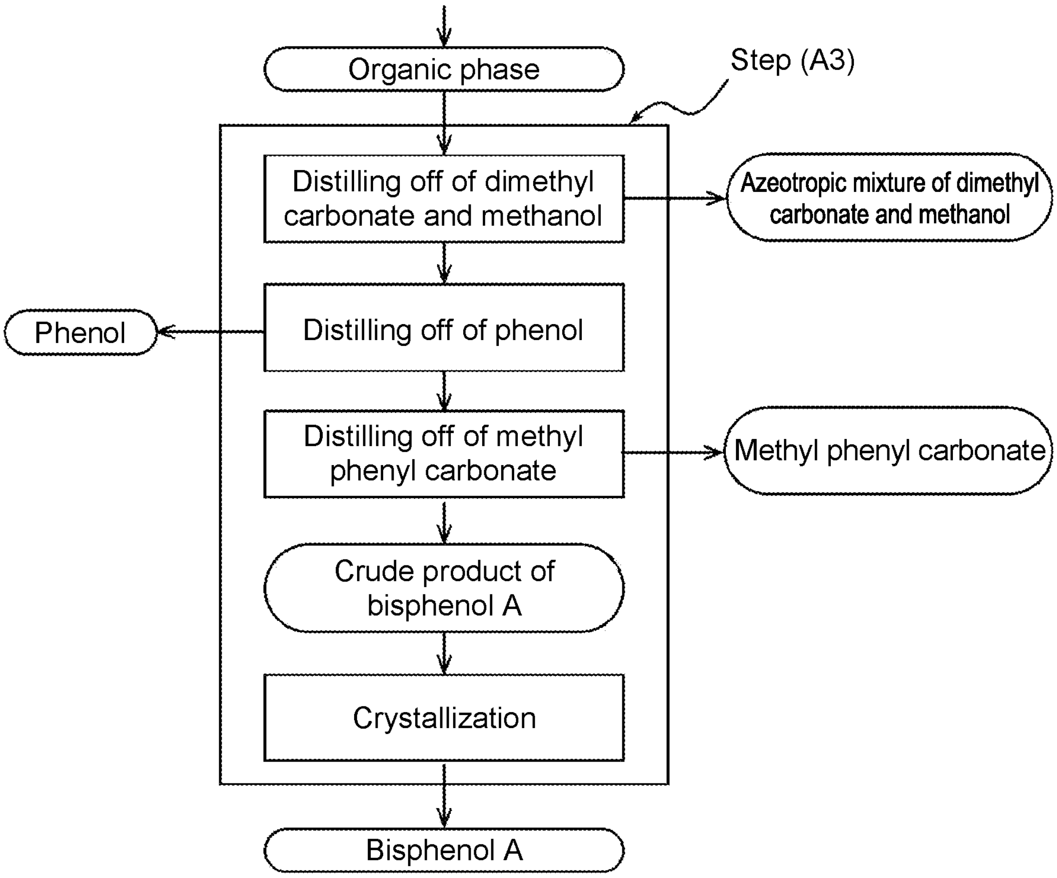
Organic phase
Step (A3)
Distilling off of dimethyl carbonate and methanol
Azeotropic mixture of dimethyl carbonate and methanol
Phenol
Distilling off of phenol
Distilling off of methyl phenyl carbonate
Methyl phenyl carbonate
Crude product of bisphenol A
Crystallization
Bisphenol A

Fig. 7

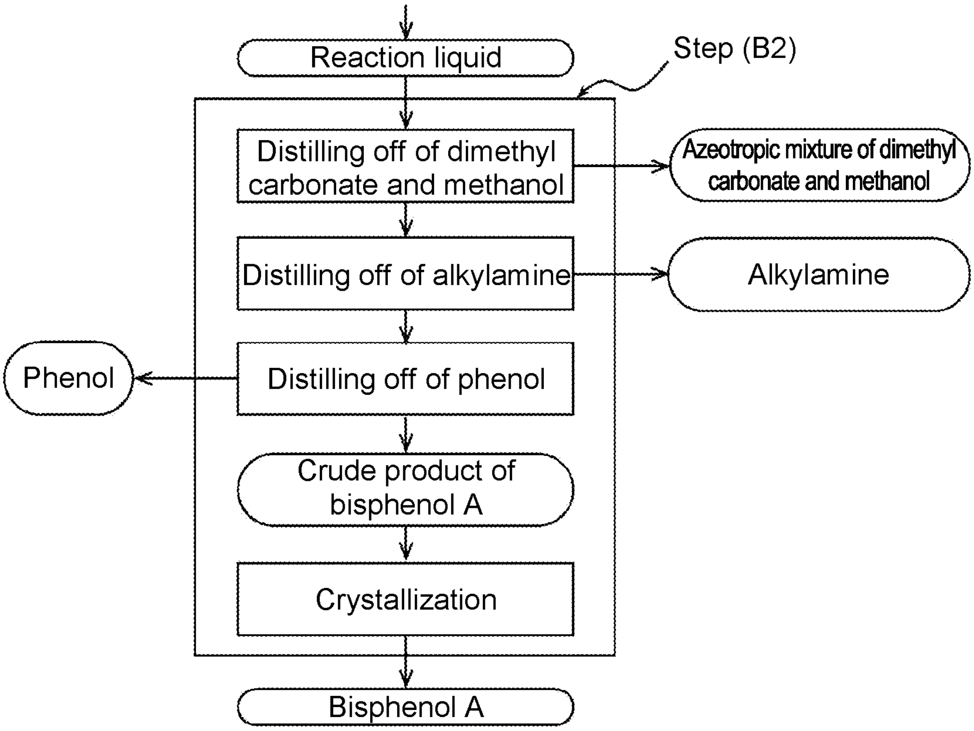
Reaction liquid
Step (B2)
Distilling off of dimethyl carbonate and methanol
Azeotropic mixture of dimethyl carbonate and methanol
Distilling off of alkylamine
Alkylamine
Phenol
Distilling off of phenol
Crude product of bisphenol A
Crystallization
Bisphenol A

Fig. 8

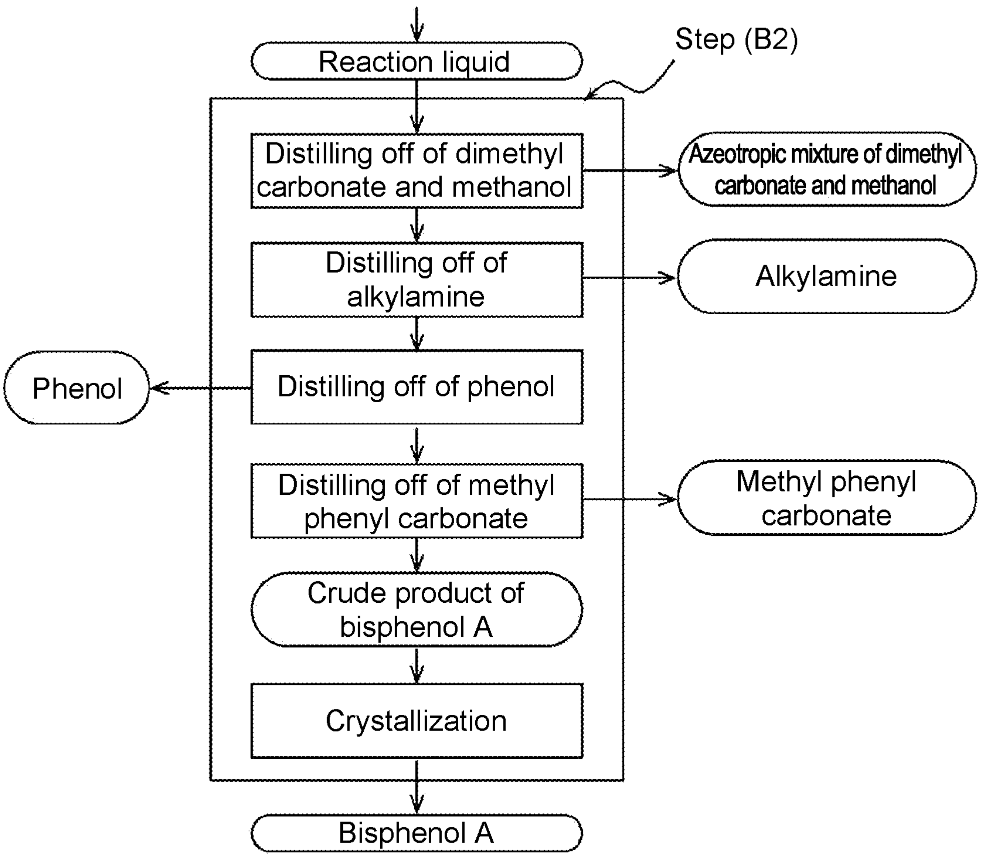
Reaction liquid
Step (B2)
Distilling off of dimethyl carbonate and methanol
Azeotropic mixture of dimethyl carbonate and methanol
Distilling off of alkylamine
Alkylamine
Phenol
Distilling off of phenol
Distilling off of methyl phenyl carbonate
Methyl phenyl carbonate
Crude product of bisphenol A
Crystallization
Bisphenol A

Fig. 9

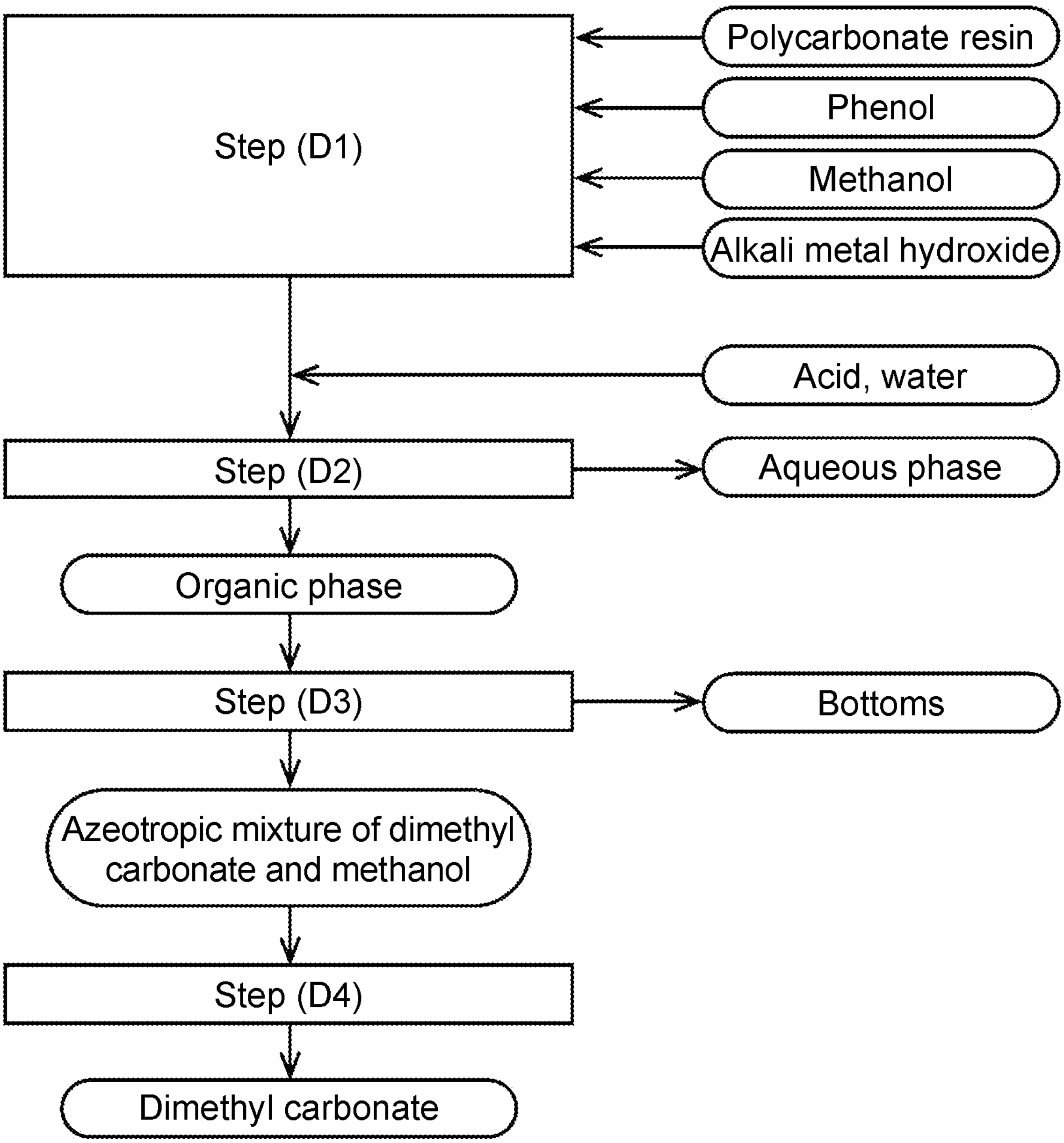
Step (D1)
Polycarbonate resin
Phenol
Methanol
Alkali metal hydroxide
Acid, water
Step (D2)
Aqueous phase
Organic phase
Step (D3)
Bottoms
Azeotropic mixture of dimethyl carbonate and methanol
Step (D4)
Dimethyl carbonate

Fig. 10

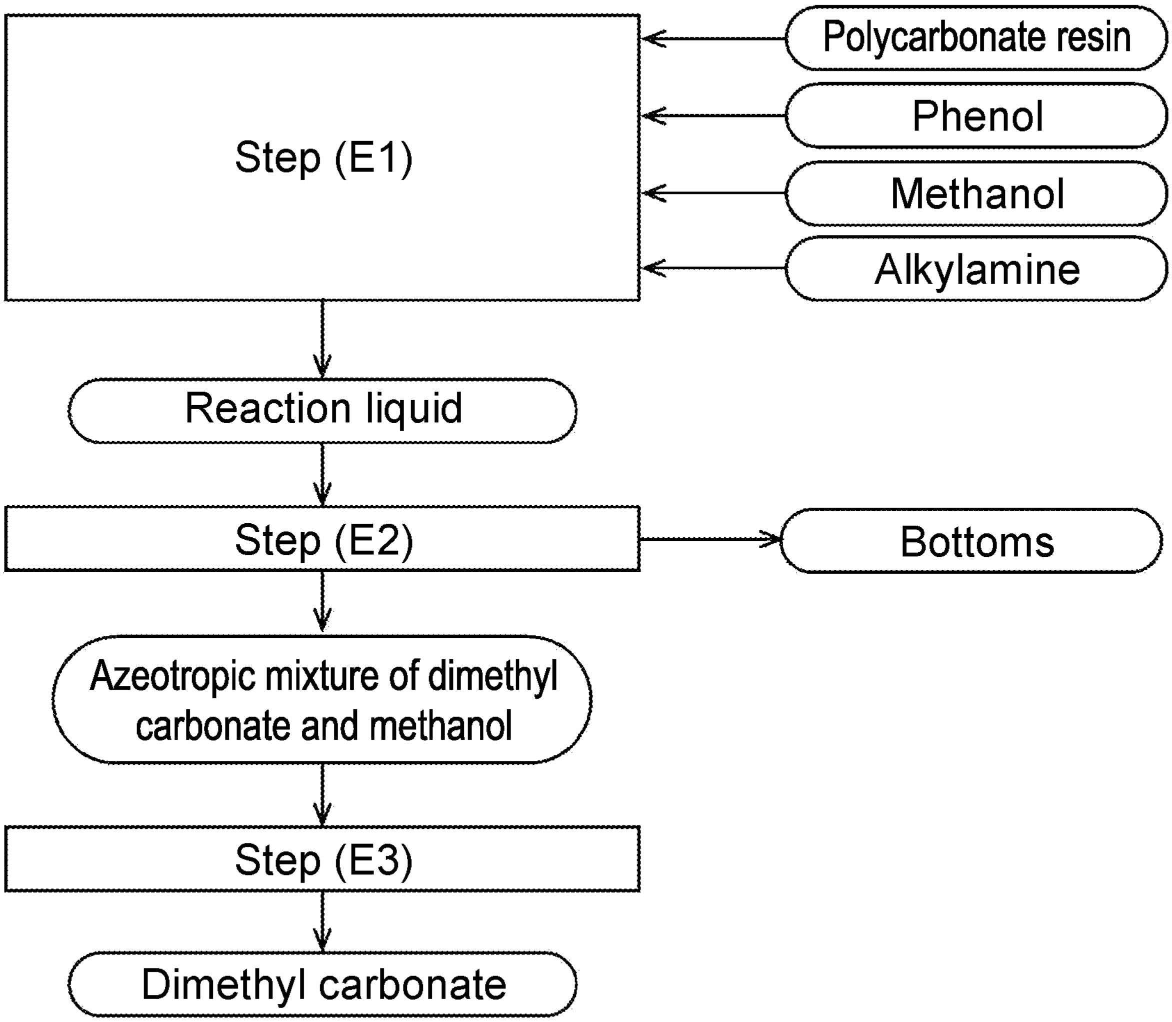
Step (E1)
Polycarbonate resin
Phenol
Methanol
Alkylamine
Reaction liquid
Step (E2)
Bottoms
Azeotropic mixture of dimethyl carbonate and methanol
Step (E3)
Dimethyl carbonate

Fig. 11

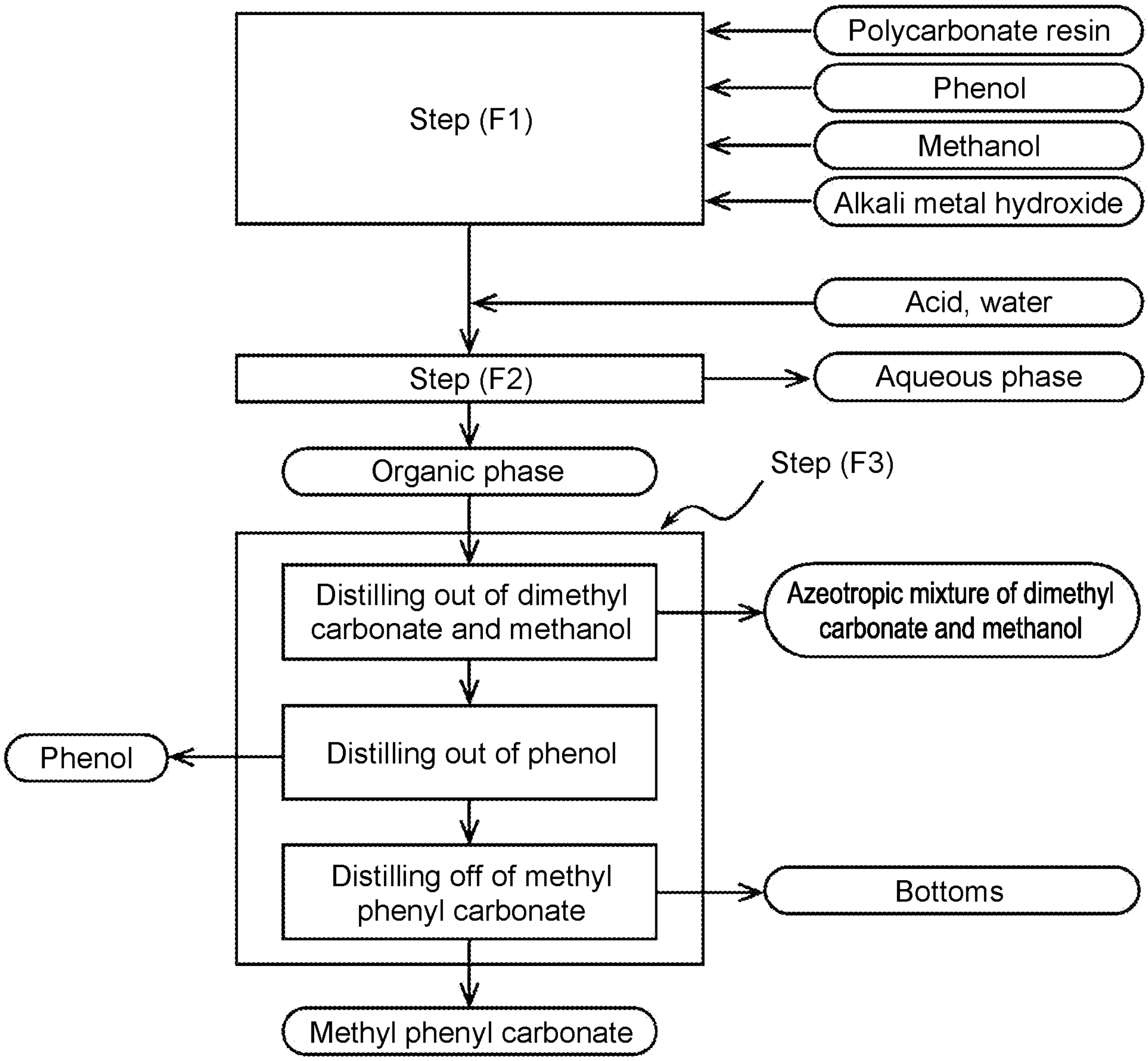
Step (F1)
Polycarbonate resin
Phenol
Methanol
Alkali metal hydroxide
Acid, water
Step (F2)
Aqueous phase
Organic phase
Step (F3)
Distilling out of dimethyl carbonate and methanol
Azeotropic mixture of dimethyl carbonate and methanol
Phenol
Distilling out of phenol
Distilling off of methyl phenyl carbonate
Bottoms
Methyl phenyl carbonate

Fig. 12

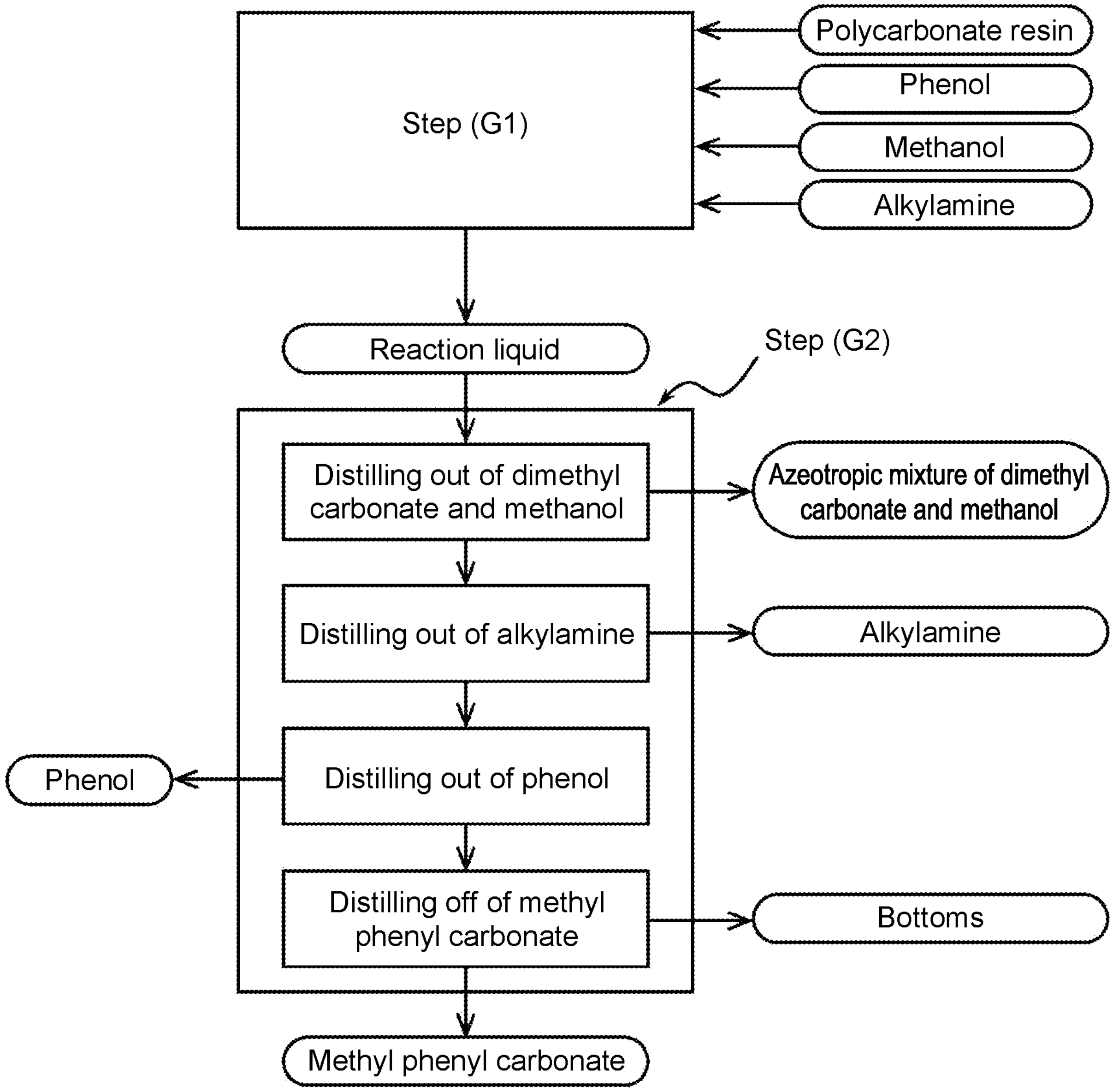
Step (G1)
Polycarbonate resin
Phenol
Methanol
Alkylamine
Reaction liquid
Step (G2)
Distilling out of dimethyl carbonate and methanol
Azeotropic mixture of dimethyl carbonate and methanol
Distilling out of alkylamine
Alkylamine
Phenol
Distilling out of phenol
Distilling off of methyl phenyl carbonate
Bottoms
Methyl phenyl carbonate

## INTERNATIONAL SEARCH REPORT

International application No.
**PCT/JP2021/042234**

**A. CLASSIFICATION OF SUBJECT MATTER**

***C07C 37/52***(2006.01)i; ***C07C 37/68***(2006.01)i; ***C07C 39/16***(2006.01)i; ***C07C 68/08***(2006.01)i; ***C07C 69/96***(2006.01)i; ***C08G 64/04***(2006.01)i; ***C08G 64/06***(2006.01)i; ***C08G 64/20***(2006.01)i; ***C08J 11/16***(2006.01)i; ***C08J 11/24***(2006.01)i; ***C08J 11/28***(2006.01)i

FI: C07C37/52; C08J11/16; C08J11/28; C08G64/20; C08G64/04; C07C39/16 ZAB; C07C37/68; C07C68/08; C07C69/96 Z; C08G64/06; C08J11/24

According to International Patent Classification (IPC) or to both national classification and IPC

**B. FIELDS SEARCHED**

Minimum documentation searched (classification system followed by classification symbols)

C07C37/52; C07C37/68; C07C39/16; C07C68/08; C07C69/96; C08G64/04; C08G64/06; C08G64/20; C08J11/16; C08J11/24; C08J11/28

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | 公益社団法人日本化学会, 化学便覧　応用化学編, 第 7 版, 2014 pp. 884-885, 1146-1148, (The Chemical Society of Japan), non-official translation (Chemistry handbook: Applied chemistry part. 7th edition.) | 21, 23-27<br>1-20, 22 |
| X<br>A | JP 2009-242301 A (MITSUBISHI CHEMICALS CORP) 22 October 2009 (2009-10-22) examples 1-6 | 21-24<br>1-20, 25-27 |
| A | JP 6-340591 A (BAYER AG) 13 December 1994 (1994-12-13) entire text, all drawings | 1-27 |
| A | JP 5-255153 A (BAYER AG) 05 October 1993 (1993-10-05) entire text, all drawings | 1-27 |

☐ Further documents are listed in the continuation of Box C.　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 January 2022** | **25 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**
**Information on patent family members**

International application No.
**PCT/JP2021/042234**

| Patent document cited in search report | Publication date (day/month/year) | Patent family member(s) | Publication date (day/month/year) |
|---|---|---|---|
| JP 2009-242301 A | 22 October 2009 | (Family: none) | |
| JP 6-340591 A | 13 December 1994 | US 5440066 A<br>entire text, all drawings | |
| JP 5-255153 A | 05 October 1993 | US 5266716 A<br>entire text, all drawings<br>EP 547479 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6340591 A
- JP 2004051620 A
- JP 2006022029 A
- JP 3291257 A
- JP 2011225711 A
- JP 2012092247 A
- JP 2012111858 A